(19) Europäisches Patentamt
European Patent Office
Office européen des brevets

(11) EP 4 144 747 A1

(12) **EUROPEAN PATENT APPLICATION**
published in accordance with Art. 153(4) EPC

(43) Date of publication:
**08.03.2023 Bulletin 2023/10**

(21) Application number: **21818327.5**

(22) Date of filing: **03.06.2021**

(51) International Patent Classification (IPC):
***C07K 1/04*** *(2006.01)* ***C07K 1/06*** *(2006.01)*

(52) Cooperative Patent Classification (CPC):
**C07K 1/04; C07K 1/06**

(86) International application number:
**PCT/JP2021/021125**

(87) International publication number:
**WO 2021/246471 (09.12.2021 Gazette 2021/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.06.2020 JP 2020096801**
**25.12.2020 JP 2020217156**
**06.05.2021 JP 2021078394**

(71) Applicant: **CHUGAI SEIYAKU KABUSHIKI KAISHA**
**Tokyo 115-8543 (JP)**

(72) Inventors:
- **IWASAKI, Kotaro**
**Kamakura-shi, Kanagawa 247-8530 (JP)**
- **WADAMOTO, Manabu**
**Kamakura-shi, Kanagawa 247-8530 (JP)**
- **SHIINA, Junichi**
**Kamakura-shi, Kanagawa 247-8530 (JP)**

(74) Representative: **Vossius & Partner Patentanwälte Rechtsanwälte mbB**
**Siebertstraße 3**
**81675 München (DE)**

(54) **EFFICIENT PEPTIDE CONDENSATION METHOD FOR DIFFICULT SEQUENCES**

(57) It was found that the use of an additive in a small amount relative to an amino acid or a peptide added to the N-terminus enables efficient progress of a condensation reaction even when an amino acid having large steric hindrance is contained, and the intended peptide compound can be obtained in a high yield and with a high purity.

EP 4 144 747 A1

## Description

[Technical Field]

The present invention relates to methods for producing a peptide compound using a condensation reaction.

[Background Art]

Peptides, a molecule in which a large number of amino acids are connected, play an essential role in life activities of living organisms. With the development of biology and chemistry, peptides have become more deeply understood and various attempts including utilization of natural peptides for creating new drugs, research and development of functional peptides by artificially designing peptides are being actively carried out (Non-Patent Literature (NPL) 1). In particular, it was reported that cyclization of a peptide and N-alkylation, particularly N-methylation, of constituent amino acids contributes to the increase in membrane permeability and metabolic stability (NPL 2 and NPL 3). There are also reports on findings and discussions of drug-like cyclic peptide structures that are key to the internalization and oral-preparation development, and thus importance of such a structure in peptide drug discovery is increasingly recognized (Patent Literature (PTL) 1).

Peptide synthesis is achieved by elongation to a desired sequence by the formation of amide bonding. More specific methods include a liquid phase method and a solid phase method (NPL 4).

Among these, the solid phase method consists of the following steps, using an atomic group connected to a polymer resin (a resin for solid-phase synthesis) as a linker:

1) formation of a covalent bond with the C-terminal carboxyl group of an amino acid or a peptide (a loading step);
2) a deprotection reaction of the N-terminal amino group of the loaded amino acid or peptide, followed by a condensation reaction with an amino acid corresponding to the next in a sequence, and repetition of such a deprotection reaction and a condensation reaction until the desired sequence is obtained (an elongation step); and
3) cleavage of a peptide from the resin for solid-phase synthesis (a resin removal (deresination) step) (NPLs 5 and 6).

The solid phase method is carried out in a two-layer reaction system composed of a solid layer and a liquid layer, and comprises the step of bringing a resin for solid-phase synthesis that is solid and to which a target molecule has been linked into contact with liquid reaction solutions in which deprotection reaction reagents or condensation reaction reagents are dissolved. Since the target peptide is linked to the resin for solid-phase synthesis, by merely incorporating the operation of washing the resin for solid-phase synthesis between the steps, excessive reagents and reagent-derived impurities can be separated from the resin for solid-phase synthesis to which the target peptide has been linked, and thus successive elongation can be conveniently carried out.

An Fmoc group and a Boc group are commonly used in the solid phase method as the protecting groups for the amino group at the N-terminus of the amino acid main chain.

Resins for solid-phase synthesis are roughly classified according to the atomic group that is linked to the polymer for the resin for serving as a linker, and a resin for solid-phase synthesis comprising a linker atomic group containing a trityl group or a benzyl group linked thereto is commonly used. More specifically, representative examples include CTC resin, Wang resin, SASRIN resin, and Rink Amide resin. The deresination step is mainly carried out under acidic conditions, and easiness of deresination depends on the stability of the linker atomic group against acid. For example, a deresination reaction of a peptide from CTC resin that is capable of linking a peptide using a trityl group as a linker can be carried out even with a weakly acidic reagent. On the other hand, strongly acidic conditions are applied to the deresination reaction of a peptide from Wang resin that is capable of linking a peptide using a benzyl group as a linker (NPL 6).

As described above, the peptide elongation step, irrespective of whether it is a solid phase method or liquid phase method, consists of the repetition of the condensation step and the deprotection step, the condensation step dehydratively condensing the amino group of an amino acid or a peptide with the carboxyl group of an amino acid being newly introduced which has its N-terminal amino group protected, and the deprotection step removing the protecting group of the newly introduced N-terminal amino group. When failure of condensation reaction and/or competition with side reactions build up during this repetition of steps, impurities having deletion or addition of amino acids from the peptide having the intended amino acid sequence are produced, which can result in an impaired yield and/or purity of the peptide having the intended sequence.

Side reactions that are reported as commonly occurring during a condensation reaction in the solid phase method are, in addition to incomplete condensation reaction, epimerization of the carboxyl group $\alpha$ position due to the activation of carboxylic acid or the property of the reaction solution, guanylation of the N-terminus, N-Fmoc removal, and combinations thereof (NPL 6). Among these, as a countermeasure against the incomplete condensation reaction,

there is a report that exposing to the same conditions once again is more effective than prolonging the reaction time (NPL 6). A defective product (deletion product) may be produced when an unreacted N-terminal amino group that has not achieved the desired condensation reaction reacts with an amino acid to be newly introduced which has its N-terminal amino group protected (also referred to as the amino acid corresponding to the next in a sequence). To prevent production of this defective product, an acylating agent such as acetic anhydride or benzoyl chloride is reacted in the presence of pyridine so that the unreacted N-terminal amino group for which the desired condensation reaction has not been achieved is capped with the acylating agent, which can block the elongation in the undesired sequence (NPL 6).

As described above, the use of CTC resin enables the deresination reaction of a peptide to be carried out under mild, weakly acidic conditions. In the production of a peptide using CTC resin, a peptide having a protecting group that is readily removed under acidic conditions can be selectively deresinated without deprotecting the protecting group, and CTC resin is thus useful for producing a peptide that is protected with a desired protecting group (NPL 7). On the other hand, in the solid phase synthesis of a peptide using CTC resin, the peptide may be deresinated from CTC resin under mild conditions. There is a report that thus the covalent bond between the amino acid or the peptide loaded onto CTC resin and the linker of the resin is cleaved under the condensation reaction conditions (also referred to as premature cleavage, premature peptide release, or premature acidolytic cleavage), resulting in a poor yield of the peptide of interest (NPL 8). In NPL 8, Gly, Pro, and Leu are exemplified as amino acids that are loaded onto CTC resin, and concerning these amino acids, the stabilities of amino acid-loaded CTC resin during the condensation reaction with Fmoc-Gly-OH were investigated. As a result, it was confirmed that when a condensation reaction was carried out using a combination of N,N'-diisopropylcarbodiimide as a condensing agent and HOAt, Oxyma, or HOBt as an additive, the yield was impaired irrespective of the type of the loaded amino acid or the additive although to varying degrees, and the reduction of yield increased over time.

Concerning the solid phase synthesis method using CTC resin, there is a report that the condensation conditions of DIC/K-Oxyma significantly improved yield and provided a partial sequence of myelin basic protein (MBP) having an equal or higher purity, compared with DIC/Oxyma (NPL 9).

However, NPL 9 discloses that the condensation reaction using DIC/K-Oxyma resulted in a greatly advanced epimerization at the C-terminal carbonyl group $\alpha$ position by just a few-minute difference of the pretreatment time for producing an active ester, which is obtained from an amino acid and a condensing agent. Thus, considering that the reagent introduction time will be extended upon scaling-up, the solid phase peptide synthesis method using K-Oxyma causes concerns about an increased epimerization ratio and a decreased purity at the time of scale-up.

TOTU is used as a substitute reagent for DIC (NPL 10), but it requires a reaction under basic conditions and thus may cause epimerization. Moreover, since TOTU is an expensive reagent, it causes an increased production cost and is not preferable. In addition, NPL 10 describes a case in which the purity decreased due to the by-production of an excessively elongated product, in addition to a decreased yield due to premature cleavage. More specifically, NPL 10 discloses that when Gly-OCTC in which Gly has been loaded onto a resin for solid-phase synthesis was used as a starting material and an Fmoc amino acid elongation reaction using DIC/HOBt was carried out, a by-product having a Gly-Gly-OCTC sequence was obtained. This by-product is considered as being obtained as follows: the Gly residue loaded onto CTC resin was removed from the resin during the elongation reaction of the second-residue amino acid, and condensed with unreacted Gly-OCTC.

Furthermore, NPL 10 discloses that the stability of a peptide on CTC resin against acid increased by elongation of the second-residue amino acid from Gly linked to CTC resin, but this statement is contradictory to the description in NPL 8 which states that the yield decreases with the extension of time. To summarize, it can be said that an effective solution for overcoming premature cleavage has not been known yet. Accordingly, problems in the initial elongation using CTC resin, i.e., dipeptide synthesis, are a decreased yield and a decreased purity resulting from by-production of an excessively elongated product which has incorporated the loaded amino acid residue in excess.

A method using Oxyma as an additive to an amino acid condensation reaction is known. NPL 11 discloses that when Oxyma is used as an additive, epimerization can be suppressed and the acylation rate can be increased (NPL 11).

In PTLs 1, 2, 3, 4, 5 and 6 and NPL 12 described below, various condensing agents and additives are used in peptide production by a condensation reaction. However, the problems in a peptide condensation reaction, such as premature cleavage, are not described in any of these documents.

PTL 1 relates to a novel peptide cyclization method that provides an effective drug discovery method for tough targets for which it was previously difficult to discover drugs, as well as novel peptides and a library containing them. Although this document includes working examples in which reagents are used in specific proportions, there is no description that the reaction conditions were set to solve the problems unique to the peptide condensation reaction, exemplified by premature cleavage.

In PTL 2, a solid phase peptide synthesis method using CTC resin was carried out. PTL 2 merely discloses that in previous production methods, a side reaction progressed due to the influence of taking an atypical conformation associated with peptide chain elongation, and that the production method was improved to solve this problem. Furthermore, since unnatural amino acids represented by N-substituted amino acids were not contained in amino acids that

constitute peptides, there is no description concerning defects such as incomplete condensation reaction resulting from an unusual amino acid structure, or for solving problems relating to the defects of condensation reaction.

In PTL 3, solid phase peptide synthesis using Rink's amide resin was carried out by a method that includes the pretreatment step of converting the C-terminal amino acid into an active ester in advance (also referred to as preactivation) and then adds a resin for solid-phase synthesis to the reaction solution that has undergone the pretreatment step. However, PTL 3 merely describes the production of a peptide having a specific sequence (AMG416 or a salt thereof), and does not disclose an improvement to the elongation reaction of a low-reactivity amino acid such as a condensation reaction between amino acids having large steric hindrance.

PTL 4 describes an improvement to the Fmoc removing reaction. However, it does not describe the problems unique to the peptide condensation reaction exemplified by premature cleavage. Furthermore, in PTL 4, since use of a large excess of amino acids is necessary, the method is not suitable for production of peptides containing unnatural amino acids represented by N-substituted amino acids that are not readily available compared with natural amino acids.

PTL 5 describes a method comprising a mild deresination step in which damage to a peptide containing an N-substituted amino acid that is unstable to acid is avoided in solid phase synthesis and protecting groups for a side-chain functional group that is not removed in the deresination step. However, PTL 5 does not describe the problems unique to the peptide condensation reaction exemplified by premature cleavage.

PTL 6 describes a method for treating with a deprotecting agent in the deprotecting step of solid phase synthesis. However, PTL 6 does not describe the problems unique to the peptide condensation reaction exemplified by premature cleavage.

NPL 12 describes a solid phase method in which neither DMF nor DCM that are commonly used in a solid phase method was used, and an alternative solvent that is highly environmentally friendly was used. However, NPL 12 does not describe a condensation reaction of amino acids having large steric hindrance such as N-substituted amino acids, $\alpha$,$\alpha$-disubstituted **amino acids, or** $\beta$-branched amino acids.

As described above, in the production of a peptide, although a decrease in the yield of a condensation reaction and a decrease in peptide purity due to a side reaction have been known, it is only recently that the problems unique to the condensation reaction of a peptide exemplified by premature cleavage have become known. The only knowledge available so far was that when Gly, Pro, or Leu is loaded onto a resin for solid-phase synthesis, bonding between the amino acid and the resin for solid-phase synthesis is cleaved during a condensation reaction and moreover, when a natural amino acid in which a protecting group has been introduced is condensed with Gly-OCTC in which Gly has been loaded onto a resin for solid-phase synthesis, an excessively elongated product having a sequence different from the amino acid sequence of interest is obtained.

[Citation List]

[Patent Literature]

[PTL 1] WO 2013100132
[PTL 2] WO 2011006644
[PTL 3] WO 2015154031
[PTL 4] WO 2017070512
[PTL 5] WO 2018225851
[PTL 6] WO 2019117274

[Non-Patent Literature]

[NPL 1] Future Med. Chem., 2009, 1, 1289-1310.
[NPL 2] Acc Chem. Res., 2008, 41, 1331-1342.
[NPL 3] Angew. Chem. Int. Ed., 2013, 52, 254-269.
[NPL 4] Amino Acids, Peptides and Proteins in Organic Chemistry: Building Blocks, Catalysis and Coupling Chemistry, Volume 3, 2011.
[NPL 5] Amino Acids, 2018, 50, 39-68.
[NPL 6] Solid phase peptide synthesis (published by Bachem) [Retrieved on May 28, 2020], Internet <URL:https://www.bachem.com/fileadmin/user_upload/pdf/Catalogs_Brochures/Solid-_Phase_Peptide_ Synthesis.pdf>
[NPL 7] QSAR Comb. Sci., 2007, 26, 1027-1035.

[NPL 8] ACS Comb. Sci., 2013, 15, 229-234.
[NPL 9] Eur. J. Org. Chem. 2013, 6372-6378.
[NPL 10] Side reactions in Peptide Synthesis, 2015, 1-31, Academic Press.
[NPL 11] Chem. Eur. J. 2009, 15, 9394-9403.
[NPL 12] Green. Chem., 2019, 21, 2594-2600.

[Summary of Invention]

[Technical Problem]

Having applied condensation reaction conditions of a solid phase method that are commonly used in conventional peptide synthesis to the production of a peptide compound containing an unnatural amino acid using CTC resin, the present inventors faced problems such as significantly decreased yield and purity due to incomplete condensation reaction and premature cleavage. Such defects during condensation reaction were found to be prominent not only in the **condensation reaction between natural** α-amino acids described in NPLs 8, 9, and 10, but also in sequences having large steric hindrance and poor reactivity in a condensation reaction, exemplified by N-substituted amino acids and amino acids having a branch at the β-position of a side chain (also referred to **as** β-branched amino acids). An objective of the present invention is to provide methods of producing a peptide compound with high purity and in a high yield, which methods can suppress premature cleavage and also can suppress incomplete reaction and side reactions such as competition of epimerization especially in the condensation reaction step of a solid phase peptide synthesis method, use a condensing agent and an additive that are readily available, and use condensation conditions that can be applied even to unnatural amino acids, which is of high rarity and use amount thereof is preferably reduced.

[Solution to Problem]

To solve the above problems, the present inventors conducted diligent research on one-residue + one-residue synthesis in which a second amino acid is condensed with a first amino acid, mainly an N-substituted amino acid which is loaded onto a resin for solid-phase synthesis, to give a two-residue peptide. Conditions commonly applied to a condensation reaction using a carbodiimide compound exemplified by DIC that is a condensing agent and an N-hydroxy compound exemplified by Oxyma that is an additive, both of which are commonly used in peptide synthesis, are as follows: using the amino acid to be introduced, the condensing agent, and the additive in the same equivalent ratio. However, such commonly-used conditions could not solve the problems unique to the condensation reaction of a peptide, exemplified by premature cleavage. To solve the problems, the present inventors conducted diligent research and investigated into various equivalent ratios of the condensing agent and the additive relative to the first amino acid or peptide and/or the second amino acid or peptide, and found methods for solving the problems unique to the condensation reaction of a peptide, exemplified by premature cleavage.

More specifically, the present inventors found a method which uses a condensing agent and an additive in a molar ratio calculated based on the first amino acid or peptide and/or the second amino acid or peptide in a condensation reaction to allow elongation with amino acids by condensing in a sequence for which amidation reaction is hard to advance due to large steric hindrance, specifically the sequence comprising N-substituted amino acids, α,α-disubstituted amino acids, β-branched amino acids, or amino acids having a bulky side chain. It was found that in one aspect, the methods of the present invention solve the problems unique to the condensation reaction of a peptide, exemplified by premature cleavage. Furthermore, it was found that in one aspect, the methods of the present invention can produce a peptide compound having the sequence of interest. It was also found that in one aspect, the methods of the present invention can carry out the intended condensation reaction in a high yield. In addition, it was found that in one aspect, the methods of the present invention can suppress a side reaction, exemplified by epimerization. Moreover, it was found that in one aspect, the methods of the present invention can achieve a high yield of a peptide compound of interest with high purity as the amidation reaction proceeds at a sufficient conversion rate.

Specifically, it was found that changing the molar ratio of the additive to the amino acid or peptide added to the N-terminus (*i.e.*, the second amino acid or peptide) is effective and in particular that it is effective to use the additive in a small amount relative to the amino acid or peptide added to the N-terminus. Moreover, it was found that changing the molar ratio of the condensing agent is also effective and in particular that it is effective to use the condensing agent in an excess relative to the amino acid or peptide added to the N-terminus. It was also found that changing the molar ratios of both the condensing agent and the additive is more effective. It was found that according to the method of the present invention, not only in the condensation of an amino acid or a peptide having a large steric hindrance but also in the condensation reaction of an amino acid or a peptide having a small steric hindrance, the amidation reaction proceeds at a sufficient conversion rate, and the peptide compound of interest can be obtained in a high yield with high purity.

More specifically, in one non-limiting specific aspect, the present invention encompasses the following:

[1] a method of producing a peptide compound, the method comprising the step of condensing a first amino acid or peptide and a second amino acid or peptide in the presence of an additive and a condensing agent to give a condensation product, wherein
the number of moles of the additive is smaller than the number of moles of the second amino acid or peptide;
[2] the method according to [1], wherein a molar ratio of the additive to the second amino acid or peptide is 0.8 or less;
[3] the method according to [1] or [2], wherein a molar ratio of the condensing agent to the second amino acid or peptide is 1.0 or more;
[4] the method according to any one of [1] to [3], wherein a molar ratio of the condensing agent to the second amino acid or peptide is 1.2 to 4.0;
[5] the method according to any one of [1] to [4], wherein a molar ratio of the second amino acid or peptide to the first amino acid or peptide is 10 or less;
[6] the method according to any one of [1] to [5], wherein a molar ratio of the second amino acid or peptide to the first amino acid or peptide is 1 to 2, and a molar ratio of the additive to the second amino acid or peptide is 0.7 or less;
[7] the method according to any one of [1] to [6], wherein a molar ratio of the second amino acid or peptide to the first amino acid or peptide (a first molar ratio) is 2 or more, and a molar ratio of the additive to the second amino acid or peptide is the first molar ratio minus 1 (first molar ratio - 1) or less;
[8] the method according to any one of [1] to [7], wherein a molar ratio of the additive to the first amino acid or peptide is 2.0 or less;
[9] the method according to any one of [1] to [8], wherein a molar ratio of the condensing agent to the first amino acid or peptide is 1.3 or more;
[10] the method according to [1], wherein a molar ratio of the second amino acid or peptide, the condensing agent, and the additive is the second amino acid or peptide : the condensing agent : the additive = about 2 : about 4 to 6 : about 1;
[11] the method according to [1], wherein a molar ratio of the first amino acid or peptide, the second amino acid or peptide, the condensing agent, and the additive is the first amino acid or peptide : the second amino acid or peptide : the condensing agent: the additive = about 1 : about 2 : about 4 : about 1, or about 1 : about 2.4 : about 7.2 : about 1.2, or about 1 : about 3 : about 6 : about 1.5;
[12] the method according to any one of [1] to [11], wherein the additive is Oxyma, HOBt, HOOBt, or HOAt;
[13] the method according to any one of [1] to [12], wherein the condensing agent is DIC, DCC, EDCI, or EDCI·HCl;
[14] the method according to any one of [1] to [13], wherein the additive is Oxyma;
[15] the method according to any one of [1] to [14], wherein the peptide compound is the condensation product or comprises the condensation product in its structure;
[16] the method according to any one of [1] to [15], which is carried out by a solid phase method;
[17] the method according to any one of [1] to [16], wherein the first amino acid or peptide is loaded onto a resin for solid-phase synthesis;
[18] the method according to [17], wherein the resin for solid-phase synthesis is a trityl resin;
[19] the method according to [18], wherein the trityl resin is a CTC resin, an Mmt resin, or an Mtt resin;
[20] the method according to any one of [1] to [19], wherein an amino group of the second amino acid or peptide is protected with a protecting group;
[21] the method according to [20], wherein the protecting group is a protecting group having an Fmoc skeleton;
[22] the production method according to any one of [1] to [21], wherein the step is carried out in a solvent selected from the group consisting of DMF, NMP, DMI, tetrahydrofuran, 2-methyltetrahydrofuran, ethyl acetate, dimethyl carbonate, and acetonitrile;
[23] the method according to any one of [17] to [22], further comprising the step of removing the resin for solid-phase synthesis;
[24] the method according to any one of [20] to [23], further comprising the step of removing the protecting group;
[25] the method according to any one of [1] to [24], wherein the first amino acid, or an N-terminal amino acid of the first peptide and/or a C-terminal amino acid of the first peptide, is an N-alkylamino acid;
[26] the method according to [24], wherein the first amino acid, or a C-terminal amino acid of the first peptide, is an N-**alkyl** β-amino acid;
[27] the method according to [25], wherein the first amino acid, or the N-terminal amino acid of the first peptide and/or the C-terminal amino acid of the first peptide, is represented by the following formula:

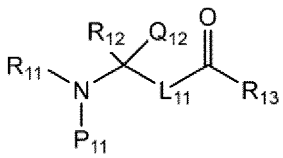

(1)

wherein,

when the first amino acid or the N-terminal amino acid of the first peptide is an amino acid represented by formula (1), $R_{11}$ is hydrogen, and when the C-terminal amino acid of the first peptide is an amino acid represented by formula (1), $R_{11}$ denotes a point of bonding with an adjacent amino acid,

$P_{11}$ is hydrogen or $C_1$-$C_6$ alkyl, and $R_{12}$ is hydrogen, $CONR_{12A}R_{12B}$, $COOR_{12c}$, $C_1$-$C_6$ alkyl, $C_3$-$C_8$ cycloalkyl, $C_7$-$C_{14}$ aralkyl, 5- to 10-membered heteroaryl $C_1$-$C_6$ alkyl, protected 5-to 10-membered heteroaryl $C_1$-$C_6$ alkyl, $C_1$-$C_6$ hydroxyalkyl, protected $C_1$-$C_6$ hydroxyalkyl, $C_1$-$C_6$ carboxyalkyl, protected $C_1$-$C_6$ carboxyalkyl, $C_1$-$C_6$ aminoalkyl, protected $C_1$-$C_6$ aminoalkyl, or $C_1$-$C_6$ alkylthio $C_1$-$C_6$ alkyl, or

$P_{11}$ and $R_{12}$ together with the nitrogen atom to which $P_{11}$ is bonded and the carbon atom to which $R_{12}$ is bonded form a 4- to 7-membered saturated heterocyclic ring,

$Q_{12}$ is hydrogen or $C_1$-$C_6$ alkyl,

$R_{12A}$ and $R_{12B}$ are independently $C_1$-$C_4$ alkyl, or

$R_{12A}$ and $R_{12B}$ together with the nitrogen atom to which they are bonded form a 4- to 8-membered ring which optionally contains one or more additional heteroatoms,

$L_{11}$ is a single bond or $-CH_2-$, and

when the first amino acid or the C-terminal amino acid of the first peptide is an amino acid represented by formula (1), $R_{13}$ denotes a point of bonding with a resin for solid-phase synthesis, and when the N-terminal amino acid of the first peptide is an amino acid represented by formula (1), $R_{13}$ denotes a point of bonding with an adjacent amino acid;

[28] the method according to [27], wherein the first amino acid or the N-terminal amino acid of the first peptide and/or the C-terminal amino acid of the first peptide is MeAsp-pip, MeAsp-aze, MeAsp-pyrro, MeAsp-mor, MeAsp-mor(26-bicyc), MeAsp-OtBu, MeAsp-NMe2, MeVal, MeGly, MeAla, MeLeu, D-3-MeAbu, bMeAla, MeIle, MeGly(cPent), MeChg, MePhe, MeTrp(Boc), MeThr(Bzl), MeGlu(OtBu), MeLys(Boc), MeMet, Aze(2), or Aib;

[29] the method according to any one of [1] to [28], wherein the second amino acid or the C-terminal amino acid of the second peptide **is an** α,α-disubstituted amino acid, **a** β-branched amino acid, an N-alkylamino acid, or another amino acid having a side chain with 2 or more carbon atoms;

[30] the **method according to [29], wherein the** β-branched amino acid is represented by the following formula:

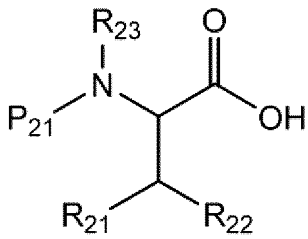

(2A)

wherein,

$P_{21}$ is hydrogen or $C_1$-$C_6$ alkyl,

$R_{21}$ and $R_{22}$ are each independently $C_1$-$C_4$ alkyl, $C_1$-$C_6$ alkoxy, or $C_1$-$C_6$ alkoxy$C_1$-$C_6$ alkyl, or $R_{21}$ and $R_{22}$ together with the carbon to which they are bonded form a 3- to 8-membered alicyclic ring, and

when the second amino acid is an amino acid represented by formula (2A), $R_{23}$ denotes a point of bonding with a protecting group for the amino group, and when the C-terminal amino acid of the second peptide is an amino acid represented by formula (2A), $R_{23}$ denotes a point of bonding with an adjacent amino acid;

[31] the method according to [30], wherein the β-branched amino acid is MeVal, D-MeVal, Val, Ile, MeIle, MeChg, Chg, MeGly(cPent), Gly(cPent), MeGly(cBu), Gly(cBu), MeGly(cPr), Gly(cPr), MeThr(tBu), or Thr(tBu);

[32] the **method according to [29], wherein the** α,α-disubstitutedamino acid is represented by the following formula:

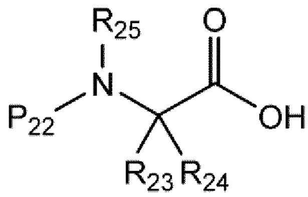
(2B)

wherein,

$P_{22}$ is hydrogen or $C_1$-$C_6$ alkyl,
$R_{23}$ and $R_{24}$ are independently selected from $C_1$-$C_6$ alkyl, $C_2$-$C_6$ alkenyl, or optionally substituted $C_7$-$C_{14}$ aralkyl, or
$R_{23}$ and $R_{24}$ together with the carbon atom to which they are bonded form a 3- to 8-membered alicyclic ring or a 4- to 7-membered saturated heterocyclic ring, and
when the second amino acid is an amino acid represented by formula (2B), $R_{25}$ denotes a point of bonding with a protecting group for the amino group, and when the C-terminal amino acid of the second peptide is an amino acid represented by formula (2B), $R_{25}$ denotes a point of bonding with an adjacent amino acid;

[33] the **method according to [32], wherein the** α,α-disubstituted amino acid is Aib, (Me)Abu, (Me)Leu, (Me)Algly, (Me)Phe, (Me)Phe(3-I), 1-ACPrC, cVal, cLeu, cHex, or Athpc;
[34] the method according to any one of [1] to [33], wherein the second amino acid or the C-terminal amino acid of the second peptide is an N-alkylamino acid;
[35] the method according to [34], wherein the N-alkylamino acid is MeAsp-pip, MeAsp-aze, MeAsp-pyrro, MeAsp-mor, MeAsp-mor(26-bicyc), MeAsp-OtBu, D-3-MeAbu, bMeAla, MeGly, MeAla, MeLeu, MePhe, Aze(2), Pro, MeAsp-NMe2, MeVal, MeIle, MeChg, MeGly(cPent), MeGly(cBu), MeGly(cPr), MeThr(tBu), D-MeVal, MeTrp(Boc), MeThr(Bzl), MeGlu(OtBu), MeLys(Boc), or MeMet;
[36] the method according to [29], wherein the other amino acid having a side chain with 2 or more carbon atoms is Lys(Z), Glu(OBzl), or Ser(tBu); and
[37] a method of producing a cyclic peptide compound, the method comprising the steps of:

obtaining a peptide compound by the method according to any one of [1] to [36]; and
cyclizing the peptide compound.

## Advantageous Effects of Invention

The present invention enables efficient production of a peptide compound comprising a sequence that is poorly reactive in a condensation reaction. Specifically, productivity can be increased, including better yield and purity of a peptide compound. More specifically, in one aspect, the methods of the present invention achieve a premature cleavage suppressing effect and, moreover, can even produce a peptide compound comprising an amino acid sequence which is susceptible to incomplete condensation reaction or to antagonization by epimerization when a conventional method is applied; thus, the yield and/or the purity of the peptide compound can be increased. Furthermore, in one aspect, the methods of the present invention can reduce a side reaction to the minimum even when the condensation reaction is prolonged, and/or can complete the reaction with a minimal amount of an amino acid or a peptide to be added to the N-terminus; thus the use amount thereof can be reduced. In the methods of the present invention, the reaction completes even with a reduced amount of reagent, and thus unlike the conventional methods, it is not necessary to use an excessive amount of reagent or to carry out double coupling in which the same conditions are repeated. As a result, the amount of reagent can be minimized even when an amino acid and a peptide of the sort that is difficult to amidate is used. This is a method particularly suitable for synthesizing a peptide compound containing an unnatural amino acid residue, which is expensive and rare. Moreover, in one aspect, the method of the present invention, compared with the conventional methods, exhibits higher reaction promoting effect when the solvent during condensation is reduced and the reaction is carried out at a high concentration; thus, the amount of amino acid used can be reduced, and the amount of solvent required not only during condensation but also in reagent washing after condensation can be reduced.
In addition, it is known that when an amino acid is loaded onto various resins for solid-phase synthesis in an amount exceeding the amount optimized by the conventional method, the condensation reaction becomes incomplete or significant side reactions occur with the conventional methods. However, even when such an amount is used, the reaction can be completed by applying one embodiment of the present invention. Accordingly, the amount of a peptide producible by a single run of a solid phase method can be increased.

According to the present invention, the condensation reaction between an amino acid or a peptide loaded onto a trityl resin (such as CTC resin) and an amino acid or a peptide for elongation can be carried out by sufficiently consuming the amino acid or the peptide loaded onto the resin while solving the problems unique to the condensation reaction of a peptide, exemplified by premature cleavage, and thus the starting material can be efficiently converted into the molecule of interest.

[Description of Embodiments]

The abbreviations used herein are as follows.

A%: Area%
Alloc: Allyloxycarbonyl
Aze: Azetidino
Boc: tert-Butoxycarbonyl
Cbz: Benzyloxycarbonyl
COMU: (1 -Cyano-2-ethoxy-2-oxoethylideneaminooxy)dimethylamino-morpholino-carbenium hexafluorophosphate
CTC: 2-Chlorotrityl
CTC resin: Cl-Trt(2-Cl) resin
DCC: N,N'-Dicyclohexylcarbodiimide
DCM: Dichloromethane
DIC: N,N'-Diisopropylcarbodiimide
DMA: N,N-Dimethylacetamide
DMF: N,N-Dimethylformamide
DMI: 1,3-Dimethyl-2-imidazolidinone
EDCI: 1-Ethyl-3-(3-dimethylaminopropyl)carbodiimide Fmoc: 9-Fluorenylmethyloxycarbonyl
HATU: O-(7-Azabenzotriazol-1-yl)-N,N,N',N'-tetramethyluronium hexafluorophosphate
HMDS: 1,1,1,3,3,3-Hexamethyldisilazane
HOAt: 1-Hydroxy-7-azabenzotriazole
HOBt: 1-Hydroxybenzotriazole
HPLC: High performance liquid chromatography
$iPr_2NEt$: N,N-diisopropylethylamine
K-Oxyma: Ethyl (hydroxyimino)cyanoacetate, potassium salt
MeCN: Acetonitrile
MeOH: Methanol
2-MeTHF: 2-Methyltetrahydrofuran
Mmt resin: 4-Methoxytrityl chloride resin
Mor: Morpholino
MTBE: Methyl tert-butyl ether
Mtt resin: 4-Methyltrityl chloride resin
NMP: N-Methyl-2-pyrrolidone
Oxyma: Ethyl (hydroxyimino)cyanoacetate
PDA: Photodiode array detector
Pip: Piperidino
Pyrro: Pyrrolidino
t-Bu: t-Butyl
Teoc: 2-(Trimethylsilyl)ethoxycarbonyl
TFA: Trifluoroacetic acid
TMSOTf: Trimethylsilyl trifluoromethanesulfonate
TMU: Tetramethylurea
TOTU: O-[(Ethoxycarbonyl)cyanomethyleneamino]-N,N,N',N'-tetramethyluronium tetrafluoroborate

**The relationship between the abbreviations and the structures of** β-branched amino acids, α**,**α**-**disubstitutedamino acids, and N-alkylamino acids used herein is shown below. In the table below, while the amino acids are listed in a form in which the amino group is protected with an Fmoc group, the relationship between abbreviations of amino acids having a free amino group, which are obtained by removing the Fmoc group, or residues thereof and their structures can also be understood from the following table. Specifically, it is evident to those skilled in the art that, for example, MeAsp(OH)-pip is an amino acid that has the following structure obtained by removing the Fmoc group from Fmoc-MeAsp(OH)-pip of the following table, and the structure of the amino acid residue thereof, *i.e.*, MeAsp-pip, is also

evident to those skilled in the art.

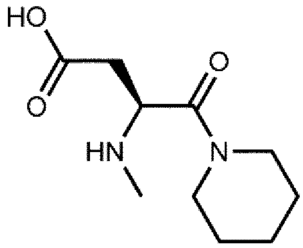

[Table 1-1]

| Abbreviation of β-branched amino acid | Structure of FmocOH amino acid | Abbreviation of α,α-disubstitutedamino acid | Structure of FmocOH amino acid | Abbreviation of N-alkyl amino acid | Structure of FmocOH amino acid |
|---|---|---|---|---|---|
| Fmoc-Val-OH | 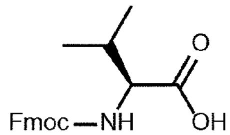 | Fmoc-Aib-OH | 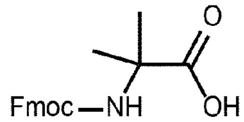 | Fmoc-MeAsp(OH)-pip | 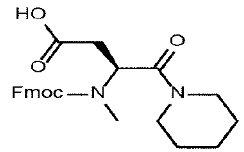 |
| Fmoc-MeVal-OH | 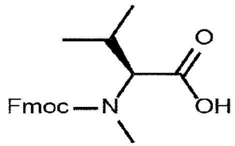 | Fmoc-(Me)Abu-OH | 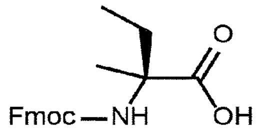 | Fmoc-MeAsp(OH)-aze | 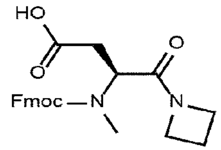 |
| Fmoc-Ile-OH | 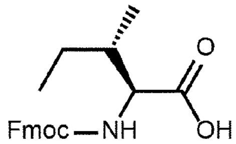 | Fmoc-(Me)Leu-OH | 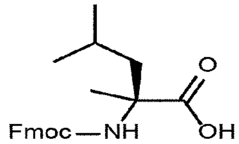 | Fmoc-MeAsp (OH)-pyrro | 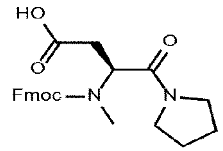 |
| Fmoc-Melle-OH | 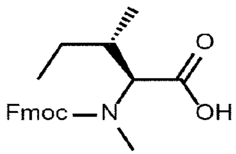 | Fmoc-(Me)Algly-OH | 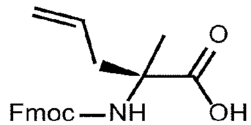 | Fmoc-MeAsp(OH)-mor | 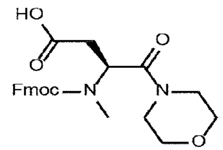 |
| Fmoc-Chg-OH | 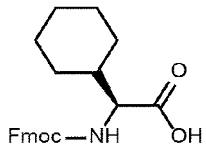 | Fmoc-(Me)Phe-OH | 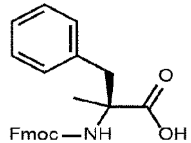 | Fmoc-MeAsp(OH)-mor (26-bicyc) | 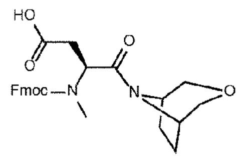 |
| Fmoc-MeChg-OH | 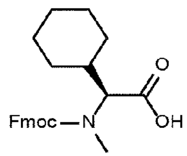 | Fmoc-(Me)Phe(3-I)-OH | 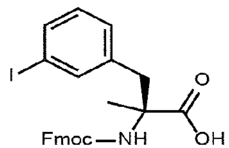 | Fmoc-MeAsp (OH)-OtBu | 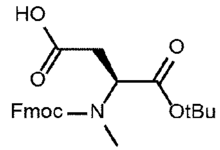 |

(continued)

| Abbreviation of β-branched amino acid | Structure of FmocOH amino acid | Abbreviation of α,α-disubstitutedamino acid | Structure of FmocOH amino acid | Abbreviation of N-alkyl amino acid | Structure of FmocOH amino acid |
|---|---|---|---|---|---|
| Fmoc-Gly(cPent)-OH | 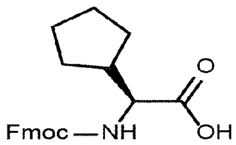 | Fmoc-1-ACPrC-OH | 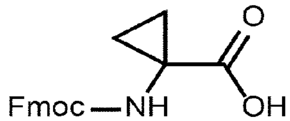 | Fmoc-D-3-MeAbu-OH | 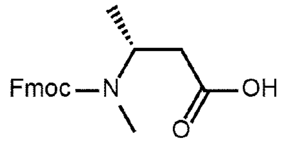 |

[Table 1-2]

| Abbreviation of β-branched amino acid | Structure of FmocOH amino acid | Abbreviation of α,α-disubstituted amino acid | Structure of FmocOH amino acid | Abbreviation of N-alkyl amino acid | Structure of FmocOH amino acid |
|---|---|---|---|---|---|
| Fmoc-MeGly(cPent)-OH | 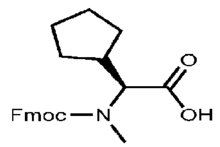 | Fmoc-cVal-OH | 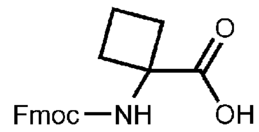 | Fmoc-bMeAla-OH | 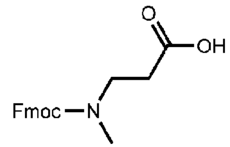 |
| Fmoc-Gly(cBu)-OH | 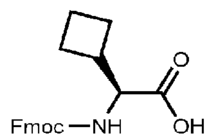 | Fmoc-cLeu-OH | 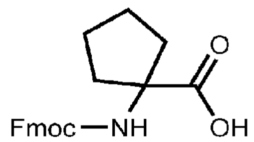 | Fmoc-MeGly-OH | 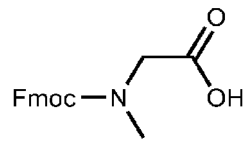 |
| Fmoc-MeGly(cBu)-OH | 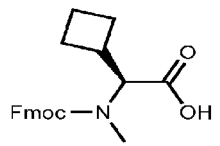 | Fmoc-cHex-OH | 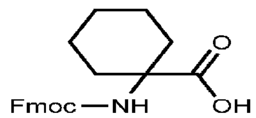 | Fmoc-MeAla-OH | 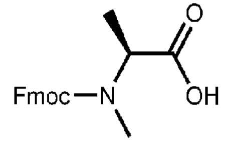 |
| Fmoc-Gly(cPr)-OH | 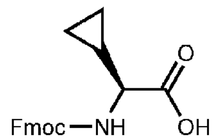 | Fmoc-Athpc-OH | 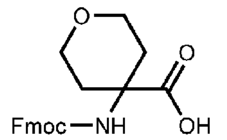 | Fmoc-MeLeu-OH | 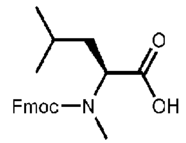 |

(continued)

| Abbreviation of β-branched amino acid | Structure of FmocOH amino acid | Abbreviation of α,α-disubstituted amino acid | Structure of FmocOH amino acid | Abbreviation of N-alkyl amino acid | Structure of FmocOH amino acid |
|---|---|---|---|---|---|
| Fmoc-MeGly(cPr)-OH | 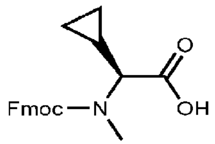 | | | Fmoc-MePhe-OH | 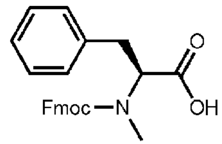 |
| Fmoc-Thr(tBu)-OH | 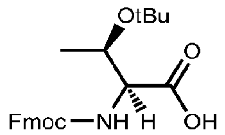 | | | Fmoc-Aze(2)-OH | 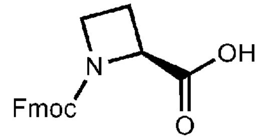 |
| Fmoc-MeThr(tBu)-OH | 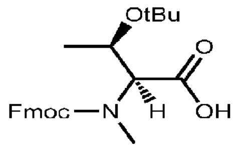 | | | Fmoc-Pro-OH | 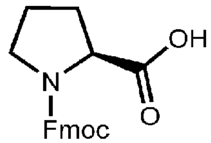 |
| Fmoc-D-MeVal-OH | 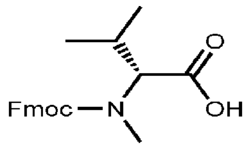 | | | Fmoc-MeAsp(OH)-NMe2 | 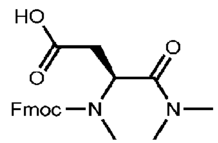 |

(Definitons of funcitional groups and the like)

Examples of "halogen atoms" herein include F, Cl, Br, and I.

"Alkyl" herein means a monovalent group derived by removing any one hydrogen atom from an aliphatic hydrocarbon, and has a subset of hydrocarbyl or hydrocarbon group structures not containing either a heteroatom (which refers to an atom other than carbon and hydrogen atoms) or an unsaturated carbon-carbon bond but containing hydrogen and carbon atoms in its backbone. The alkyl includes linear and branched alkyls. Specifically, the alkyl has 1 to 20 carbon atoms ($C_1$-$C_{20}$, hereinafter "$C_p$-$C_q$" means that the number of carbon atoms is p to q), and is preferably $C_1$-$C_{10}$ alkyl, and more preferably $C_1$-$C_6$ alkyl. Specific examples of alkyl include methyl, ethyl, n-propyl, i-propyl, n-butyl, s-butyl, t-butyl, isobutyl(2-methylpropyl), n-pentyl, s-pentyl(1-methylbutyl), t-pentyl(1,1-dimethylpropyl), neopentyl(2,2-dimethylpropyl), isopentyl(3-methylbutyl), 3-pentyl(1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

"Alkenyl" herein means a monovalent group having at least one double bond (two adjacent $SP^2$ carbon atoms). Depending on the configuration of a double bond and a substituent (if present), the geometrical form of the double bond can be entgegen (E) or zusammen (Z) as well as cis or trans configuration. The alkenyl includes linear and branched alkenyls. The alkenyl includes preferably $C_2$-$C_{10}$ alkenyl, and more preferably $C_2$-$C_6$ alkenyl, and specific examples include vinyl, allyl, 1-propenyl, 2-propenyl, 1-butenyl, 2-butenyl (including cis and trans forms), 3-butenyl, pentenyl, 3-methyl-2-butenyl, and hexenyl.

"Alkynyl" herein means a monovalent group having at least one triple bond (two adjacent SP carbon atoms). The alkynyl includes linear and branched alkynyls. The alkynyl includes preferably $C_2$-$C_{10}$ alkynyl, and more preferably $C_2$-$C_6$ alkynyl, and specific examples include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

"Cycloalkyl" herein means a saturated or partially saturated cyclic monovalent aliphatic hydrocarbon group and includes a monocyclic ring, a bicyclo ring, and a spiro ring. The cycloalkyl includes preferably $C_3$-$C_8$ cycloalkyl, and specific examples include cyclopropyl, cyclobutyl, cyclopentyl, cyclohexyl, cycloheptyl, cyclooctyl, bicyclo[2.2.1]heptyl, and spiro[3.3]heptyl.

"Aryl" herein means a monovalent aromatic hydrocarbon ring, and includes preferably $C_6$-$C_{10}$ aryl. Specific examples of the aryl include phenyl and naphthyl (*e.g.*, 1-naphthyl and 2-naphthyl).

"Heterocyclyl" herein means a non-aromatic cyclic monovalent group containing 1 to 5 hetero atoms in addition to carbon atoms. The heterocyclyl may have a double and/or triple bond within the ring, a carbon atom within the ring may be oxidized to form carbonyl, and heterocyclyl may be a monocyclic ring or a condensed ring. The number of atoms constituting the ring is preferably 4 to 10 (4- to 10-membered heterocyclyl), and more preferably 4 to 7 (4- to 7-membered heterocyclyl). Specific examples of the heterocyclyl include azetidinyl, oxetanyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, azetidinyl, oxazolidone, benzodioxanyl, benzoxazolyl, dioxolanyl, dioxanyl, tetrahydropyrrolo[1,2-c]imidazole, thietanyl, 3,6-diazabicyclo[3. 1. 1]heptanyl, 2,5-diazabicyclo[2.2.1]heptanyl, 3-oxa-8-azabicyclo[3.2.1]octanyl, sultam, and 2-oxaspiro[3.3]heptyl.

"Heteroaryl" herein means an aromatic cyclic monovalent group containing 1 to 5 heteroatoms in addition to carbon atoms. The ring may be a monocyclic ring, may be a condensed ring formed with another ring, or may be partially saturated. The number of atoms constituting the ring is preferably 5 to 10 (5- to 10-membered heteroaryl) and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzoimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

"Alkoxy" herein means an oxy group to which the above-defined "alkyl" is bonded, and includes preferably $C_1$-$C_6$ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

"Alkylthio" herein means a thio group to which the above-defined "alkyl" is bonded, and includes preferably $C_1$-$C_6$ alkylthio. Specific examples of the alkoxy include methylthio, ethylthio, 1-propylthio, 2-propylthio, n-butylthio, i-butylthio, s-butylthio, and t-butylthio.

"Amino" herein means $-NH_2$ in a narrow sense and -NRR' in a broad sense, wherein R and R' are independently selected from hydrogen, alkyl, alkenyl, alkynyl, cycloalkyl, heterocyclyl, aryl, and heteroaryl, or R and R' together with the nitrogen atom to which they are bonded form a ring. Preferred examples of the amino include $-NH_2$, mono-$C_1$-$C_6$ alkylamino, di-$C_1$-$C_6$ alkylamino, and 4- to 8-membered cyclic amino.

"Acyl (alkanoyl)" herein means a group in which a carbonyl group is bonded to hydrogen or the "alkyl", and includes preferably $C_1$-$C_6$ acyl, and more preferably $C_2$-$C_4$ acyl. Specific examples of the acyl include formyl, acetyl, propionyl, and butanoyl.

"Cycloalkoxy" herein means an oxy group to which the above-defined "cycloalkyl" is bonded, and includes preferably $C_3$-$C_8$ cycloalkoxy. Specific examples of the cycloalkoxy include cyclopropoxy, cyclobutoxy, and cyclopentyloxy.

"Alkylsulfonyl" herein means a sulfonyl group to which the above-defined "alkyl" is bonded, and includes preferably $C_1$-$C_6$ alkylsulfonyl. Specific examples of the alkylsulfonyl include methylsulfonyl.

"Hydroxyalkyl" herein means a group in which one or more hydrogens of the above-defined "alkyl" are replaced with hydroxyl groups, and is preferably $C_1$-$C_6$ hydroxyalkyl. Specific examples of the hydroxyalkyl include hydroxymethyl, 1-hydroxyethyl, 2-hydroxyethyl, 2-hydroxy-2-methylpropyl, and 5-hydroxypentyl.

"Carboxyalkyl" herein means a group in which one or more hydrogens of the above-defined "alkyl" are replaced with carboxyl groups, and is preferably $C_1$-$C_6$ carboxyalkyl. Specific examples of the carboxyalkyl include carboxymethyl, 1-carboxyethyl, and 2-carboxyethyl.

"Protected carboxyalkyl" herein means a group in which the carboxyl group contained in the above-defined "carboxyalkyl" is protected with a protecting group. Specific examples of the protecting group for the carboxyl group include a methyl group, an ethyl group, a t-Bu group, a benzyl group, a trityl group, a cumyl group, a methoxytrityl group, a 2-(trimethylsilyl)ethyl group, a 2,2,2-trichloroethyl group, and an allyl group.

"Aminoalkyl" herein means a group in which one or more hydrogens of the above-defined "alkyl" are replaced with the above-defined "amino", and is preferably $C_1$-$C_6$ aminoalkyl. Specific examples of the aminoalkyl include 1-pyridylmethyl, 2-(1-piperidyl)ethyl, 3-(1-piperidyl)propyl, and 4-aminobutyl.

"Protected aminoalkyl" herein means a group in which the amino group contained in the above-defined "aminoalkyl" is protected with a protecting group. Specific examples of the protecting group for the amino group include Fmoc, Boc, Cbz, Alloc, Teoc, trifluoroacetyl, pentafluoropropionyl, phthalloyl, tosyl, 2-nitrobenzenesulfonyl, 4-nitrobenzenesulfonyl, and 2,4-dinitrobenzenesulfonyl.

"Haloalkyl" herein means a group in which one or more hydrogens of the above-defined "alkyl" are replaced with halogen, and is preferably $C_1$-$C_6$ haloalkyl, and more preferably $C_1$-$C_6$ fluoroalkyl. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

"Alkoxyalkyl" herein means a group in which one or more hydrogens of the above-defined "alkyl" are replaced with the above-defined "alkoxy", and is preferably $C_1$-$C_6$ alkoxy $C_1$-$C_6$ alkyl, and more preferably $C_1$-$C_6$ alkoxy $C_1$-$C_2$ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

"Alkylthioalkyl" herein means a group in which one or more hydrogens of the above-defined "alkyl" are replaced with the above-defined "alkylthio". The alkylthioalkyl is preferably $C_1$-$C_6$ alkylthio $C_1$-$C_6$ alkyl, and more preferably $C_1$-$C_6$ alkylthio $C_1$-$C_2$ alkyl. Specific examples of the alkylthioalkyl include methylthiomethyl, ethylthiomethyl, 1-propylthiomethyl, 2-propylthiomethyl, n-butylthiomethyl, i-butylthiomethyl, s-butylthiomethyl, t-butylthiomethyl, 1-methylthioethyl, and 2-ethylthioethyl.

"Haloalkoxy" herein means a group in which one or more hydrogens of the above-defined "alkoxy" are replaced with halogen, and is preferably $C_1$-$C_6$ haloalkoxy. Specific examples of the haloalkoxy include difluoromethoxy, trifluoromethoxy, 2,2-difluoroethoxy, and 2,2,2-trifluoroethoxy.

"Haloacyl (haloalkanoyl)" herein means a group in which a carbonyl group is bonded to the "haloalkyl", and includes preferably $C_2$-$C_6$ haloacyl, and more preferably $C_2$-$C_4$ haloacyl. Specific examples of the haloacyl include trifluoroacetyl, trichloroacetyl, pentafluoropropionyl, 2,3,3,3-tetrafluoro-2-(trifluoromethyl)propionyl, and 3,3,3-trifluoro-2-(trifluoromethyl)propionyl.

"Cycloalkylalkyl" herein means a group in which one or more hydrogens of the above-defined "alkyl" are replaced with the above-defined "cycloalkyl", and is preferably $C_3$-$C_8$ cycloalkyl $C_1$-$C_6$ alkyl, and more preferably $C_3$-$C_6$ cycloalkyl $C_1$-$C_2$ alkyl. Specific examples of the cycloalkylalkyl include cyclopropylmethyl, cyclobutylmethyl, cyclopentylmethyl, and cyclohexylmethyl.

"Cycloalkoxylalkyl" herein means a group in which one or more hydrogens of the above-defined "alkyl" are replaced with the above-defined "cycloalkoxy", and is preferably $C_3$-$C_8$ cycloalkoxy $C_1$-$C_6$ alkyl, and more preferably $C_3$-$C_6$ cycloalkoxy $C_1$-$C_2$ alkyl. Specific examples of the cycloalkoxyalkyl include cyclopropoxymethyl and cyclobutoxymethyl.

"Alkylsulfonylalkyl" herein means a group in which one or more hydrogens of the above-defined "alkyl" are replaced with the above-defined "alkylsulfonyl", and is preferably $C_1$-$C_6$ alkylsulfonyl $C_1$-$C_6$ alkyl, and more preferably $C_1$-$C_6$ alkylsulfonyl $C_1$-$C_2$ alkyl. Specific examples of the alkylsulfonylalkyl include methylsulfonylmethyl and 2-(methylsulfonyl)ethyl.

"Aralkyl (arylalkyl)" herein means a group in which at least one hydrogen atom of the above-defined "alkyl" is replaced with the above-defined "aryl", and is preferably $C_7$-$C_{14}$ aralkyl, and more preferably $C_7$-$C_{10}$ aralkyl. Specific examples of the aralkyl include benzyl, phenethyl, and 3-phenylpropyl.

"Heteroarylalkyl" herein means a group in which at least one hydrogen atom of the above-defined "alkyl" is replaced with the above-defined "heteroaryl", and is preferably 5- to 10-membered heteroaryl $C_1$-$C_6$ alkyl, and more preferably 5- to 10-membered heteroaryl $C_1$-$C_2$ alkyl. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5 -benzofuranyl)ethyl.

"Protected heteroarylalkyl" herein means a group in which one or more functional groups, e.g., amino groups, contained in the above-defined "heteroarylalkyl" are protected with a protecting group. Specific examples of the protecting group include Fmoc, Boc, Cbz, Alloc, Teoc, trifluoroacetyl, pentafluoropropionyl, phthalloyl, tosyl, 2-nitrobenzenesulfonyl, 4-nitrobenzenesulfonyl, and 2,4-dinitrobenzenesulfonyl.

Examples of the "protecting group for a carboxyl group" herein include an alkyl ester-type protecting group, a benzyl ester-type protecting group, and a substituted alkyl ester-type protecting group. Specific examples of the protecting group for a carboxyl group include a methyl group, an ethyl group, a t-Bu group, a benzyl group, a trityl group, a cumyl group, a methoxytrityl group, a 2-(trimethylsilyl)ethyl group, a 2,2,2-trichloroethyl group, and an allyl group.

Examples of the "protecting group for an amino group" herein include a carbamate-type protecting group, an amide-type protecting group, an imide-type protecting group, and a sulfonamide-type protecting group. Specific examples of the protecting group for an amino group include Fmoc, Boc, Cbz, Alloc, Teoc, trifluoroacetyl, pentafluoropropionyl, phthaloyl, tosyl, 2-nitrobenzenesulfonyl, 4-nitrobenzenesulfonyl, and 2,4-dinitrobenzenesulfonyl.

"Alicyclic ring" herein means a non-aromatic hydrocarbon ring. The alicyclic ring may have an unsaturated bond within the ring, and may be a polycyclic ring having two or more rings. A carbon atom constituting the ring may be oxidized to form carbonyl. The alicyclic ring includes preferably a 3- to 8-membered alicyclic ring, and specific examples include a cyclopropane ring, a cyclobutane ring, a cyclopentane ring, a cyclohexane ring, a cycloheptane ring, a cyclooctane ring, and a bicyclo[2.2.1]heptane ring.

"Saturated heterocyclic ring" herein means a non-aromatic heterocyclic ring containing 1 to 5 hetero atoms in addition to carbon atoms and not containing a double bond and/or a triple bond within the ring. The saturated heterocyclic ring may be a monocyclic ring, or may form a condensed ring with another ring, *e.g.*, an aromatic ring such as a benzene ring. The saturated heterocyclic ring includes preferably a 4- to 7-membered saturated heterocyclic ring, and specific examples include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolididine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, and an indoline ring.

"Peptide" herein refers to a peptide in which one or more natural amino acids and/or unnatural amino acids are linked by an amide bond and/or an ester bond. The peptide is preferably a peptide comprising 1 to 15 amino acid residues, and more preferably a peptide chain consisting of 5 to 12 amino acid residues.

"Peptide compound" herein is not particularly limited as long as it is a peptide compound in which natural amino acids and/or unnatural amino acids are linked by way of an amide bond or an ester bond, and is a peptide compound having preferably 5 to 30 residues, more preferably 8 to 15 residues, and even more preferably 9 to 13 residues. The peptide compound synthesized in the present invention preferably contains at least 3 N-substituted amino acids, and more preferably contains at least 5 or more N-substituted amino acids, per peptide. These N-substituted amino acids may be present consecutively or nonconsecutively in the peptide compound. The peptide compound in the present invention may be linear or cyclic, and is preferably a cyclic peptide compound.

"Cyclic peptide compound" herein is a cyclic peptide compound that can be obtained by cyclizing the group on the N-terminal side and the group on the C-terminal side of a linear peptide compound. Cyclization may be performed in any manner, such as cyclization by a carbon-nitrogen bond as in an amide bond, cyclization by a carbon-oxygen bond as in an ester bond and an ether bond, cyclization by a carbon-sulfur bond as in a thioether bond, cyclization by a carbon-carbon bond, or cyclization by heterocyclic ring construction. Among these, cyclization *via* a covalent bond such as an amide bond or a carbon-carbon bond is preferable, and cyclization via an amide bond formed between a side-chain carboxyl group and a main-chain amino group at the N-terminus is more preferable. The positions of the carboxyl group, amino group, and the like used in cyclization may be a position on the main chain or on a side chain, and are not particularly limited as long as cyclization is possible.

"Cyclization" of a peptide compound means forming a cyclic moiety containing 4 or more amino acid residues. The number of amino acids contained in the cyclic moiety of the cyclic peptide compound herein is not particularly limited, and examples include 4 to 20 residues, 5 to 15 residues, and 6 to 13 residues. The methods of converting a linear peptide compound into a cyclic peptide compound can be carried out by performing an intramolecular bond forming reaction according to the method described in, for example, Comprehensive Organic Transformations, A Guide to

Functional Group Preparations, 3rd Edition (Ed. R. C. Larock) or March's Advanced Organic Chemistry: Reactions, Mechanisms, and Structure, 7th Edition (Ed. M. B. Smith, J. March). After the bond forming reaction, a functional group converting reaction can be further carried out as well. Examples of the bond forming reaction include a C(O)-N bond formed from a carboxylic acid and an amine; a C-O-C bond, a C(O)-O bond, and a C(S)-O bond using an oxygen atom; a C(O)-S bond, a C(S)-S bond, a C-S-S-C bond, a C-S-C bond, a C-S(O)-C bond, a C-S(O2)-C bond using a sulfur atom; and a C-N-C bond, a C=N-C bond, an N-C(O)-N bond, an N-C(S)N bond, and a C(S)-N bond using a nitrogen atom. Further examples include reactions for forming a C-C bond using a transition metal as a catalyst, such as Suzuki reaction, Heck reaction, and Sonogashira reaction. Examples of the functional group converting reaction further carried out after the bond forming reaction include an oxidation reaction and a reduction reaction. A specific example includes a reaction in which a sulfur atom is oxidized to be converted into a sulfoxide group or a sulfone group. Another example includes a reduction reaction in which, among carbon-carbon bonds, a triple bond or a double bond is reduced to be converted into a double bond or a single bond. When two amino acids are bonded in the amino acid main chains, a ring-closed structure is formed by a peptide bond, and a covalent bond between the two amino acids may be formed by, for example, bonding of the side chains, or the side chain and the main chain, of the two amino acids.

"One or more" herein means one or two or more. When "one or more" is used in a context relating to the substituent of a group, the phrase means a number encompassing one to the maximum number of substituents permitted by that group. Specific examples of "one or more" include 1, 2, 3, 4, 5, 6, 7, 8, 9, 10, and/or a greater number.

Herein, the "resin for solid-phase synthesis" is not particularly limited as long as it can be used in the synthesis of a peptide compound by a solid-phase method. Specific examples of such a resin for solid-phase synthesis include those that enable removal under acidic conditions, such as CTC resin, NovaSyn TGT resin (TGT resin), Wang resin, SASRIN resin, trityl chloride resin (Trt resin), 4-methyltrityl chloride resin (Mtt resin), and 4-methoxytrityl chloride resin (Mmt). The resin can be suitably selected according to the functional group used on the amino acid. For example, when a carboxyl group (a main-chain carboxyl group, or a side-chain carboxyl group represented by Asp or Glu) or a hydroxy group on an aromatic ring (a phenol group represented by Tyr) is used as the functional group on the amino acid, a trityl chloride resin (Trt resin) or a 2-chlorotrityl chloride resin (CTC resin) is preferably used as the resin. When an aliphatic hydroxy group (an aliphatic alcohol group represented by Ser or Thr) is used as the functional group on the amino acid, a trityl chloride resin (Trt resin), a 2-chlorotrityl chloride resin (CTC resin), or a 4-methyltrityl chloride resin (Mtt resin) is preferably used as the resin. Herein, the resin may be referred to as resin.

The type of the polymer constituting the resin is also not particularly limited. In the case of a resin composed of polystyrene, polystyrene having either 100 to 200 mesh or 200 to 400 mesh may be used. The extent of crosslinking is also not particularly limited, and a resin crosslinked with 1% DVB (divinylbenzene) is preferable. Examples of the type of the polymer constituting the resin include Tentagel (registered trademark) and Chemmatrix (registered trademark).

In the production of the compound described herein, when the defined group undergoes undesired chemical conversion under the conditions of the performed method, the compound can be produced by means of, for example, protection and deprotection of a functional group. Selection and introduction/removal procedures of a protecting group can be performed according to, for example, the methods described in Greene's "Protective Groups in Organic Synthesis" (5th Ed., John Wiley & Sons, 2014), which may be suitably used depending on the reaction conditions. Further, the order of reaction steps such as introduction of a substituent can be changed as necessary.

Herein, when the modifier "optionally substituted" is used, examples of substituents therefor include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

Moreover, each of these substituents may be substituted, the substituents not being limited, and one or two or more may be freely and independently selected from, for example, any substituents including a halogen atom, an oxygen atom, a sulfur atom, a nitrogen atom, a boron atom, a silicon atom, or a phosphorus atom. That is, examples include optionally substituted alkyl, alkenyl, alkynyl, aryl, heteroaryl, aralkyl, and cycloalkyl.

The compound herein can be a salt thereof or a solvate of the compound or the salt. Examples of salts of the compound include hydrochloride; hydrobromide; hydroiodide; phosphate; phosphonate; sulfate; sulfonates such as methanesulfonate and p-toluenesulfonate; carboxylates such as acetate, citrate, malate, tartrate, succinate, and salicylate; alkali metal salts such as a sodium salt and a potassium salt; alkaline earth metal salts such as a magnesium salt and a calcium salt; and ammonium salts such as an ammonium salt, an alkylammonium salt, a dialkylammonium salt, a trialkylammonium salt, and a tetraalkylammonium salt. These salts are produced by, for example, bringing the compound into contact with an acid or a base. The solvate of the compound refers to a phenomenon in which solute molecules attract solvent molecules in a solution and form one molecular group, and is called a hydrate when the solvent is water. The compound herein may not only be a solvate formed of a single solvent selected from water, organic solvents such as alcohol (*e.g.*, methanol, ethanol, 1-propanol, or 2-propanol), dimethylformamide, or diglyme, and the like, but may also be a solvate formed of a plurality of solvents.

The term "amino acid" as used herein includes natural amino acids and unnatural amino acids (sometimes

referred to as amino acid derivatives). The term "amino acid" herein may mean an amino acid residue. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, and Pro. The unnatural amino acids (amino acid derivatives) are not particularly limited, and examples include β-amino acids, D-amino acids, N-su**bstituted amino acids,** α,α-disubstituted amino acids, amino acids having side chains that are different from those of natural amino acids, and hydroxycarboxylic acids. Amino acids herein may have any conformation. There is no particular limitation on the selection of amino acid side chain, but in addition to a hydrogen atom, it can be freely selected from, for example, an alkyl group, an alkenyl group, an alkynyl group, an aryl group, a heteroaryl group, an aralkyl group, a heteroaralkyl group, a cycloalkyl group, and a spiro-bonded cycloalkyl group. Each group may have a substituent, and there are no limitations on the substituent. For example, one or two or more substituents may be freely and independently selected from any substituents including a halogen atom, an O atom, an S atom, an N atom, a B atom, an Si atom, or a P atom. That is, examples include an optionally substituted alkyl group, alkoxy group, alkoxyalkyl group, alkenyl group, alkynyl group, aryl group, heteroaryl group, aralkyl group, and cycloalkyl group, as well as oxo, aminocarbonyl, and a halogen atom. In a non-limiting embodiment, amino acids herein may be compounds having a carboxyl group and an amino group in the same molecule (even in this case, imino acids such as proline and hydroxyproline are also included in amino acids).

Substituents derived from halogen include fluoro (-F), chloro (-Cl), bromo (-Br), and iodo (-I).

Substituents derived from an O atom include groups such as hydroxy (-OH), oxy (-OR), carbonyl (-C=O-R), carboxyl ($-CO_2H$), oxycarbonyl (-C=O-OR), carbonyloxy (-O-C=O-R), thiocarbonyl (-C=O-SR), carbonylthio (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C(=O)-SH), and carboxylcarbonyl ($-C(=O)-CO_2H$).

Examples of oxy (-OR) include alkoxy, cycloalkoxy, alkenyloxy, alkynyloxy, aryloxy, heteroaryloxy, and aralkyloxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Additional examples include groups in which the H atom bonded to the N atom in -C=O-NHR is further replaced with alkyl, cycloalkyl, alkenyl, alkynyl, aryl, heteroaryl, or aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroaryl carbonyl amino, and aralkylcarbonylamino. Additional examples include groups in which the H atom bonded to the N atom in -NH-C=O-R is further replaced with alkyl, cycloalkyl, alkenyl, alkynyl, aryl, heteroaryl, or aralkyl.

Examples of oxycarbonylamino (-NH-C=O-OR) include alkoxycarbonylamino, cycloalkoxycarbonylamino, alkenyloxycarbonylamino, alkynyl oxycarbonylamino, aryloxycarbonylamino, heteroaryloxycarbonylamino, and aralkyloxycarbonylamino. Additional examples include groups in which the H atom bonded to the N atom in -NH-C=O-OR is further replaced with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples include groups in which the H atom bonded to the N atom in $-NH-SO_2-R$ is further replaced with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkyl amino sulfonyl. Additional examples include groups in which the H atom bonded to the N atom in $-SO_2-NHR$ is further replaced with alkyl, cycloalkyl, alkenyl, alkynyl, aryl, heteroaryl, or aralkyl.

Examples of sulfamoylamino ($-NH-SO_2-NHR$) include alkylsulfamoylamino, cycloalkylsulfamoylamino, alkenyl sulfamoyl amino, alkynylsulfamoylamino, arylsulfamoylamino, heteroarylsulfamoylamino, and aralkylsulfamoylamino. The two H atoms bonded to the N atoms in $-NH-SO_2-NHR$ may be further replaced with substituents independently selected from the group consisting of alkyl, cycloalkyl, alkenyl, alkynyl, aryl, heteroaryl, and aralkyl, and these two substituents may form a ring.

Substituents derived from an S atom include thiol (-SH), thio (-S-R), sulfinyl (-S=O-R), sulfonyl ($-S(O)_2-R$), sulfo ($-SO_3H$) and pentafluorosulfanyl ($-SF_5$).

Examples of thio (-S-R) is selected from alkylthio, cycloalkylthio, alkenylthio, alkynylthio, arylthio, heteroarylthio, aralkylthio, and such.

Examples of sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl ($-S(O)_2-R$) include alkylsulfonyl, cycloalkylsulfonyl, alkenylsulfonyl, alkynylsulfonyl, arylsulfonyl, heteroarylsulfonyl, and aralkylsulfonyl.

Substituents derived from an N atom include azido ($-N_3$, also called "azido group"), cyano (-CN), primary amino ($-NH_2$), secondary amino (-NH-R), tertiary amino (-NR(R’)), amidino ($-C(=NH)-NH_2$), substituted amidino (-C(=NR)-NR’R"), guanidino ($-NH-C(=NH)-NH_2$), substituted guanidino (-NR-C(=NR‴)-NR’R"), and aminocarbonylamino (-NR-CO-NR’R").

Examples of secondary amino (-NH-R) include alkylamino, cycloalkylamino, alkenylamino, alkynylamino, arylamino, heteroarylamino, and aralkylamino.

Examples of tertiary amino (-NR(R’)) include amino groups having any two substituents each independently selected from alkyl, cycloalkyl, alkenyl, alkynyl, aryl, heteroaryl, and aralkyl, such as alkyl(aralkyl)amino, where any two such substituents may form a ring.

Examples of substituted amidino (-C(=NR)-NR’R") include groups in which three substituents R, R’, and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR‴)-NR’R") include groups in which R, R’, R", and R‴ are each independently selected from alkyl, cycloalkyl, alkenyl, alkynyl, aryl, heteroaryl, and aralkyl, and groups in which these form a ring.

Examples of aminocarbonylamino (-NR-CO-NR’R") include groups in which R, R’, and R" are each independently selected from a hydrogen atom, alkyl, cycloalkyl, alkenyl, alkynyl, aryl, heteroaryl, and aralkyl, and groups in which these form a ring.

Examples of a substituent derived from a B atom include boryl (-BR(R’)) and dioxyboryl (-B(OR)(OR’)). These two substituents R and R’ are each independently selected from alkyl, cycloalkyl, alkenyl, alkynyl, aryl, heteroaryl, aralkyl, and the like, or these may form a ring. Specific examples include a cyclic boryl group, and more specific examples include a pinacholatoboryl group, a neopentanediolatoboryl group, and a catecholatoboryl group.

Specific examples of the substituent on the nitrogen atom of the N-substituted amino acid herein include alkyl, $C_1$-$C_6$ alkyl, $C_1$-$C_4$ alkyl, methyl, $C_7$-$C_{14}$ aralkyl, benzyl, and phenethyl.

The main-chain amino group of the amino acid may be unsubstituted ($-NH_2$) or substituted (*i.e.*, -NHR, wherein R represents optionally substituted alkyl, alkenyl, alkynyl, aryl, heteroaryl, aralkyl, or cycloalkyl, and the carbon chain bonded to the N atom and the carbon atom at **the** $\alpha$-position may form a ring as in proline). Such an amino acid in which the main-chain amino group is substituted may be referred to as an "N-substituted amino acid" herein. Preferable examples of the "N-substituted amino acid" herein include, but are not limited to, N-alkyl amino acid, N-$C_1$-$C_6$ alkyl amino acid, N-$C_1$-$C_4$ alkyl amino acid, N-methyl amino acid, N-$C_7$-$C_{14}$ aralkyl amino acid, N-benzyl amino acid, and N-phenethyl amino acid.

The "amino acids" as used herein include all isotopes corresponding to the respective amino acids. In an isotope of an "amino acid", at least one atom is replaced with an atom having the same atomic number (number of protons) but a different mass number (sum of protons and neutrons). Examples of isotopes contained in the "amino acids" herein include a hydrogen atom, a carbon atom, a nitrogen atom, an oxygen atom, a phosphorus atom, a sulfur atom, a fluorine atom, and a chlorine atom, such as $^{2}H$, $^{3}H$, $^{13}C$, $^{14}C$, $^{1s}N$, $^{17}O$, $^{IS}O$, $^{32}P$, $^{35}S$, $^{18}F$, and $^{36}Cl$, respectively.

Herein, "to" indicating a numerical range includes the values at both ends and, for example, "A to B" means a numerical range that is A or more and B or less.

Herein, the term "about" when used in combination with a numerical value means a numerical range of +10% and -10% of the numerical value.

Herein, the meaning of the term "and/or" includes any combination obtained by suitably combining "and" and "or". Specifically, for example, "A, B, and/or C" includes the following 7 variations:
(i) A; (ii) B; (iii) C; (iv) A and B; (v) A and C; (vi) B and C; and (vii) A, B, and C.

(Production method)

The present invention relates to methods of producing a peptide compound, the method comprising the step of condensing a first amino acid or peptide and a second amino acid or peptide in the presence of an additive and a condensing agent to give a condensation product, wherein the number of moles of the additive is smaller than the number of moles of the second amino acid or peptide.

In an embodiment, the peptide compound obtained by the production method of the present invention may be an intermediate or a final product of an amino acid or peptide elongation step by a solid phase method or a liquid phase method. The elongation step can be carried out multiple times depending on the length of the amino acid sequence of the desired peptide compound, and the condensation step according to the present invention is included at least once or may be included multiple times in the elongation step. In the elongation step, a method known in the art can be used in a condensation step other than the condensation step according to the present invention.

In an embodiment, when the condensation step of the present invention is used in the final stage of the elongation step, the condensation product obtained by the condensation step of the present invention may be a peptide compound having the desired sequence. On the other hand, when a peptide compound having the desired sequence is obtained by further elongation by a known method in addition to the condensation step of the present invention, the condensation product obtained by the condensation step of the present invention is contained in the peptide compound as a partial structure of the peptide compound.

In the present invention, the "first amino acid" may be a natural amino acid or an unnatural amino acid.

In the present invention, the "first peptide" may be composed solely of natural amino acids, may be composed solely of unnatural amino acids, or may be composed of any combination of natural and unnatural amino acids.

In an embodiment, the first amino acid or the first peptide is preferably an amino acid, and more preferably an unnatural amino acid.

In an embodiment, the first amino acid or the first peptide may be an amino acid or a peptide in which the main-chain amino group is not protected. The main-chain amino group that is not protected is preferably at the N-terminus of the first amino acid or the first peptide.

When a solid phase method is used in the condensation reaction of the present invention, the first amino acid or the C-terminal amino acid of the first peptide is loaded onto a resin for solid-phase synthesis. In this case, when the first amino acid or the N-terminal and/or C-terminal amino acid of the first peptide is an unnatural amino acid or, in particular, an N-substituted amino acid such as an N-alkylamino acid, the intended condensation reaction does not sufficiently proceed in some cases with conventional methods due to premature cleavage. This is prominent when a trityl-based atomic group is used as the resin linker that links the resin for solid-phase synthesis and the amino acid. Even in such a case, the condensation reaction can efficiently proceed by using the method of the present invention.

In an embodiment, when the first amino acid or the N-terminal and/or C-terminal amino acid of the first peptide is an N-alkylamino acid, examples of the N-alkylamino acid include those having a side chain that is not a natural amino acid as well as $\alpha$**,** $\beta$ **and** $\gamma$ **amino acids, and a** preferred example includes an N-alkyl-$\beta$-amino acid.

Specific examples of the first amino acid or the N-terminal amino acid of the first peptide and/or the C-terminal amino acid of the first peptide include those having the following formula (1):

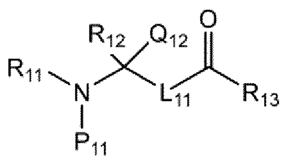

(1)

When the first amino acid or the N-terminal amino acid of the first peptide is an amino acid represented by formula (1), $R_{11}$ is hydrogen. On the other hand, when the C-terminal amino acid of the first peptide is an amino acid represented by formula (1), $R_{11}$ denotes a point of bonding with an adjacent amino acid, and usually the amino acid of formula (1) forms an amide bond with an adjacent amino acid at this site.

In an embodiment, in formula (1), $P_{11}$ is hydrogen or $C_1$-$C_6$ alkyl, and $R_{12}$ is hydrogen, $CONR_{12A}R_{12B}$, $COOR_{12c}$, $C_1$-$C_6$ alkyl, $C_3$-$C_8$ cycloalkyl, $C_7$-$C_{14}$ aralkyl, 5- to 10-membered heteroaryl $C_1$-$C_6$ alkyl, protected 5- to 10-membered heteroaryl $C_1$-$C_6$ alkyl, $C_1$-$C_6$ hydroxyalkyl, protected $C_1$-$C_6$ hydroxyalkyl, $C_1$-$C_6$ carboxyalkyl, protected $C_1$-$C_6$ carboxyalkyl, $C_1$-$C_6$ aminoalkyl, protected $C_1$-$C_6$ aminoalkyl, or $C_1$-$C_6$ alkylthio $C_1$-$C_6$ alkyl.

$P_{11}$ is preferably hydrogen or methyl, and is more preferably methyl.

When $R_{12}$ is $CONR_{12A}R_{12B}$, $R_{12A}$ and $R_{12B}$ are independently $C_1$-$C_4$ alkyl (preferably methyl or ethyl), or $R_{12A}$ and $R_{12B}$ together with the nitrogen atom to which they are bonded form a 4- to 8-membered ring optionally containing one or more additional heteroatoms (preferably a piperidine ring, an azetidine ring, a pyrrolidine ring, a morpholine ring, or a 3-oxa-8-azabicyclo[3.2.1]octane ring).

When $R_{12}$ is $COOR_{12C}$, $R_{12C}$ is allyl, t-butyl, or benzyl.

When $R_{12}$ is $C_1$-$C_6$ alkyl, $R_{12}$ is preferably methyl, isopropyl, or isobutyl.

When $R_{12}$ is $C_3$-$C_8$ cycloalkyl, $R_{12}$ is preferably cyclopentyl or cyclohexyl.

When $R_{12}$ is $C_7$-$C_{14}$ aralkyl, $R_{12}$ is preferably benzyl or phenethyl.

When $R_{12}$ is 5- to 10-membered heteroaryl $C_1$-$C_6$ alkyl, $R_{12}$ is preferably 5- to 10-membered heteroarylmethyl

or 5- to 10-membered heteroarylethyl. When $R_{12}$ is protected 5- to 10-membered heteroaryl $C_1$-$C_6$ alkyl, $R_{12}$ is preferably a group obtained by protecting a functional group such as an amino group contained in an aforementioned group with a Boc group.

When $R_{12}$ is $C_1$-$C_6$ hydroxyalkyl, $R_{12}$ is preferably hydroxymethyl, 1-hydroxyethyl, or 2-hydroxyethyl, and is more preferably 1-hydroxyethyl. When $R_{12}$ is protected $C_1$-$C_6$ hydroxyalkyl, $R_{12}$ is preferably a group obtained by protecting an aforementioned group with a protecting group for a hydroxyl group, such as a Bzl group or a tBu group.

When $R_{12}$ is $C_1$-$C_6$ carboxyalkyl, $R_{12}$ is preferably carboxymethyl, 1-carboxyethyl, or 2-carboxyethyl, and more preferably 2-carboxyethyl. When $R_{12}$ is protected $C_1$-$C_6$ carboxyalkyl, $R_{12}$ is preferably a group obtained by protecting an aforementioned group with a protecting group for a carboxyl group, such as a Bzl group or a tBu group.

When $R_{12}$ is $C_1$-$C_6$ aminoalkyl, $R_{12}$ is preferably 4-aminobutyl. When $R_{12}$ is protected $C_1$-$C_6$ aminoalkyl, $R_{12}$ is preferably a group obtained by protecting an aforementioned group with a protecting group for an amino group, such as a Boc group.

When $R_{12}$ is $C_1$-$C_6$ alkylthio $C_1$-$C_6$ alkyl, $R_{12}$ is preferably 2-methylthioethyl.

In an embodiment, $P_{11}$ and $R_{12}$ together with the nitrogen atom to which $P_{11}$ is bonded and the carbon atom to which $R_{12}$ is bonded form a 4- to 7-membered saturated heterocyclic ring. The 4- to 7-membered saturated heterocyclic ring is preferably an azetidine ring, a pyrrolidine ring, a piperidine ring, a piperazine ring, or a morpholine ring.

In formula (1), $Q_{12}$ is hydrogen or $C_1$-$C_6$ alkyl, and is preferably hydrogen or methyl.

In formula (1), $L_{11}$ is a single bond or $-CH_2-$,

When the first amino acid or the C-terminal amino acid of the first peptide is an amino acid represented by formula (1), $R_{13}$ denotes a point of bonding with the resin for solid-phase synthesis. On the other hand, when the N-terminal amino acid of the first peptide is an amino acid represented by formula (1), $R_{13}$ denotes a point of bonding with an adjacent amino acid, and usually the amino acid of formula (1) forms an amide bond with an adjacent amino acid at this site.

More specific examples of the first amino acid or the N-terminal and/or C-terminal amino acid of the first peptide include MeAsp-pip, MeAsp-aze, MeAsp-pyrro, MeAsp-mor, MeAsp-mor(26-bicyc), MeAsp-OtBu, MeAsp-NMe2, MeVal, MeGly, MeAla, MeLeu, D-3-MeAbu, bMeAla, MeIle, MeGly(cPent), MeChg, MePhe, MeTrp(Boc), MeThr(Bzl), MeGlu(OtBu), MeLys(Boc), MeMet, Aze(2), and Aib.

According to the condensation step of the present invention, even when the first amino acid or peptide is loaded onto the resin for solid-phase synthesis in such a high loaded amount (for example, 0.5 mmol/g or more based on the Fmoc quantification method) that the peptide would fall out of the resin under conventional methods, a condensation product can be obtained with this loaded amount maintained.

Accordingly, in the method of the present invention, any amount can be used as the loaded amount of the first amino acid or peptide to the resin for solid-phase synthesis, such as an amount of 0.2 mmol/g or more and 0.8 mmol/g or less based on the Fmoc quantification method, and the reaction can efficiently proceed even with a large loaded amount of 0.3 mmol/g or more or even 0.5 mmol/g or more.

In the present invention, the "second amino acid" may be a natural amino acid or an unnatural amino acid.

In an embodiment, the second amino acid or peptide may be an amino acid or a peptide in which the main-chain carboxyl group is not protected. The unprotected main-chain carboxyl group is preferably located at the C-terminus of the second amino acid or peptide.

In the present invention, the "second peptide" may be composed solely of natural amino acids, may be composed solely of unnatural amino acids, or may be composed of any combination of natural and unnatural amino acids.

In an embodiment, for example, when the second amino acid or the C-terminal amino acid of the second peptide is an unnatural amino acid, in particular, an N-substituted amino acid such as N-alkylamino acid or an amino acid having a side chain with two or more carbons (such **as an** $\alpha,\alpha$-disubstituted amino ac**id or a** $\beta$-branched amino acid), the condensation reaction may not sufficiently proceed under conventional methods. Even in such a case, efficient condensation is possible using the method of the present invention.

In the present invention, the amino group of the second amino acid or peptide is preferably protected with a protecting group. Examples of such a protecting group include those described above as "protecting groups for an amino group", and specific examples include protecting groups having an Fmoc skeleton, Cbz, Boc, Teoc, and Alloc.

In the present invention, the term "protecting group having an Fmoc skeleton" means an Fmoc group or a group in which any substituent has been introduced into any position in the constituent skeleton of an Fmoc group. Such a protecting group having an Fmoc skeleton specifically includes a protecting group represented by the following formula:

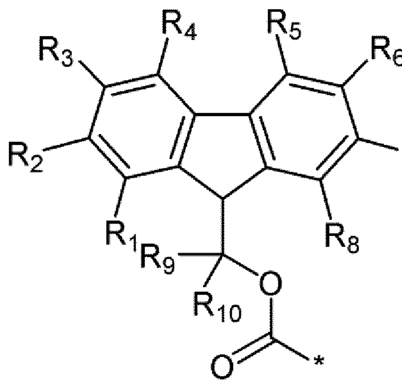

,

wherein

$R_1$ to $R_8$ are independently selected from the group consisting of hydrogen, $C_1$-$C_8$ alkyl, $C_1$-$C_8$ fluoroalkyl, halogen, sulfo, and trimethylsilyl; and
$R_9$ and $R_{10}$ are independently hydrogen or methyl.

More specifically, examples of the protecting group having the Fmoc skeleton include 9-fluorenylmethyloxycarbonyl (Fmoc) group, 2,7-di-tert-butyl-Fmoc (Fmoc(2,7tb)) group, 1-methyl-Fmoc (Fmoc(1Me)) group, 2-fluoro-Fmoc (Fmoc(2F)) group, 2,7-dibromo-Fmoc (Fmoc(2,7Br)) group, 2-monoisooctyl-Fmoc (mio-Fmoc) group, 2,7-diisooctyl-Fmoc (dio-Fmoc) group, 2,7-(3,3,4,4,5,5,6,6,7,7,8,8,8-tridecafluorooctyl)-Fmoc (tdf-Fmoc) group, 2,7-bis(trimethylsilyl)-Fmoc (Fmoc(2TMS)) group, (2-sulfo-9H-fluorene-9-yl)methoxycarbonyl group (Fmoc(2so3h)), [(1S)-1-(9H-fluorene-9-yl)ethoxy]carbonyl group (sm-Fmoc), and [(1R)-1-(9H-fluorene-9-yl)ethoxy]carbonyl group (rm-Fmoc).

When the second amino acid or the C-terminal amino acid of the second peptide **is a** β-bran**ched amino acid, specific examples of the** β-branched amino acid include those having the following formula (2A):

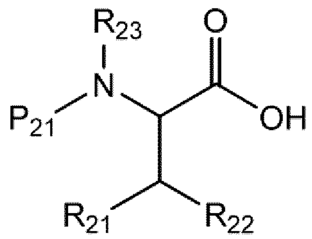

(2A)

In formula (2A), $P_{21}$ is hydrogen or $C_1$-$C_6$ alkyl. When $P_{21}$ is $C_1$-$C_6$ alkyl, it is preferably methyl.

In formula (2A), $R_{21}$ and $R_{22}$ are each independently $C_1$-$C_4$ alkyl, $C_1$-$C_6$ alkoxy, or $C_1$-$C_6$ alkoxy $C_1$-$C_6$ alkyl, or $R_{21}$ and $R_{22}$ together with the carbon to which they are bonded form a 3- to 8-membered alicyclic ring. When $R_{21}$ and $R_{22}$ are $C_1$-$C_4$ alkyl, $C_1$-$C_6$ alkoxy, or $C_1$-$C_6$ alkoxy $C_1$-$C_6$ alkyl, $R_{21}$ and $R_{22}$ are preferably methyl, ethyl, or tert-butoxy, and preferable combinations of $R_{21}$ and $R_{22}$ include methyl and methyl, methyl and ethyl, and methyl and tert-butoxy.

When $R_{21}$ and $R_{22}$ together form a 3- to 8-membered alicyclic ring, the 3- to 8-membered alicyclic ring is preferably a cyclopropane ring, a cyclobutane ring, a cyclopentane ring, or a cyclohexane ring.

When the second amino acid is an amino acid represented by formula (2A), $R_{23}$ denotes a point of bonding with a protecting group for an amino group. On the other hand, when the C-terminal amino acid of the second peptide is an amino acid represented by formula (2A), $R_{23}$ denotes a point of bonding with the adjacent amino acid, and usually the amino acid of formula (2A) forms an amide bond with an adjacent amino acid at this site.

More specific examples of such a β-branched amino acid of the second amino acid or C-terminal amino acid of the second peptide include MeVal, D-MeVal, Val, Ile, MeIle, MeChg, Chg, MeGly(cPent), Gly(cPent), MeGly(cBu), Gly(cBu), MeGly(cPr), Gly(cPr), MeThr(tBu), and Thr(tBu).

When the second amino acid or the C-terminal amino acid of the second peptide is an α,α-disubstituted **amino acid, specific examples of the** α,α-disubstituted amino acid include those having the following formula (2B):

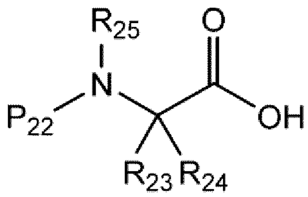

(2B)

In formula (2B), $P_{22}$ is hydrogen or $C_1$-$C_6$ alkyl. When $P_{22}$ is $C_1$-$C_6$ alkyl, it is preferably hydrogen.

In formula (2B), $R_{23}$ and $R_{24}$ are independently selected from $C_1$-$C_6$ alkyl, $C_2$-$C_6$ alkenyl, or optionally substituted $C_7$-$C_{14}$ aralkyl, or $R_{23}$ and $R_{24}$ together with the carbon atom to which they are bonded form a 3- to 8-membered alicyclic ring or a 4- to 7-membered saturated heterocyclic ring.

When $R_{23}$ and $R_{24}$ are $C_1$-$C_6$ alkyl, $C_1$-$C_6$ alkyl is preferably methyl, ethyl, or isopropyl. When $R_{23}$ and $R_{24}$ are $C_2$-$C_6$ alkenyl, $C_2$-$C_6$ alkenyl is preferably allyl. When $R_{23}$ and $R_{24}$ are $C_7$-$C_{14}$ aralkyl, $C_7$-$C_{14}$ aralkyl is preferably benzyl optionally substituted with halogen. The combination of $R_{23}$ and $R_{24}$ is preferably methyl and methyl, methyl and ethyl, methyl and isopropyl, methyl and isobutyl, methyl and allyl, or methyl and benzyl optionally substituted with halogen.

When $R_{23}$ and $R_{24}$ together with the carbon atom to which they are bonded form a 3- to 8-membered alicyclic ring, the alicyclic ring is preferably a cyclopropane ring, a cyclobutane ring, a cyclopentane ring, or a cyclohexane ring, and when $R_{23}$ and $R_{24}$ together with the carbon atom to which they are bonded form a 4- to 7-membered saturated heterocyclic ring, the saturated heterocyclic ring is preferably a tetrahydropyran ring.

When the second amino acid is an amino acid represented by formula (2B), $R_{25}$ denotes a point of bonding with a protecting group for an amino group. On the other hand, when the C-terminal amino acid of the second peptide is an amino acid represented by formula (2B), $R_{25}$ denotes a point of bonding with an adjacent amino acid, and usually the amino acid of formula (2B) forms an amide bond with an adjacent amino acid at this site.

More specific examples of **such a** α,α-di-substituted amino acid of the second amino acid or C-terminal amino acid of the second peptide include Aib, (Me)Abu, (Me)Leu, (Me)Algly, (Me)Phe, (Me)Phe(3-I), 1-ACPrC, cVal, cLeu, cHex, and Athpc.

In an embodiment, the second amino acid or the C-terminal amino acid of the second peptide is an N-alkylamino acid. Specific examples include MeAsp-pip, MeAsp-aze, MeAsp-pyrro, MeAsp-mor, MeAsp-mor(26-bicyc), MeAsp-OtBu, D-3-MeAbu, bMeAla, MeGly, MeAla, MeLeu, MePhe, Aze(2), Pro, MeAsp-NMe2, MeVal, MeIle, MeChg, MeGly(cPent), MeGly(cBu), MeGly(cPr), MeThr(tBu), D-MeVal, MeTrp(Boc), MeThr(Bzl), MeGlu(OtBu), MeLys(Boc) and MeMet, and MeVal and MeIle are preferable.

In an embodiment, the second amino acid or the C-terminal amino acid of the second peptide may be an amino acid that has two or more carbons in its side chain and that is neither a β-branched amino acid nor an α,α-disubstituted amino acid. For example, Lys(Z), Glu(OBzl), and Ser(tBu) correspond amino acids that have two or more carbons in the side chain.

In the present invention, the second amino acid or peptide is used in an equivalent amount or an excessive amount relative to the first amino acid or peptide. Specifically, the molar ratio of the second amino acid or peptide to the first amino acid or peptide can be in a range that can be specified by the combination of a lower limit selected from the group consisting of 1.0, 1.5, 2.0, 3.0, 4.0, 5.0, 6.0, 7.0, 8.0 and 9.0, and an upper limit selected from the group consisting of 2.0, 3.0, 4.0, 5.0, 6.0, 7.0, 8.0, 9.0 and 10.0. Herein, the "lower limit" includes the meanings of both "or more" and "more than", and the "upper limit" includes the meanings of both "or less" and "less than". The molar ratio of the second amino acid or peptide to the first amino acid or peptide is preferably 1.5 or more, and more preferably 2 or more. The molar ratio of the second amino acid or peptide to the first amino acid or peptide is preferably 8 or less, and more preferably 4 or less. The molar ratio of the second amino acid or peptide to the first amino acid or peptide is most preferably about 2.

Examples of the additive used in the condensation reaction of the present invention include Oxyma, HOBt, HOOBt, and HOAt.

In the present invention, the number of moles of the additive used is smaller than the number of moles of the second amino acid or peptide. In other words, in the present invention, the molar ratio of the additive to the second amino acid or peptide is less than 1. The molar ratio is preferably 0.8 or less, such as 0.1 to 0.8. In this case, the molar ratio of the additive to the second amino acid or peptide, for example, can be in a range that can be specified by the combination of a lower limit selected from the group consisting of 0.1, 0.2, 0.3, 0.4, 0.5, 0.6 and 0.7, and an upper limit selected from the group consisting of 0.2, 0.3, 0.4, 0.5, 0.6, 0.7 and 0.8. The molar ratio of the additive to the second amino acid or peptide is more preferably 0.3 to 0.7.

In an embodiment, when the molar ratio of the second amino acid or peptide to the first amino acid or peptide is 1 to 2, the molar ratio of the additive to the second amino acid or peptide is preferably 0.7 or less. Specifically, when the molar ratio of the second amino acid or peptide to the first amino acid or peptide is 1 to 2, the molar ratio of the

additive to the second amino acid or peptide can be in a range of 0.7 or less, a range of 0.6 or less, a range of 0.5 or less, a range of 0.4 or less, a range of 0.3 or less, a range of 0.2 or less, or a range of 0.1 or less.

Alternatively, in the present invention, when the molar ratio of the second amino acid or peptide to the first amino acid or peptide is 1 to 2, the molar ratio of the additive to the second amino acid or peptide can be in a range that can be specified by the combination of a lower limit selected from the group consisting of 0.1, 0.2, 0.3, 0.4, 0.5 and 0.6, and an upper limit selected from the group consisting of 0.2, 0.3, 0.4, 0.5, 0.6, and 0.7.

In another embodiment, when the molar ratio of the second amino acid or peptide to the first amino acid or peptide (this molar ratio may be referred to as the "first molar ratio") is 2 or more, the molar ratio of the additive to the second amino acid or peptide is preferably "(the first molar ratio) minus 1 ((the first molar ratio) - 1)" or less. Specifically, when the molar ratio of the second amino acid or peptide to the first amino acid or peptide is 2 or more, the molar ratio of the additive to the second amino acid or peptide can be in a range of (the molar ratio of the second amino acid or peptide to the first amino acid or peptide) - 1 or less, a range of (the molar ratio of the second amino acid or peptide to the first amino acid or peptide) - 2 or less, a range of (the molar ratio of the second amino acid or peptide to the first amino acid or peptide) - 3 or less, a range of (the molar ratio of the second amino acid or peptide to the first amino acid or peptide) - 4 or less, a range of (the molar ratio of the second amino acid or peptide to the first amino acid or peptide) - 5 or less, a range of (the molar ratio of the second amino acid or peptide to the first amino acid or peptide) - 6 or less, a range of (the molar ratio of the second amino acid or peptide to the first amino acid or peptide) - 7 or less, a range of (the molar ratio of the second amino acid or peptide to the first amino acid or peptide) - 8 or less, or a range of (the molar ratio of the second amino acid or peptide to the first amino acid or peptide) - 9 or less.

Alternatively, in the present invention, when the molar ratio of the second amino acid or peptide to the first amino acid or peptide is 2 or more, the molar ratio of the additive to the second amino acid or peptide can be in a range that can be specified by the combination of a lower limit selected from the group consisting of (the molar ratio of the second amino acid or peptide to the first amino acid or peptide) - 9, (the molar ratio of the second amino acid or peptide to the first amino acid or peptide) - 8, (the molar ratio of the second amino acid or peptide to the first amino acid or peptide) - 7, (the molar ratio of the second amino acid or peptide to the first amino acid or peptide) - 6, (the molar ratio of the second amino acid or peptide to the first amino acid or peptide) - 5, (the molar ratio of the second amino acid or peptide to the first amino acid or peptide) - 4, (the molar ratio of the second amino acid or peptide to the first amino acid or peptide) - 3, and (the molar ratio of the second amino acid or peptide to the first amino acid or peptide) - 2, and an upper limit selected from the group consisting of (the molar ratio of the second amino acid or peptide to the first amino acid or peptide) - 8, (the molar ratio of the second amino acid or peptide to the first amino acid or peptide) - 7, (the molar ratio of the second amino acid or peptide to the first amino acid or peptide) - 6, (the molar ratio of the second amino acid or peptide to the first amino acid or peptide) - 5, (the molar ratio of the second amino acid or peptide to the first amino acid or peptide) - 4, (the molar ratio of the second amino acid or peptide to the first amino acid or peptide) - 3, (the molar ratio of the second amino acid or peptide to the first amino acid or peptide) - 2, and (the molar ratio of the second amino acid or peptide to the first amino acid or peptide) - 1.

In an embodiment, the molar ratio of the additive to the first amino acid or peptide can be 0.5 to 2.0. Specifically, the molar ratio used of the additive to the first amino acid or peptide can be a molar ratio in a range that can be specified by the combination of a lower limit selected from values consisting of 0.5, 0.6, 0.7, 0.8, 0.9, 1.0, 1.1, 1.2, 1.3, 1.4, 1.5, 1.6, 1.7, 1.8 and 1.9 and an upper limit selected from values consisting of 0.6, 0.7, 0.8, 0.9, 1.0, 1.1, 1.2, 1.3, 1.4, 1.5, 1.6, 1.7, 1.8, 1.9 and 2.0. The molar ratio of the additive to the first amino acid or peptide is particularly preferably about 1.

Examples of the condensing agent used in the condensation reaction of the present invention include DIC, DCC, EDCI, and EDCI-HCl.

In the present invention, the number of moles of the condensing agent used is equivalent to or more than the number of moles of the second amino acid or peptide. Specifically, in the present invention, the molar ratio of the condensing agent to the second amino acid or peptide can be in a range of 1 or more, a range of 2 or more, a range of 3 or more, or a range of 4 or more. More specifically, the molar ratio of the condensing agent to the second amino acid or peptide can be in a range that can be specified by the combination of a lower limit selected from the group consisting of 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, and 4.0 and an upper limit selected from the group consisting of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9 and 5.0. Examples of preferable ranges of the molar ratio of the condensing agent to the second amino acid or peptide include 1.0 to 5.0, 1.2 to 4.0, 1.2 to 4.0, 1.2 to 3.0, and 2.0 to 3.0.

In an embodiment, the condensing agent is used in an equivalent amount or an excessive amount relative to the first amino acid or peptide. Specifically, for example, the molar ratio of the condensing agent to the first amino acid or peptide can be in a range of 1.0 or more, a range of 1.1 or more, a range of 1.2 or more, a range of 1.3 or more, a range of 1.4 or more, a range of 1.5 or more, a range of 1.6 or more, a range of 1.7 or more, a range of 1.8 or more, a range of 1.9 or more, a range of 2.0 or more, a range of 2.1 or more, a range of 2.2 or more, a range of 2.3 or more, a range of 2.4 or more, a range of 2.5 or more, a range of 2.6 or more, a range of 2.7 or more, a range of 2.8 or more, a

range of 2.9 or more, a range of 3.0 or more, a range of 3.1 or more, a range of 3.2 or more, a range of 3.3 or more, a range of 3.4 or more, a range of 3.5 or more, a range of 3.6 or more, a range of 3.7 or more, a range of 3.8 or more, a range of 3.9 or more, a range of 4.0 or more, a range of 4.1 or more, a range of 4.2 or more, a range of 4.3 or more, a range of 4.4 or more, a range of 4.5 or more, a range of 4.6 or more, a range of 4.7 or more, a range of 4.8 or more, a range of 4.9 or more, a range of 5.0 or more, a range of 5.1 or more, a range of 5.2 or more, a range of 5.3 or more, a range of 5.4 or more, a range of 5.5 or more, a range of 5.6 or more, a range of 5.7 or more, a range of 5.8 or more, a range of 5.9 or more, a range of 6.0 or more, a range of 6.1 or more, a range of 6.2 or more, a range of 6.3 or more, a range of 6.4 or more, a range of 6.5 or more, a range of 6.6 or more, a range of 6.7 or more, a range of 6.8 or more, a range of 6.9 or more, a range of 7.0 or more, a range of 7.1 or more, a range of 7.2 or more, a range of 7.3 or more, a range of 7.4 or more, a range of 7.5 or more, a range of 7.6 or more, a range of 7.7 or more, a range of 7.8 or more, a range of 7.9 or more, a range of 8.0 or more, a range of 8.1 or more, a range of 8.2 or more, a range of 8.3 or more, a range of 8.4 or more, a range of 8.5 or more, a range of 8.6 or more, a range of 8.7 or more, a range of 8.8 or more, a range of 8.9 or more, a range of 9.0 or more, a range of 9.1 or more, a range of 9.2 or more, a range of 9.3 or more, a range of 9.4 or more, a range of 9.5 or more, a range of 9.6 or more, a range of 9.7 or more, a range of 9.8 or more, or a range of 9.9 or more.

Alternatively, in the present invention, the molar ratio of the condensing agent to the first amino acid or peptide can be in a range that can be specified by the combination of a lower limit selected from the group consisting of 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, and 9.0 and an upper limit selected from the group consisting of 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 and 10.0.

The molar ratio of the condensing agent to the first amino acid or peptide is preferably 1.3 or more, and more preferably 2.0 or more. The molar ratio of the condensing agent to the first amino acid or peptide is preferably 10 or less, and more preferably 8.0 or less. The molar ratio of the condensing agent to the first amino acid or peptide is most preferably about 4.

In the present invention, concerning the molar ratios of the condensing agent and the additive to the second amino acid or peptide,

the molar ratio of the condensing agent to the second amino acid or peptide can be in a range that can be specified by the combination of a lower limit selected from values consisting of 1.0, 1.1, 1.2, 1.3, 1.4, 1.5, 1.6, 1.7, 1.8, 1.9, and 2.0 and an upper limit selected from values consisting of 2.0, 2.1, 2.2, 2.3, 2.4, 2.5, 2.6, 2.7, 2.8, 2.9, and 3.0, and
the molar ratio of the additive to the second amino acid or peptide can be in a range that can be specified by the combination of a lower limit selected from values consisting of 0.01, 0.02, 0.03, 0.04, 0.05, 0.06, 0.07, 0.08, 0.09, 0.1, 0.2, 0.3, 0.4, and 0.5 and an upper limit selected from values consisting of 0.6, 0.7, 0.8, 0.9, and 1.0.

The molar ratios of the condensing agent and the additive to the second amino acid or peptide are preferably the second amino acid or peptide : the condensing agent: the additive = about 2 : about 4 to 6 : about 1.

In the present invention, concerning the molar ratios of the second amino acid or peptide, the condensing agent, and the additive to the first amino acid or peptide,

the molar ratio of the second amino acid or peptide to the first amino acid or peptide can be in a range that can be specified by the combination of a lower limit selected from values consisting of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0 and an upper limit selected from values consisting of 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, and 4.0,
the molar ratio of the condensing agent to the first amino acid or peptide can be in a range that can be specified by the combination of a lower limit selected from values consisting of 1.0, 2.0, 3.0, and 4.0 and an upper limit selected from values consisting of 5.0, 6.0, 7.0, 8.0, and 9.0, and
the molar ratio of the additive to the first amino acid or peptide can be in a range that can be specified by the combination of a lower limit selected from values consisting of 0.1, 0.2, 0.3, 0.4, 0.5, 0.6, 0.7, 0.8, 0.9, and 1.0 and an upper limit selected from values consisting of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0.

The molar ratio of the first amino acid or peptide, the second amino acid or peptide, the condensing agent, and the additive is preferably the first amino acid or peptide : the second amino acid or peptide : the condensing agent : the additive = about 1 : about 2 : about 4 : about 1; the first amino acid or peptide : the second amino acid or peptide : the

condensing agent: the additive = about 1 : about 2.4 : about 7.2 : about 1.2; or the first amino acid or peptide : the second amino acid or peptide : the condensing agent: the additive = about 1 : about 3 : about 6 : about 1.5; and more preferably the first amino acid or peptide : the second amino acid or peptide : the condensing agent: the additive = about 1 : about 2 : about 4 : about 1.

The condensation reaction of the present invention can be carried out at a reaction temperature of 0°C to 100°C, preferably 5°C to 60°C, and more preferably 10°C to 40°C.

The condensation reaction of the present invention can be carried out for a reaction time of 10 minutes to 1 week, preferably 10 minutes to 3 days, and more preferably 1 hour to 2 days.

Preferably, in the present invention, the additive is Oxyma, and the condensing agent is DIC, DCC, EDCI, or EDCI-HCl.

More preferably, in the present invention, the additive is Oxyma, and the condensing agent is DIC.

The condensation reaction of the present invention can be carried out in a suitable solvent. As for the solvent, an aprotic solvent can be used, and examples include amide solvents, ester solvents, ether solvents, alkylnitrile solvents, and urea solvents. Examples of amide solvents include DMF, DMA, and NMP. Examples of ester solvents include ethyl acetate and dimethyl carbonate. Examples of ether solvents include tetrahydrofuran and 2-methyltetrahydrofuran. Examples of alkylnitrile solvents include acetonitrile. Examples of urea solvents include DMI and TMU.

The method of the present invention is applicable to solid phase methods and liquid phase methods.

When carried out by a solid phase method, the method of the present invention can be carried out by bringing the second amino acid or peptide, the condensing agent, and the additive into contact with the first amino acid or peptide linked to the resin. The order for bringing the second amino acid or peptide, the condensing agent, and the additive into contact with the first amino acid or peptide is arbitrary, and the second amino acid or peptide, the condensing agent, and the additive may be simultaneously brought into contact or sequentially brought into contact with the first amino acid or peptide. A mixture obtained by preliminarily mixing all of or any of the second amino acid or peptide, the condensing agent, and the additive may be brought into contact with the first amino acid or peptide. When bringing the second amino acid or peptide, the condensing agent, and the additive into contact with the first amino acid or peptide, mixtures prepared by mixing these with suitable solvents may also be used.

The method of the present invention may be carried out using a solid phase synthesizer. In an embodiment, the method of the present invention can be carried out by mixing the second amino acid or peptide, the condensing agent, and the additive with the first amino acid or peptide linked to a resin in a suitable solvent. When mixing the resin for solid-phase synthesis with these reagents, the resin for solid-phase synthesis can be swollen through contact with a suitable solvent as a pretreatment, thus enabling the intended condensation reaction to proceed efficiently. The amount of the solvent used in this pretreatment can be any amount as long as the swollen resin is immersed in the solvent and, for example, when DMF is used as the solvent, an amount of 3 v/w to 15 v/w, preferably 4 v/w to 10 v/w, and more preferably 4 v/w to 8 v/w can be used. When the amount of solvent is indicated as being 4 v/w, it means that the amount of solvent is 4 mL per 1 g of the resin weight.

After the reaction is complete, the reaction solution is discharged from the solid phase synthesizer, the remaining resin for solid-phase synthesis is washed with a suitable solvent to thereby discharge excessive reagents and by-products, and the intended peptide compound linked to the solid phase synthesis resin can be obtained. Examples of solvents suitable for washing and/or swelling the resin for solid-phase synthesis include amide solvents and alcohol solvents, and DMF or 2-propanol is preferable. These solvents may be used multiple times or may be used alternately. The swollen resin for solid-phase synthesis can be shrunk as necessary, and washing with an alcohol solvent or an ether solvent is used therefor. The alcohol solvent is preferably methanol, and the ether solvent is preferably MTBE.

In the present invention, even when premature cleavage does not concomitantly occur, since the yield of the condensation reaction and the purity of the condensation product can be increased as compared with conventional methods, any resin can be used. Examples of resin linkers with which premature cleavage may occur include those classified as having an acid sensitivity of "H (<5% TFA in $CH_2C1_2$)" as described in the Novabiochem solid phase synthesis handbook. Examples of resins having such resin linkers include resins for solid-phase synthesis in which a trityl atomic group is used as a resin linker (such resins may be referred to as "trityl resins"), such as 2-chlorotrityl chloride resin (CTC resin), TGT resin, trityl chloride resin (Trt resin), 4-methyltrityl chloride resin (Mtt resin), and 4-methoxytrityl chloride resin (Mmt resin), and the present invention is particularly useful when such a resin is used.

The type of a polymer that constitutes the resin for solid-phase synthesis used in the present invention is not particularly limited, and a resin composed of polystyrene is preferable. A solid phase synthesis resin having a particle size of 100 to 200 mesh or 200 to 400 mesh or so can be preferably used. The crosslinking rate of the resin is not particularly limited, and a resin having 1% DVB (divinylbenzene) crosslink is preferable. Examples of the type of the polymer constituting the resin include TentaGel (registered trademark) and ChemMatrix (registered trademark).

In an embodiment, a mixture in which the second amino acid or peptide, the condensing agent, and the additive are preemptively combined in a solvent prior to carrying out the condensation reaction may be prepared in advance, and used in the condensation reaction. The mixing time is not particularly limited, and is preferably 0 minutes to 2 hours,

more preferably 0 minutes to 1 hour, and even more preferably 30 minutes.

Stirring or shaking the resin using an automatic synthesizer may be important for sufficiently impregnating the resin with the reaction solution to cause the reaction to proceed as desired. The stirring speed, shaking speed, and frequency thereof are not particularly limited, but excessive stirring may cause physical damage to the resin and, thus, for example, stirring at 60 rpm may be performed for 2 minutes or so every hour. If impregnation is sufficient, stirring and shaking do not need to be necessarily carried out.

In an embodiment, the method of the present invention may further include the step of removing the resin for solid-phase synthesis, and a method known in the art can be used therefor. The peptide compound that has been elongated to the desired sequence can be detached from the resin and isolated.

In an embodiment, the method of the present invention may further include the step of removing the protecting group, and a method known in the art can be used therefor. Examples include methods described in Greene's, "Protective Groups in Organic Synthesis" (5th edition, John Wiley & Sons 2014), and such methods may be suitably used according to the reaction conditions. Specifically, when the amino group of the second amino acid or the amino group of the N-terminal amino acid of the second peptide is protected with a protecting group, by removing the protecting group, the peptide compound can be ready for the next condensation reaction. The protecting group may be removed simultaneously with the condensation reaction, or may be removed separately from the condensation reaction.

In an embodiment, the present invention also relates to a method of producing a cyclic peptide compound by obtaining a linear peptide compound using the method of the present invention and then cyclizing the group on its N-terminal side and the group on its C-terminal side by a known method described in, for example, Comprehensive Organic Transformations, A Guide to Functional Group Preparations, 3rd Edition (Ed. R. C. Larock) or March's Advanced Organic Chemistry: Reactions, Mechanisms, and Structure, 7th Edition (Ed. M. B. Smith, J. March).

In an embodiment, the present invention also relates to a method of suppressing premature cleavage using the method of the present invention.

All prior art references cited herein are incorporated by reference into this description.

## Examples

In Examples below, analyses were carried out by any one or more of HPLC methods A to D by Waters.

Analysis: HPLC by Waters (Reaction conversion rate, purity)

HPLC method A

Instrument: ACQUITY UPLC H-Class
Column: Ascentis Express C18 (2.7 μm, 4.6 mm × 50 mm), Supelco
Column temperature: 35 deg.
Eluent: A) 0.05% TFA/water, B) 0.05% TFA/MeCN
**Gradient (B): 5% (0 min.) → 100% (4 min.) → 100% (4.5 min.) → 5% (4.6 min.) → 5% (6** min.)
Flow rate: 1.0 mL/min
Detection: PDA 210 nm (200-400 nm PDA total)
Injection volume: **A-a) 0.30 μL, A-b) 1.0 μL, A-c) 2.0 μL**

Sample preparation:

**A-a) 5 μL of supernatant / 995 μL MeCN for loading** evaluation
**A-b) 5 μL of supernatant / 995 μL MeCN for loading evaluation**
**A-c) 40 μL of supernatant** / **960 μL,** MeCN for Gly-cap evaluation

HPLC method B

Instrument: ACQUITY UPLC H-Class
Column: **CAPCELL Core ADME (2.7 μm, 2.1 mm x 50 mm)**

Column temperature: 50 deg.
Eluent: A) 0.05% TFA/water, B) 0.05% TFA/MeCN
**Gradient (B): 30% (0 min.) → 70% (20.0 min.) → 100% (20.1 min.) -+ 100% (22.0 min.) → 30% (22.1 min.) →** 30% (24.0 min.)
Flow rate: 0.3 mL/min
Detection: PDA 210 nm (200-400 nm PDA total)
Injection **volume: 1.0 μL**
Sample **preparation: 40 μL of supernatant / 960 μL MeCN**

HPLC method C

Instrument: ACQUITY UPLC H-Class
Column: ACQUITY UPLC CSH Fluoro-Phenyl **(1.7 μm, 2.1 mm x 150 mm)**
Column temperature: 35 deg.
Eluent: A) 0.1% formic acid/water, B) 0.1% formic acid/MeCN
**Gradient (B): 5% (0 min.) → 100% (15.0 min.) → 100% (18.0 min.) → 5% (18.01 min.) → 5%** (20.0 min.)
Flow rate: 0.3 mL/min
Detection: PDA 254 nm (200-380 nm PDA total)
Injection **volume: 2.0 μL**
Sample preparation: 0.37 mg/mL

HPLC method D

Instrument: ACQUITY UPLC H-Class
**Column: Bioshell A160 peptide (2.7 μm, 2.1 mm x** 150 mm)
Column temperature: 40 deg.
Eluent: A) 0.05% TFA/water, B) 0.05% TFA/MeCN
Gradient (B): 20% (0 min.) → **60% (20 min.) → 100% (20.1 min.) → 100% (22.0 min.) → 100% (22.1 min.) → 20% (24** min.)
Flow rate: 0.5 mL/min
Detection: PDA 254 nm
Injection volume: **2.0 μL**
**Sample preparation: 5 μL of supernatant** / **1000 μL MeCN**

HPLC method E

Instrument: Acquity UPLC/SQD2
Column: Ascentis Express C18
Column temperature: 35 deg.
Eluent: A) 0.1% FA/water, B) 0.1% FA/acetonitrile
**Gradient (B): 5% (0 min.) -+ 100% (1.0 min.) → 100% (1.4 min.)**
Flow rate: 1.0 mL/min
Detection: PDA 210-400 nm (total)

HPLC method F

Instrument: Acquity UPLC/SQD2
Column: Ascentis Express C18
Column temperature: 35 deg.
Eluent: A) 0.1% FA/water, B) 0.1% FA/acetonitrile

Gradient (B): 5% (0 **min.)** → **100% (4.5 min.)** → **100% (5.0 min.)**
Flow rate: 1.0 mL/min
Detection: PDA 210-400 nm (total)

HPLC method G

Instrument: Acquity UPLC/SQD2
Column: Ascentis Express C18
Column temperature: 35 deg.
Eluent: A) 10 mM AA/water, B) methanol
Gradient **(B): 50% (0 min.)** → **100% (4.5 min.)** → **100% (5.0 min.)**
Flow rate: 1.0 mL/min
Detection: PDA 210-400 nm (total)

HPLC method H

Instrument: ACQUITY UPLC H-Class
Column: Ascentis Express **RP-amide (2.7 μm, 2.1 mm x 50 mm), Supelco**
Column temperature: 35 deg.
Eluent: A) 0.05% TFA/water, B) 0.05% TFA/MeCN
Gradient (B): 5% (0 min.) → **100% (4 min.)** → **100% (4.5 min.)** → **5% (4.6 min.)** → **5% (6** min.)
Flow rate: 0.5 mL/min
Detection: PDA 210 nm (200-400 nm PDA total)
Injection volume: **E-a) 0.50 μL,** E-b) 0.50 **μL, E-c) 1.0 μL**

Sample preparation:

**E-a) 5 μL of supernatant** / **960 μL MeCN for** Gly-cap evaluation or purity evaluation

HPLC method I

Instrument: ACQUITY UPLC H-Class
Column: Ascentis Express **RP-amide (2.7 μm, 2.1 mm × 50 mm), Supelco**
Column temperature: 50 deg.
Eluent: A) 0.05% TFA/water, B) 0.05% TFA/MeCN
Gradient (B): 5% (0 **min.)** → **70% (20 min.)** → **70% (21 min.)** → **100% (22 min.)** → **100% (22.1 min.)** → **5% (22.6 min.)** → 5% (24.0 min.)
Flow rate: 0.5 mL/min
Detection: PDA 210 nm (200-400 nm PDA total)
Injection **volume: 0.50 or 1.0 μL**
**Sample preparation: 40 μL of supernatant / 960 μL MeCN**

HPLC method J

Instrument: ACQUITY UPLC H-Class
Column: ACQUITY UPLC CSH Fluoro-Phenyl (1.7 μm, 2.1 mm x 150 mm)
Column temperature: 40 deg.
Eluent: A) 0.05% TFA/water, B) 0.05% TFA/MeCN
**Gradient (B): 20% (0 min.)** → **100% (20 min.)** → **20% (20.1 min.)** → **12% (24.0 min.)**
Flow rate: 0.3 mL/min

Detection: PDA 254 nm (200-400 nm PDA total)
**Injection volume: 1.0 μL**
**Sample preparation: 40 μL of supernatant / 960 μL MeCN**

The following Fmoc amino acids were used by purchasing commercially available ones.

[Table A]

| Abbreviation | Structure | Abbreviation | Structure |
|---|---|---|---|
| **Fmoc-Ile-OH** | 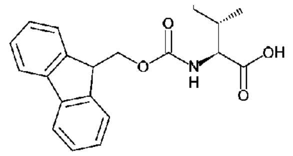 | **Fmoc-MeChg-OH** | 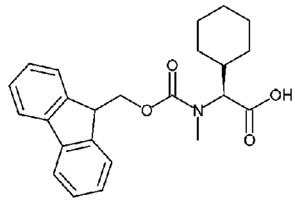 |
| **Fmoc-MeVal-OH** | 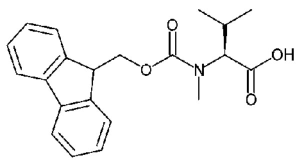 | **Fmoc-MeLeu-OH** | 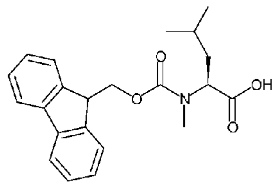 |
| **Fmoc-D-MeVal-OH** | 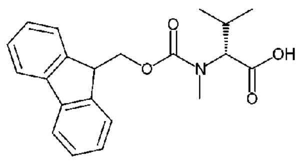 | **Fmoc-MeGly-OH** | 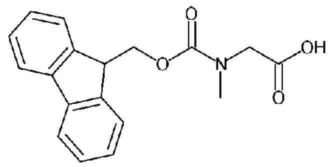 |
| **Fmoc-MeIle-OH** | 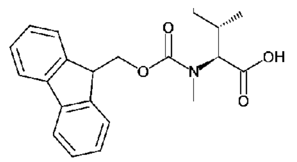 | **Fmoc-D-3-MeAbu-OH** | 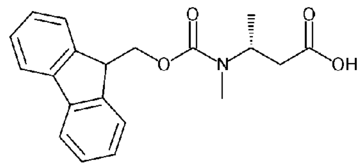 |
| **Fmoc-MeGly (cPent)-OH** | 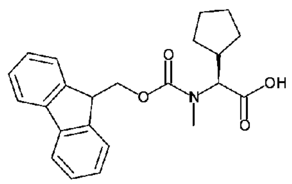 | **Fmoc-cLeu-OH** | 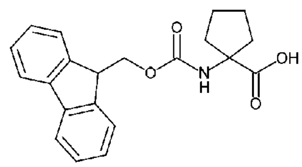 |
| **Fmoc-Glu (OBzl)-OH** | 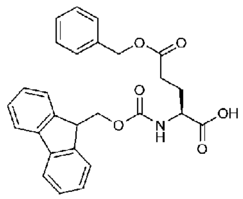 | **Fmoc-Aze (2)-OH** | 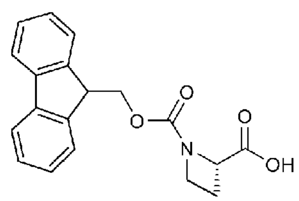 |
| **Fmoc-Thr(tBu)-OH** | 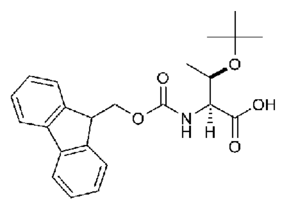 | **Fmoc-cVal-OH** | 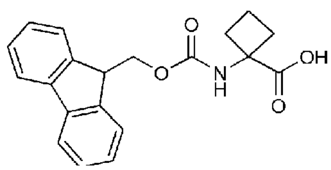 |

(continued)

| Abbreviation | Structure | Abbreviation | Structure |
|---|---|---|---|
| **Fmoc-MeAla-OH** | 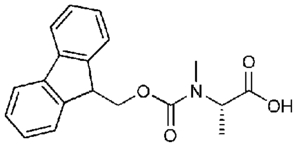 | **Fmoc-Aib-OH** | 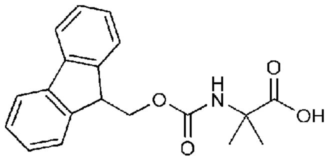 |
| **Fmoc-bAla-OH** | 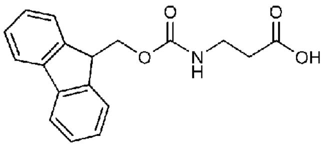 | **Fmoc-bMeAla-OH** | 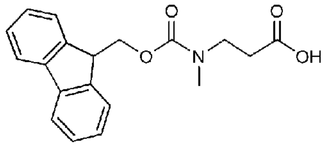 |
| **Fmoc-MeMet-OH** | 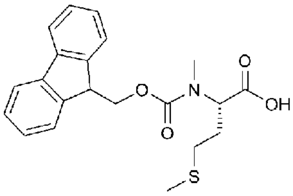 | | |

[Liquid-phase Synthesis Experiment]

[Synthesis of Starting Material]

(Synthesis of Starting Material 1)

Synthesis of Fmoc-MeAsp(OtBu)-pip (Compound 2)

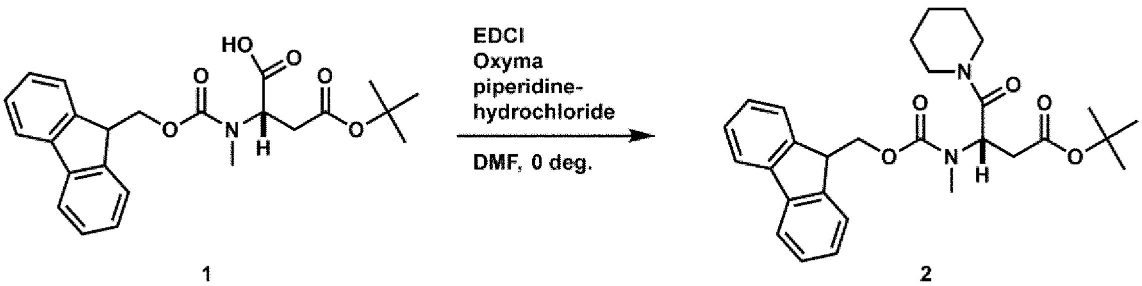


1 2

First, 26.9 g (63.2 mmol, 1.0 eq) of Fmoc-MeAsp(OtBu)-OH (Compound 1), 9.23 g (75.9 mmol, 1.2 eq) of piperidine hydrochloride, 10.8 g (69.5 mmol, 1.1 eq) of Oxyma, and 135 mL of N,N-dimethylformamide were added to a 500 mL three-neck flask, and stirred. Using a dripping funnel, 12.2 mL (69.5 mmol, 1.1 eq) of 1-ethyl-3-(3-dimethylaminopropyl)carbodiimide was added dropwise at 0°C over 10 minutes. After dropwise addition, the dripping funnel was washed twice with 1.25 mL of N,N-dimethylformamide. Five hours after the beginning of the reaction, 269 mL of 2-MeTHF was added, and then 135 mL of a 0.5 mol/L aqueous hydrochloric acid solution was added over 10 minutes. After discharging the aqueous layer, 135 mL of a 5% aqueous sodium carbonate solution was added to the organic layer. After discharging the aqueous layer, 135 mL of a 10% aqueous sodium chloride solution was added to the organic layer. After discharging the aqueous layer, the organic layer was stored overnight at room temperature. After about 60 mL of the organic solvent was removed under reduced pressure, 81 mL of ethyl acetate was added to the concentrate at room temperature. Then, 323 mL of heptane was added while stirring. The external temperature was set to 0°C, and after 1 hour and 20 minutes, 161 mL of heptane was added. After crystals were filtered off and washed using a Kiriyama funnel, the resulting crystals were dried under reduced pressure at an external temperature of 40°C to give 28.2 g of a

white solid (Compound 2).

[Table 2]

| | Mass (g) | Yield (%) |
|---|---|---|
| Starting material (Compound 1) | 26.9 | - |
| Product (Compound 2) | 28.2 | 90.5 |

[Table 3]

| Analysis (HPLC method C: Purity determination) | | | | |
|---|---|---|---|---|
| | Mw | m/z | Rt (min) | LC A% |
| Starting material (Compound 1) | 425.2 | - | - | - |
| Product (Compound 2) | 492.3 | * | 10.3 | 99.3 |
| * LCMS peak of product was confirmed when removing t-Bu.<br>See Synthesis Example of Compound 3. | | | | |

(Synthesis of Starting Material 2)

Synthesis of Fmoc-MeAsp(OH)-pip (Compound 3)

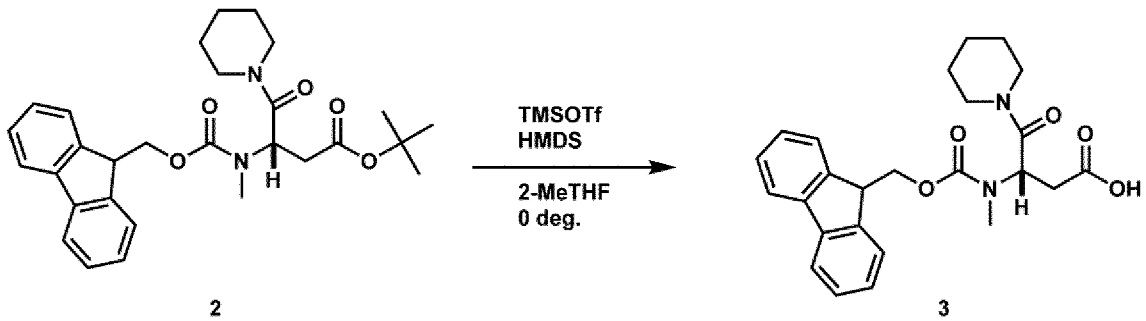


2 3

After 1.58 g (3.20 mmol, 1.0 eq) of Fmoc-MeAsp(OtBu)-pip (Compound 2) was weighed into a 100 mL three-neck recovery flask, under a nitrogen atmosphere, 9.5 mL of 2-MeTHF was added with a syringe and stirred, and 1.0 mL (4.8 mmol, 1.5 eq) of HMDS was added with a syringe. The flask was immersed in an ice bath, and 0.79 mL (4.4 mmol, 1.4 eq) of TMSOTf was added with a syringe when the internal temperature reached 1°C. The temperature was increased to room temperature 5 minutes after the completion of dropwise addition, and 1 hour later, the reaction rate was confirmed as being 100% by HPLC (method A-a). The reaction vessel was immersed in an ice bath again, and when the internal temperature reached 5°C, 15 mL of a 5% aqueous sodium carbonate solution was added. After completing the addition of the aqueous sodium carbonate solution, the reaction solution was stirred for several minutes, and then stirring was terminated. The reaction vessel was transferred to a 50 mL separatory funnel and separated into layers, and then the aqueous layer was collected. The resulting aqueous layer was washed with 15 mL of MTBE. After 15 mL of MTBE was added to the collected aqueous layer, 1.1 mL of phosphoric acid (a mass fraction of 85% or more) was added. After the solution was shaken in a separatory funnel, the solution was separated into layers to collect the organic layer. After magnesium sulfate was added to the resulting organic layer, magnesium sulfate was filtered off. Then, 1.5 mL of heptane was added to the filtrate, and the mixture was concentrated under reduced pressure to give 1.42 g of a white solid (Compound 3) (content not measured).

[Table 4]

| | Mass (g) | Yield (%) |
|---|---|---|
| Starting material (Compound 2) | 1.58 | - |
| Product (Compound 3) | 1.42 | Quantitative |

[Table 5]

| Analysis (HPLC method A-a: Product identification and purity determination*) | | | | |
|---|---|---|---|---|
| | Mw | m/z | Rt (min) | LC A% |
| Starting material (Compound 2) | 492.6 | 493.3 ([M+H]$^+$) | 3.2 | 99.4 |
| Product (Compound 3) | 436.5 | 437.3 ([M+H]$^+$) | 2.4 | 99.9 |
| *LC A% was calculated by excluding the peak of stabilizer (2,6-di-t-butyl-p-crezol) contained in a 2-MeTHF medium. | | | | |

(Synthesis of Starting Material 3)

Synthesis of Fmoc-MeAsp(OH)-NMe2 (Compound 21)

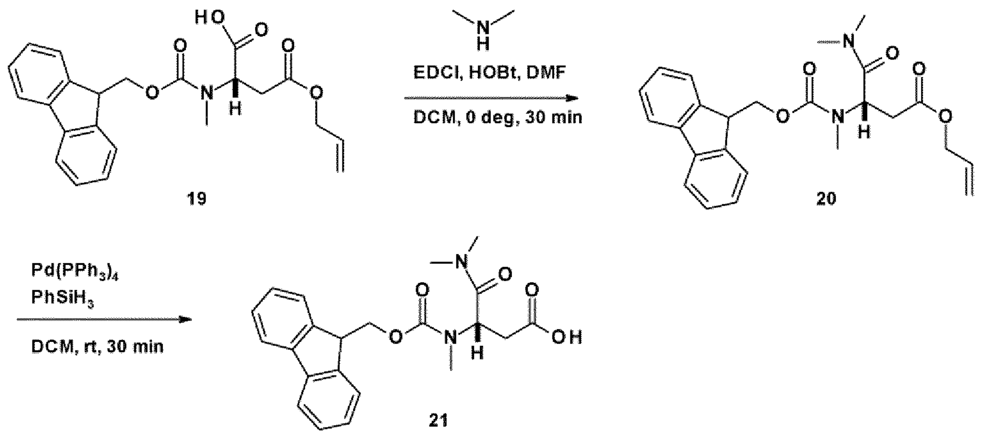


19

20

21

Under a nitrogen stream, EDCI (16.9 g, 88.0 mmol) and DMF (144 mL) were added to a reaction vessel, and cooled to 0°C. HOBt (10.9 g, 80.6 mmol) and a solution of Compound 19 (30.0 g, 73.3 mmol) synthesized according to the method described in WO 2018/225864 and dissolved in DCM/DMF (60 mL/60 mL) were successively added at 0°C, and the mixture was stirred at 0°C for 30 minutes. Dimethylamine (2 mol/L THF solution, 40.5 mL, 80.6 mmol) was added dropwise at 0°C, and the mixture was stirred at 0°C for 30 minutes. Ethyl acetate (300 mL) was added to the reaction solution, the organic layer was successively washed with a 1 mol/L aqueous hydrochloric acid solution (240 mL) twice, with water (300 mL) twice, with a saturated aqueous sodium hydrogen carbonate solution/water (1/1, 300 mL) twice and with a saturated aqueous sodium chloride solution/water (1/1, 300 mL), and then the organic layer was dried over sodium sulfate. After the desiccant was filtered off, the filtrate was concentrated under reduced pressure to give 32.7 g (yield 102%) of Compound 20 as light brown amorphous crystals.

LCMS(ESI) m/z = 437.2 (M+H)$^+$

Retention time: 0.86 min (Analytical condition HPLC METHOD E)

Compound 20 (32.0 g, 73.3 mmol), used as a starting material, was mixed with tetrakistriphenylphosphine palladium and DCM, then phenylsilane was added dropwise, and thereafter the reaction solution was stirred at room temperature for 30 minutes. An extraction operation for the intended product was carried out, and the solution containing the intended product was concentrated to dryness to thereby give 25.1 g (yield 86%) of Compound 21 as light brown amorphous crystals.

LCMS(ESI) m/z = 397.2 $(M+H)^+$

Retention time: 0.68 min (Analytical condition HPLC METHOD E)

Cl-Trt(2-Cl) resin (1.25 to 1.60 mmol/g, 100 to 200 mesh, 1% DVB) was purchased from Watanabe Chemical Industries Ltd. or SUNRESIN.

[Solid phase synthesis experiment]

Herein, when a solid phase support and a compound are linked, a polymer or resin moiety may be depicted as ●. In addition, to clarify the point of reaction with the solid phase support moiety, the chemical structure of a linker, which is the reaction site, may be depicted as being attached to ●. For example, in the following structure (Fmoc-MeAsp(O-Trt(2-Cl)-resin)pip (Compound 3), the 2-chlorotrityl group of the solid phase support forms an ester bond with the side chain carboxy group of MeAsp. The "pip" denotes a piperidino group and, in the structure, the C-terminal carboxy group forms an amide bond with piperidine.

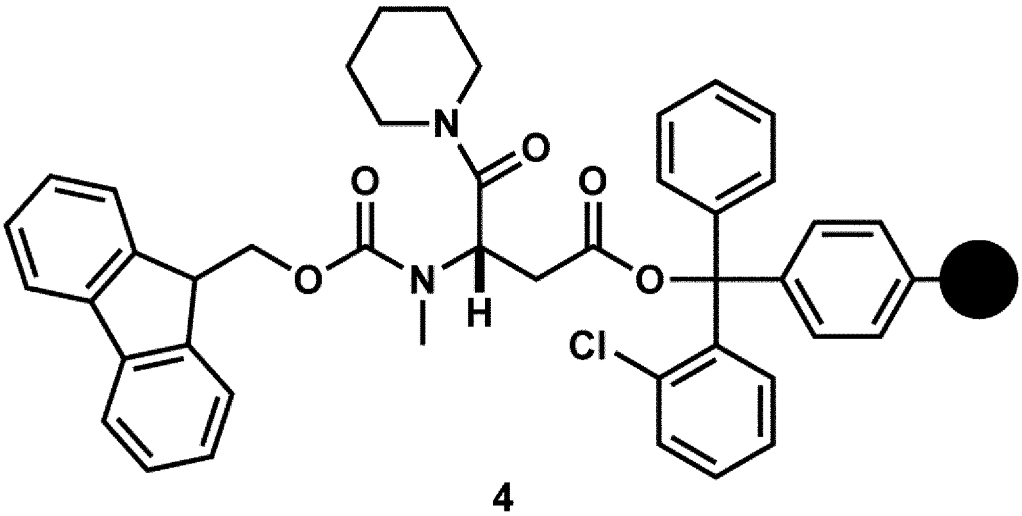

**4**

[One-residue + one-residue condensation experiment]

Herein, the amount of solvent when carrying out a solid phase reaction may be expressed in multiple (v/w). The standard for multiple is the mass of Cl-Trt(2-Cl) resin in the loading step and, for example, in Synthesis of Solid phase raw material 1, when 40 mL of dichloromethane is used relative to 3.97 g of Cl-Trt(2-Cl) resin, it is 40/3.97 ≈ 10 v/w. In the one-residue + one-residue condensation experiment, the resin intermediate obtained in the loading step was divided into small portions and used. The amount of solvent in the one-residue + one-residue condensation experiment is 4 v/w, which, in terms of volume, corresponds to 4 (mL/g) × divided resin (g) / resin after loading (g) × loading-step raw material resin (g) = 4 (mL/g) × divided resin (g) / 5.79 × 3.97 mL.

Herein, as for the assessment of purity in the solid phase condensation reaction, the reaction solution after the deresination reaction using the dry resin obtained after elongation was measured by LC to verify the purity of the compound loaded onto the resin for solid-phase synthesis. Specific procedure will now be shown. About 20 mg of a dry resin after elongation was placed in a 5 mL filter-equipped disposable syringe, dichloromethane was aspirated with a 1 mL disposable syringe, and this was left to stand at room temperature for 15 minutes. Dichloromethane was discharged, 0.20 mL of dichloromethane containing 1% TFA was added, and the mixture was shaken at room temperature for 30 seconds. The solution was discharged into a 1 mL vial. Then, 40 μL of the discharged solution was diluted with 960 μL of acetonitrile to prepare a LC sample solution.

In Examples other than Examples 2-2 to 2-11 (Examples 2-1 and 2-12 to 2-31) herein, the amount of substance (mmol) of the target molecule was calculated by multiplying the Fmoc quantitative value (mmol/g), the weight of dry resin (g), and LC purity (area%) to verify the yield of the solid phase condensation reaction. The specific procedure of Fmoc quantification will now be shown. About 20 mg of a dry resin was weighed into a 100 mL volumetric flask, and then an N,N-dimethylformamide solution containing 20% piperidine was added so as to adjust the volume. This solution was shaken at room temperature for 45 minutes. The absorbance (wavelength 289.80 nm, coefficient ε = 6089) of the solution was measured using a spectrophotometer (UV3900-01), and the amount of substance per unit weight of a compound

having an Fmoc group and loaded onto a resin was calculated from the following formula (Fmoc quantification):

$$\text{Molar concentration of measurement solution (mol/L)} = \text{Absorbance} / \varepsilon$$

$$\text{Fmoc quantitative value (mmol/g)} = \text{Molar concentration of measurement solution (mol/L)} / \text{Resin for measurement (g)} \times 100 \text{ (mL)}$$

In Examples 2-2 to 2-11 herein, the following Fmoc quantification method was used.

Fmoc quantification method (Example 2-2 to Example 2-11)

About 10 mg of commercially available Fmoc-Gly-OH was weighed into a 10 mL volumetric flask, and then DMF (4 mL) was added. After the mixture was shaken at room temperature for 30 minutes, DBU (40 μL) was added, and this solution was shaken at room temperature for 15 minutes. Then, DMF was added so as to adjust the volume to 10 mL. A sample solution was prepared from 30 μL of the solution supernatant and 4 mL of a DMF solution, and LC data was acquired (5 μL injection). Calibration curves were created at wavelengths of 294 nm and 304 nm, respectively, from the acquired data.

About 10 mg of the resin after reaction was weighed into a 10 mL volumetric flask, and then DMF (4 mL) was added. After the mixture was shaken at room temperature for 30 minutes, DBU (40 μL) was added, and this solution was shaken at room temperature for 15 minutes. Then, DMF was added so as to adjust the volume to 10 mL. A sample solution was prepared from 80 μL of the solution supernatant and 920 μL of a DMF solution, and LC data was acquired (5 μL injection). From the areas of the acquired data and the created calibration curves concerning the respective wavelengths, the amount of substance per unit weight of a compound having an Fmoc group and loaded onto the resin was calculated, and the average value thereof was used as an Fmoc quantitative value.

[Synthesis of Starting Material]

(Synthesis of Solid phase raw material 1)

Loading of Fmoc-MeAsp(OH)-pip (Compound 3) onto Trt(2-Cl) resin

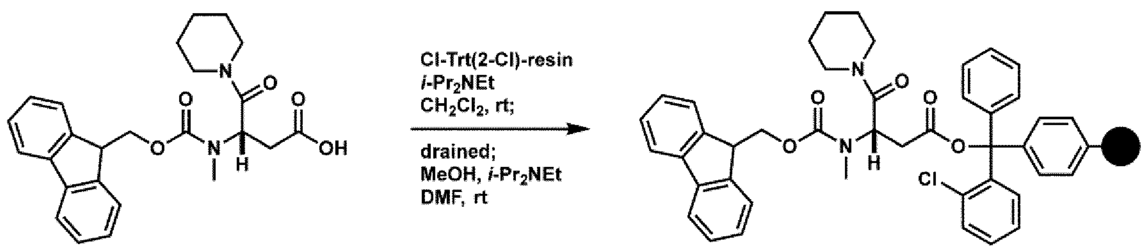


3 4

First, 3.97 g (1.36 mmol/g, 5.40 mmol, 1.7 eq) of Cl-Trt(2-Cl) resin was weighed into a solid phase reaction column, 40 mL (10 v/w) of dichloromethane was added, and the mixture was left to stand at room temperature for 30 minutes. After dichloromethane was discharged, a solution obtained by dissolving 1.40 g (3.20 mmol, 1.0 eq) of Fmoc-MeAsp(OH)-pip (Compound 3) and 1.6 mL (9.0 mmol, 2.8 eq) of N,N-diisopropylethylamine in 30 mL (7.5 v/w) of dichloromethane was added to the reaction column at room temperature, and the mixture was shaken at room temperature for 2 hours. After the reaction solution was discharged, a solution obtained by dissolving 3.2 mL (0.8 v/w) of methanol and 1.6 mL (0.4 v/w) of N,N-diisopropylethylamine in 27 mL (6.8 v/w) of N,N-dimethylformamide was added to the reaction column at room temperature. After the mixture was shaken at room temperature for 2 hours, the reaction solution was discharged. The resin was washed with 40 mL (10 v/w) of N,N-dimethylformamide four times, with 40 mL (10 v/w) of 2-propanol twice and with 40 mL (10 v/w) of methanol four times, and then dried under reduced pressure overnight at room temperature to give 5.79 g of a dry resin (Compound 4). Then, 19.4 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance

of the solution using a spectrophotometer, and the yield was calculated from the resulting Fmoc quantitative value, the mass of the dry resin, and LC A%.

[Table 6]

| | Mass (g) | Fmoc quantitative value (mmol/g) | LC A% | Yield (%)* |
|---|---|---|---|---|
| CTC resin | 3.97 | - | | - |
| Product (Compound 4) | 5.79 | 0.525 | 99.0 | 93.9 |
| * Two-stage yield including t-Bu removing step (calculated assuming the content of (Compound 2) being 100%) | | | | |

[Table 7]

| Analysis (HPLC method B: Product identification and purity determination) | | | | |
|---|---|---|---|---|
| | Mw | m/z | Rt (mi n) | LC A% |
| Product (Compound 3)* | 436.2 | 437.3 ([M+H]$^+$) | 6.8 | 99.0 |
| * Deresinated Compound 3 was analyzed to verify change in purity before and after loading | | | | |

(Synthesis of Solid phase raw material 2)

Loading of Fmoc-MeAsp(OH)-NMe2 (Compound 21) onto Trt(2-Cl) resin

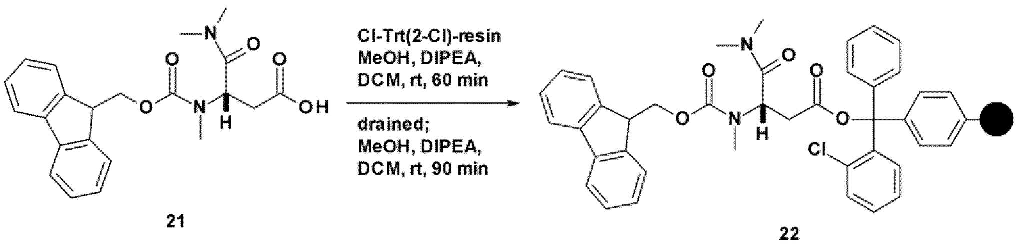


21

22

Cl-Trt(2-Cl) resin (1.60 mmol/g, 8.83 g, 14.1 mmol) and DCM (90 mL) were introduced into a filter-equipped reaction vessel, and the mixture was shaken at room temperature for 20 minutes. After a nitrogen pressure was applied to remove DCM, a mixed solution of Compound 21 (2.80 g, 7.06 mmol), methanol (2.29 mL, 56.5 mmol), DIPEA (5.91 mL, 33.9 mmol), and DCM (64 mL) was added to the reaction vessel, and the mixture was shaken at room temperature for 60 minutes. After a nitrogen pressure was applied to remove the reaction solution, a mixed solution of methanol (9.17 mL, 226 mmol), DIPEA (5.91 mL, 33.9 mmol), and DCM (64 mL) was added to the reaction vessel, and the mixture was shaken at room temperature for 90 minutes. After a nitrogen pressure was applied to remove the reaction solution, DCM (90 mL) was introduced, the mixture was shaken for 5 minutes, and then a nitrogen pressure was applied to remove the reaction solution. This resin washing operation using DCM was repeated twice, and the resulting resin was dried under reduced pressure overnight to give 10.4 g of Compound 22. Using the dry resin (11.04 mg), the loaded ratio was calculated by the Fmoc quantification method and was 0.442 mmol/g (UVarea value at 294 nm: 4990.63, UVarea value at 304 nm: 4516.89).

(Synthesis of Solid phase raw material 3)

Loading of Fmoc-D-3-MeAbu-OH (Compound 23) onto Trt(2-Cl)resin

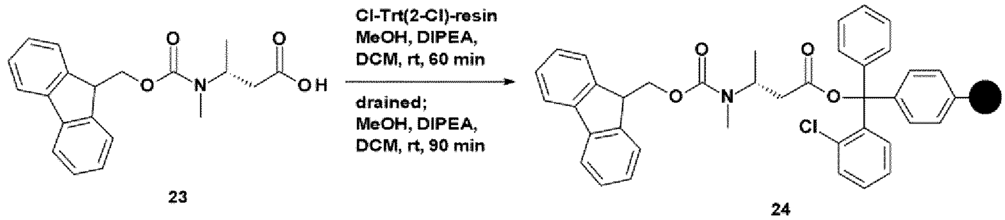


23 24

Cl-Trt(2-Cl) resin (1.60 mmol/g, 25.0 g, 40.0 mmol) and DCM (125 mL) were introduced into a filter-equipped reaction vessel, and the mixture was shaken at room temperature for 20 minutes. After a nitrogen pressure was applied to remove DCM, a mixed solution of Fmoc-D-3-MeAbu-OH (Compound 23, 3.60 g, 10.6 mmol), methanol (0.859 mL, 21.2 mmol), and DIPEA (12.3 mL, 70.7 mmol) adjusted to be total 145 mL by adding DCM was added to the reaction vessel, and the mixture was shaken at room temperature for 30 minutes. After a nitrogen pressure was applied to remove the reaction solution, a mixed solution of methanol (12.5 mL, 143 mmol) and DIPEA (12.5 mL, 71.8 mmol) adjusted to be total 250 mL by adding DCM was added to the reaction vessel, and the mixture was shaken at room temperature for 90 minutes. After a nitrogen pressure was applied to remove the reaction solution, DCM (180 mL) was introduced, the mixture was shaken for 5 minutes, and then a nitrogen pressure was applied to remove the reaction solution. This resin washing operation using DCM was repeated three times, and the resulting resin was dried under reduced pressure overnight to give 28.3 g of Compound 24. Using the dry resin (10.36 mg), the loaded ratio was calculated by the Fmoc quantification method and was 0.369 mmol/g (UVarea value at 294 nm: 3920.38, UVarea value at 304 nm: 3530.84).

(Synthesis of Solid phase raw material 4)

Loading of Fmoc-MeGly-OH (Compound 25) onto Trt(2-Cl) resin

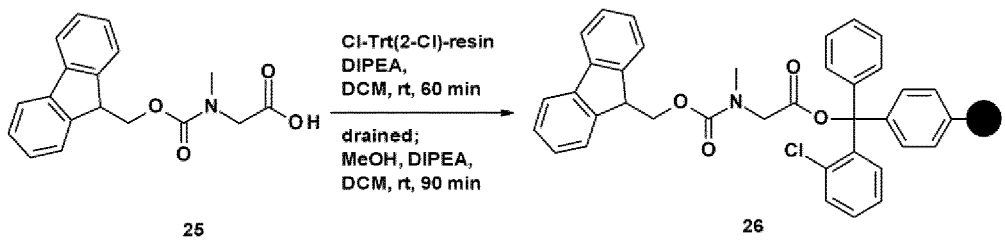


25 26

Cl-Trt(2-Cl) resin (1.60 mmol/g, 12.3 g, 19.7 mmol) and DCM (125 mL) were introduced into a filter-equipped reaction vessel, and the mixture was shaken at room temperature for 20 minutes. After a nitrogen pressure was applied to remove DCM, a mixed solution of Fmoc-MeGly-OH (3.07 g, 9.87 mmol) purchased from a commercial supplier, DIPEA (8.25 mL, 47.4 mmol), and DCM (110 mL) was added to the reaction vessel and shaken at room temperature for 60 minutes. After a nitrogen pressure was applied to remove the reaction solution, a mixed solution of methanol (12.8 mL, 316 mmol), DIPEA (8.25 mL, 47.4 mmol), and DCM (110 mL) was added to the reaction vessel, and the mixture was shaken at room temperature for 90 minutes. After a nitrogen pressure was applied to remove the reaction solution, DCM (180 mL) was introduced, the mixture was shaken for 5 minutes, and then a nitrogen pressure was applied to remove the reaction solution. This resin washing operation using DCM was repeated twice, and the resulting resin was dried under reduced pressure overnight to give 22.2 g of Compound 26. Using the dry resin (10.00 mg), the loaded ratio was calculated by the Fmoc quantification method and was 0.573 mmol/g (UVarea value at 294 nm: 5879.66, UVarea value at 304 nm: 5289.40).

(Synthesis of Solid phase raw material 5)

Loading of Fmoc-MeLeu-OH (Compound 37) onto Trt(2-Cl) resin

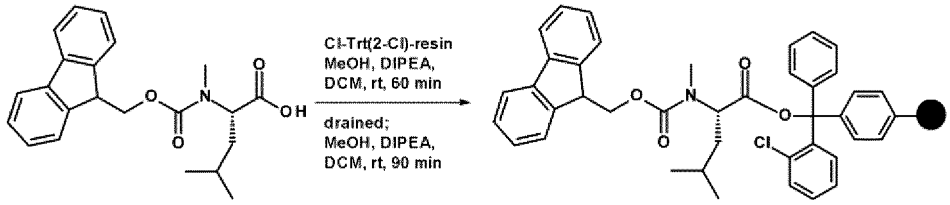


37 38

First, 1.01 g (1.13 mmol/g, 1.14 mmol, 1.4 eq) of Cl-Trt(2-Cl) resin was weighed into a solid phase reaction column, 10 mL (10 v/w) of dichloromethane was added, and the mixture was left to stand at room temperature for 30 minutes. After dichloromethane was discharged, a solution obtained by dissolving 0.29 g (0.79 mmol, 1.0 eq) of Fmoc-MeLeu-OH (Compound 1) and 0.40 mL (2.3 mmol, 2.9 eq) of N,N-diisopropylethylamine in 8.0 mL (8 v/w) of dichloromethane was added to the reaction column at room temperature, and then the mixture was shaken at room temperature for 2 hours. After the reaction solution was discharged, a solution obtained by dissolving 0.80 mL (0.8 v/w) of methanol and 0.40 mL (0.4 v/w) of N,N-diisopropylethylamine in 6.8 mL (6.8 v/w) of N,N-dimethylformamide was added to the reaction column at room temperature. After the mixture was shaken at room temperature for 2 hours, the reaction solution was discharged. The resin was washed with 10 mL (10 v/w) of N,N-dimethylformamide four times, with 10 mL (10 v/w) of 2-propanol twice and with 10 mL (10 v/w) of methanol four times, and then dried under reduced pressure overnight at room temperature to give 1.27 g of a dry resin (Compound 38). Then, 17.7 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance of the solution using a spectrophotometer, and the yield was calculated from the resulting Fmoc quantitative value, the mass of the dry resin, and LC A%.

[Table 8]

| | Mass (g) | Fmoc quantitative value (mmol/g) | LC A% | Yield (%) |
|---|---|---|---|---|
| CTC resin | 1. 01 | - | | - |
| Product (Compound 38) | 1. 27 | 0. 650 | 100 | 102 |

[Table 9]

| Analysis (HPLC method H: Purity determination) | | |
|---|---|---|
| | Rt (min) | LC A% |
| Deresinated product (Compound 37) | 3.1 | 100 |

The results of loading the following commercially available amino acids onto CTC resin, with the same reaction conditions as above being applied, are shown below.

[Table 10]

| Entry | Amino acid | Fmoc quantitative value (mmol/g) | LC A% | Yield (%) |
|---|---|---|---|---|
| 1 | MeVal | 0.651 | 100 | 100 |
| 2 | MeAla | 0.649 | 100 | 99 |
| 3 | MeGly | 0.628 | 99.7 | 95 |
| 4 | MePhe | 0.616 | 99.6 | 99 |
| 5 | MeTrp (Boc) | 0.611 | 96.9 | 102 |
| 6 | MeThr (Bzl) | 0.607 | 98.0 | 97 |
| 7 | MeGlu (0tbu) | 0.603 | 99.3 | 96 |
| 8 | MeLys (Boc) | 0.600 | 99.1 | 101 |

(continued)

| Entry | Amino acid | Fmoc quantitative value (mmol/g) | LC A% | Yield (%) |
|---|---|---|---|---|
| 9 | MeMet | 0.650 | ND | 103 |
| 10 | Aze (2) | 0.642 | 99.8 | 95 |
| 11 | Gly | 0.664 | 100 | 99 |
| 12 | Leu | 0.639 | 99.9 | 98 |
| 13 | Aib | 0.652 | 100 | 98 |
| 14 | bAla | 0.669 | 100 | 99 |
| 15 | bMeAla | 0.582 | ND | 99 |
| 16 | D-3-MeAbu | 0.607 | ND | 98 |
| 17 | MeLeu* | 0.660 | 100 | 102 |
| ND: no data<br>*Data when performing re-synthesis is described. | | | | |

In Example 1, Example 2-1, and Examples 2-12 to 2-31, the amino acid elongation was performed using 2 eq, 4 eq, and 1 eq of Fmoc amino acid, DIC, and Oxyma, respectively, relative to the amino acid onto a resin for solid-phase synthesis (Example Condition A).

[Example 1]

(One-residue + one-residue condensation experiment)

Synthesis of Fmoc-MeVal-MeAsp(OCTC)-pip (Compound 5) (Reaction condition having Fmoc amino acid : DIC : Additive (Oxyma) = 2 : 4 : 1 relative to amino acid onto resin: hereinafter referred to as Example Condition A)

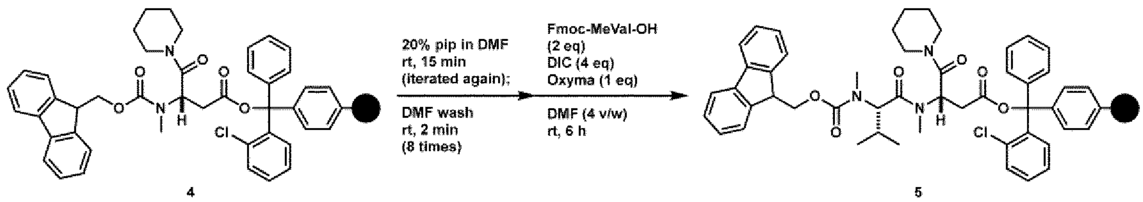


4 5

First, 3.01 g (0.525 mmol/g, 1.57 mmol, 1.0 eq) of a dry resin (Compound 4) was weighed into a solid phase reaction column, 21 mL (10 v/w) of N,N-dimethylformamide was added, and the mixture was left to stand at room temperature for 30 minutes. After N,N-dimethylformamide was discharged, 17 mL (8 v/w) of an N,N-dimethylformamide solution containing 20% piperidine was added, and the mixture was shaken at room temperature for 15 minutes. After the reaction solution was discharged, 17 ml (8 v/w) of an N,N-dimethylformamide solution containing 20% piperidine was added again, and the mixture was shaken at room temperature for 15 minutes. After the reaction solution was discharged, the resin was washed with 21 mL (10 v/w) of N,N-dimethylformamide eight times. Next, 1.12 g (3.16 mmol, 2.0 eq) of Fmoc-MeVal-OH and 0.226 g (1.58 mmol, 1.0 eq) of Oxyma were weighed into a vial and dissolved by adding 8.2 mL (4 v/w) of N,N-dimethylformamide, and this solution was added to a solid phase synthesis column. Subsequently, 0.99 mL (6.3 mmol, 4.0 eq) of DIC was added, and the column was sealed and then shaken at room temperature for 6 hours. After the reaction solution was discharged, the resin was washed with 21 mL (10 v/w) of N,N-dimethylformamide four times, with 21 mL (10 v/w) of 2-propanol twice, and with 21 mL (10 v/w) of methanol four times, and then dried under reduced pressure at room temperature overnight to give 2.78 g of a dry resin (Compound 5). Then, 23.2 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification from the absorbance of the solution was carried out using a spectrophotometer, and the yield was calculated from the resulting Fmoc quantification value, the mass of the dry resin, and LC A%.

[Table 11]

| (HPLC method B: Identification of target molecule and purity measurement) | | | | | |
|---|---|---|---|---|---|
| | Mass (g) | Fmoc quantitative value (mmol/g) | LC A% | Amount of substance (mmol) | Yield (%) |
| Starting material (Compound 4) | 3.01 | 0.525 | 99.0 | 1.57 | - |
| Product (Compound 5) | 2.78 | 0.532 | 96.1 | 1.42 | 90.8 |

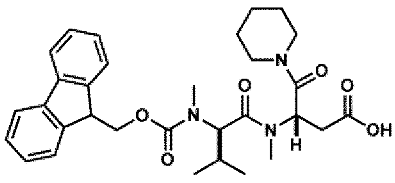
6

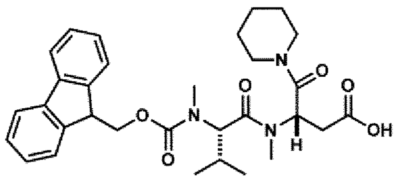
7

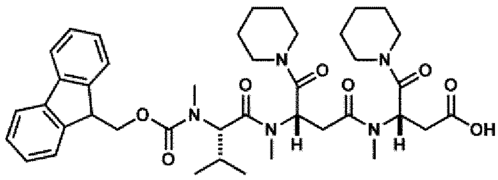
8

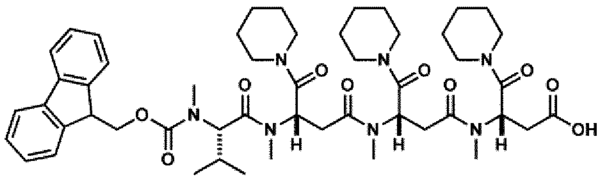
9

[Table 12]

| | Mw | m/z | Rt (mi n) | LC A% |
|---|---|---|---|---|
| Epimer (Compound 6) | 549.7 | 550.4 ([M+H]$^+$) | 9.0 | 0.4 |
| Target molecule (Compound 7) | 549.7 | 550.5 ([M+H]$^+$) | 9.3 | 96.1 |
| Excessively elongated product (Compound 8, | 745.9 | 746.6 ([M+H]$^+$) | 12.2 | 2.3 |
| Excessively elongated product (Compound 9) | 942.2 | Not detected | Not detected | Not detected |

The target molecule (Compound 7) could be obtained in a high yield. An excessively elongated product (Compound 8) and an epimer (Compound 6) were produced in 2.3 A% and 0.4 A%, respectively.

[Example 2-1]

<u>Synthesis of Fmoc-D-MeVal-MeAsp(OCTC)-pip (Compound 10) (Synthesis by Example Condition A)</u>

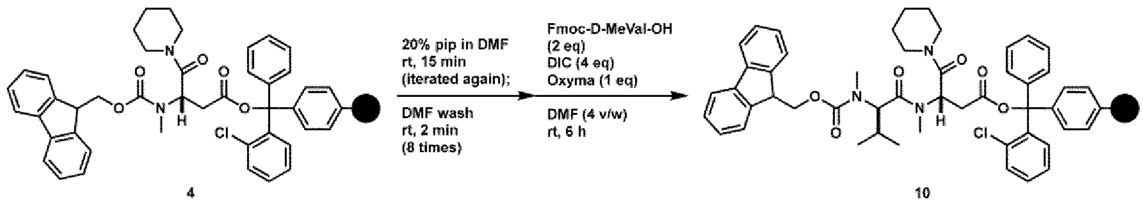

4 10

First, 0.195 g (0.525 mmol/g, 0.101 mmol, 1.0 eq) of a dry resin (Compound 4) was weighed into a 5 mL filter-equipped disposable syringe, 1.3 mL (10 v/w) of N,N-dimethylformamide was added, and the mixture was left to stand at room temperature for 30 minutes. After N,N-dimethylformamide was discharged, 1.1 mL (8 v/w) of an N,N-dimethylformamide solution containing 20% piperidine was added, and the mixture was shaken at room temperature for 15

minutes. After the reaction solution was discharged, 1.1 mL (8 v/w) of an N,N-dimethylformamide solution containing 20% piperidine was added again, and the mixture was shaken at room temperature for 15 minutes. After the reaction solution was discharged, the resin was washed with 1.3 mL (10 v/w) of N,N-dimethylformamide eight times. Next, 72 mg (0.21 mmol, 2.0 eq) of Fmoc-D-MeVal-OH and 15 mg (0.11 mmol, 1.0 eq) of Oxyma were weighed into a vial and dissolved by adding 0.54 mL (4 v/w) of N,N-dimethylformamide, and this solution was sucked into the disposable syringe. Subsequently, 66 μL (0.42 mmol, 4 eq) of DIC was added, and the syringe was sealed and then shaken at room temperature for 6 hours. After the reaction solution was discharged, the resin was washed with 1.3 mL (10 v/w) of N,N-dimethylformamide four times, with 1.3 mL (10 v/w) of 2-propanol twice and with 1.3 mL (10 v/w) of methanol four times, and then dried under reduced pressure at room temperature overnight to give 0.188 g of a dry resin (Compound 10). Then, 22.6 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance of the solution using a spectrophotometer, and the yield was calculated from the resulting Fmoc quantitative value, the mass of the dry resin, and LC A%.

[Table 13]

| (HPLC method B: Identification of target molecule and purity measurement) | | | | | |
|---|---|---|---|---|---|
| | Mass (g) | Fmoc quantitative value (mmol/g) | LC A% | Amount of substance (mmol) | Yield (%) |
| Starting material (Compound 4) | 0.195 | 0.525 | 99.0 | 0.101 | - |
| Product (Compound 10) | 0.188 | 0.517 | 96.8 | 0.0939 | 92.6 |

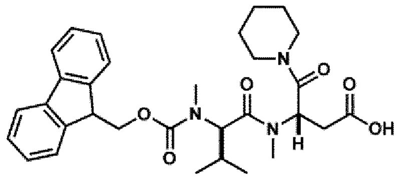
6

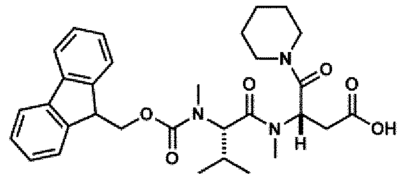
7

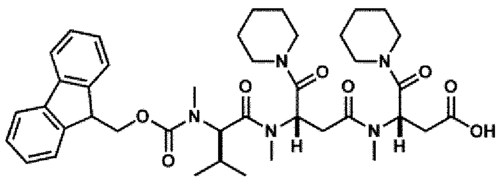
11

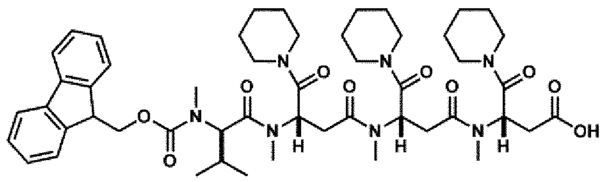
12

[Table 14]

| | Mw | m/z | Rt (min) | LC A% |
|---|---|---|---|---|
| Target molecule (Compound 6) | 549.7 | 550.5 ([M+H]$^+$) | 9.0 | 96.8 |
| Epimer (Compound 7) | 549.7 | 550.5 ([M+H]$^+$) | 9.3 | 0.1 |
| Excessively elongated product (Compound 11) | 745.9 | 746.6 ([M+H]$^+$) | 12.1 | 1.9 |
| Excessively elongated product (Compound 12) | 942.2 | Not detected | Not detected | Not detected |

The target molecule (Compound 10) could be obtained in a high yield. An excessively elongated product (Compound 11) and an epimer (Compound 7) were produced in 1.9 A% and 0.1 A%, respectively.

Peptide syntheses by solid phase reactions described in Example 2-2 to Example 2-11 were carried out by the Fmoc method using a peptide synthesizer (Multipep RS; manufactured by Intavis). For the detailed operational procedure, the manual appended to the synthesizer was followed.

Detailed synthesis conditions in Example 2-2 to Example 2-11 are presented in the following Synthesis Method 1.

Synthesis Method 1

An Fmoc amino acid (the second amino acid) that will constitute the intended peptide, HOAt or Oxyma as an additive, and NMP were dissolved such that the concentration of the Fmoc amino acid was 0.6 mol/L and that the concentration of the additive was 0.375 mol/L to prepare Solution 1. When an Fmoc amino acid was poorly soluble, DMSO was added so as to be 20 to 30% (v/v) to prepare Solution 1. Solution 2 was prepared by mixing with DMF such that DIC was 10% (v/v). A resin (100 mg) onto which an Fmoc amino acid or a peptide was loaded as prepared in Syntheses of Solid phase raw material 1 to 4 was placed in a filter (frit)-equipped solid phase reaction vessel, which was then placed in the peptide synthesizer. The resin was swollen by adding DCM (1.2 mL) and leaving the mixture to stand for 30 minutes, and then the solution was discharged from the frit. Solution 1 and Solution 2 were placed in the peptide synthesizer to initiate automatic synthesis by the peptide synthesizer.

A solution of DBU in DMF (2% v/v, 0.7 mL) was added to a solid phase reaction vessel containing the resin, and an Fmoc group removing reaction was carried out at room temperature. The reaction was carried out for 4.5 minutes for deprotection of the first residue, the reaction was carried out for 10 minutes for deprotection of the second and subsequent residues, and then the solution was discharged from the frit. DMF (0.7 mL) was added thereto, and the solution was left to stand for 5 minutes and then discharged from the frit. This resin washing step was repeated three more times. Subsequently, Solution 1 (0.3 mL) and Solution 2 (0.36 mL) were mixed in the mixing vial of the synthesizer (the mixing ratio of Fmoc amino acid : condensing agent: additive was about 1.6 : 2 : 1) and then added to the resin, the solid phase reaction vessel was heated to 40°C or 50°C to allow reaction for 2.5 hours or 10 hours, thus a condensation reaction between the amino group on the resin and the Fmoc amino acid was carried out, and then the solution was discharged from the frit. Then, the resin was washed three times with DMF (0.7 mL). This Fmoc group removing reaction and subsequent Fmoc amino acid condensation reaction were regarded as one cycle, and this cycle was repeated to allow elongation of the peptide.

After completing the peptide elongation, the resulting resin was washed with DMF (0.7 mL) four times and with DCM (0.7 mL) four times, and then air-dried at room temperature for 48 hours. A part (about 10 mg) of the resulting resin was placed in a reaction vessel, and the loading rate of the peptide on the resin was calculated according to the above Fmoc quantification method. Furthermore, a part (about 20 mg) of the resulting resin was placed in a reaction vessel, a TFE/DCM solution (1/1, 1 mL) with or without 0.75% (v/v) DIPEA was added, and the mixture was shaken at room temperature for 2 hours to carry out a peptide cleaving reaction. After the reaction, the cleaving solution was analyzed by LCMS.

For the tripeptides shown in [Example 2-2] to [Example 2-11], the recovery rate and the purity were calculated when synthesized under the following two conditions by a solid phase reaction using a peptide synthesizer.

[Table 15]

| | Elongation reaction condition of 2nd amino acid residue | Elongation reaction condition of 3rd amino acid residue |
|---|---|---|
| Condition 1 | HOAt 40°C, 2.5 hours | HOAt 40°C, 2.5 hours |
| Condition 2 | Oxyma, 50°C, 10 hours | HOAt 40°C, 2.5 hours |

The definition and the calculation method of the recovery rate in Examples 2-1 to 2-30 are shown in the following recovery rate calculation method.

Recovery rate calculation method

In Example 2, the recovery rate was defined as follows. It means that the higher the recovery rate is, the more suppressed the premature cleavage is.

Recovery rate = Actual Fmoc quantitative value (mmol/g) after elongation reaction / Theoretical Fmoc quantitative value (mmol/g) after elongation reaction (Formula 1)

The theoretical Fmoc quantitative value (mmol/g) after elongation reaction is calculated as follows.

Theoretical Fmoc quantitative value (mmol/g) after elongation reaction = Fmoc quantitative value (mmol/g) of raw material resin × Weight (g) of raw material resin / Weight (g) of resin when 100% of target molecule was produced (Formula 2)

The weight (g) of resin when 100% of the target molecule was produced is calculated as follows.

Weight (g) of resin when 100% of target molecule was produced = Weight (g) of raw material resin - Weight (g) of amino acid or peptide component on raw material resin + Weight (g) of peptide component on resin when 100% of target molecule was produced (Formula 3)

The weight (g) of the amino acid or peptide component on the raw material resin is calculated as follows.

Weight (g) of amino acid or peptide component on raw material resin = Fmoc quantitative value (mmol/g) of raw material resin × Weight (g) of raw material resin × Molecular weight (g/mol) of amino acid or peptide component on raw material resin × 0.001 (mol/mmol) (Formula 4)

The weight (g) of the peptide component on the resin when 100% of target molecule was produced is calculated as follows.

Weight (g) of peptide component on resin when 100% of target molecule was produced = Fmoc quantitative value (mmol/g) of raw material resin × Weight (g) of raw material resin × Molecular weight (g/mol) of peptide component of target molecule × 0.001 (mol/mmol) (Formula 5)

Substituting Formula 3, Formula 4, and Formula 5 for Formula 2 gives:

Theoretical Fmoc quantitative value (mmol/g) after elongation reaction = Fmoc quantitative value (mmol/g) of raw material resin / (1 - Fmoc quantitative value (mmol/g) of raw material resin) × Molecular weight (g/mol) of amino acid or peptide component on raw material resin × 0.001 (mol/mmol) + Fmoc quantitative value (mmol/g) of raw material resin × Molecular weight (g/mol) of peptide component of target molecule × 0.001 (mol/mmol)) =1 / (1 / Fmoc quantitative value (mmol/g) of raw material resin - Molecular weight (g/mol) of amino acid or peptide component on raw material resin × 0.001 (mol/mmol) + Molecular weight (g/mol) of peptide component of target molecule × 0.001 (mol/mmol)) (Formula 6)

When Formula 6 is substituted for Formula 1, the recovery rate is calculated by the following formula:

Recovery rate = Actual Fmoc quantitative value (mmol/g) after elongation reaction × (1 / Fmoc quantitative value (mmol/g) of raw material resin - Molecular weight (g/mol) of amino acid or peptide component on raw material resin × 0.001 (mol/mmol) + Molecular weight (g/mol) of peptide component of target molecule × 0.001 (mol/mmol))

[Example 2-2]

Synthesis of Fmoc-Ile-MeVal-MeAsp(O-Trt(2-Cl)resin)-pip (Compound 27)

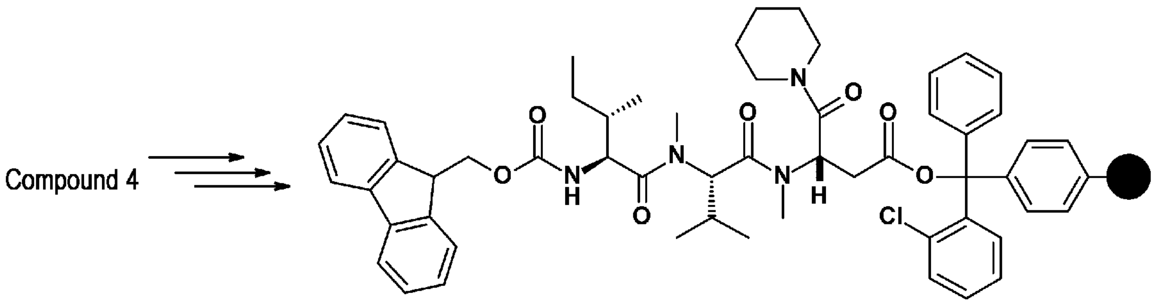


**27**

Synthesis of Compound 27 by Condition 1 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeVal-OH (HOAt, 40°C, 2.5 hours) and then elongation of Fmoc-Ile-OH (HOAt, 40°C, 2.5 hours) were carried out according to Synthesis Method 1 to synthesize Compound 27. Using a dry resin (10.45 mg), the loading rate was calculated by the Fmoc quantification method and was 0.292 mmol/g (UVarea value at 294 nm: 3046.82, UVarea value at 304 nm: 2746.87).

The recovery rate was calculated by the following formula according to the recovery rate calculation method.

Recovery rate = 0.292 × (1 / 0.455 - 436.51 × 0.001 + 662.83 × 0.001) = 70.8%

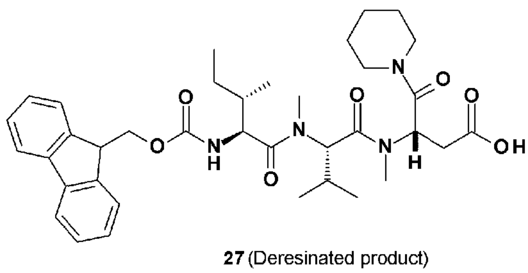

**27** (Deresinated product)

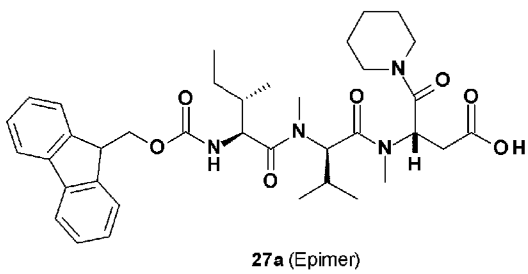

**27a** (Epimer)

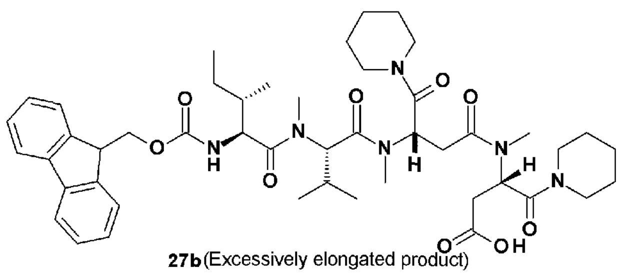

**27b**(Excessively elongated product)

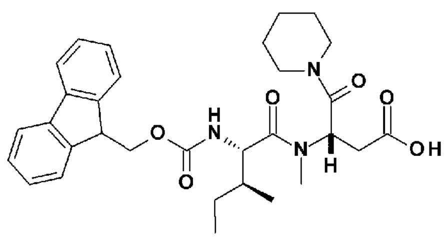

**27c** (Defective product)

When the cleaving reaction solution was analyzed by LCMS, it was observed that the purity of a deresinated product of Compound 27 was 61.6 area%, an epimer (Compound 27a) was 6.5 area%, an excessively elongated product (Compound 27b) was 2.4 area%, and a defective product (Compound 27c) was 29.5 area%.

Analysis Condition: HPLC method G

[Table 16]

| | Mw | m/z | Rt (min) | LC A% |
|---|---|---|---|---|
| Deresinated product (Compound 27) | 662.83 | 663.7 ([M+H]$^+$) | 1.78 | 61.6 |
| Epimer (Compound 27a) | 662.83 | 663.7 ([M+H]$^+$) | 2.00 | 6.5 |
| Excessively elongated product (Compound 27b) | 859.08 | 859.8 ([M+HD$^+$) | 2.18 | 2.4 |
| Defective product (Compound 27c) | 549.67 | 550.5 ([M+H]$^+$) | 1.63 | 29.5 |

Synthesis of Compound 27 by Condition 2 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeVal-OH (Oxyma, 50°C, 10 hours) and then elongation of Fmoc-Ile-OH (HOAt, 40°C, 2.5 hours) were carried out according to Synthesis Method 1 to synthesize Compound 27. Using a dry resin (10.89 mg), the loading rate was calculated by the Fmoc quantification method and was 0.332 mmol/g (UVarea value at 294 nm: 3612.81, UVarea value at 304 nm: 3267.35).

The recovery rate was calculated by the following formula according to the recovery rate calculation method.

$$\text{Recovery rate} = 0.332 \times (1 / 0.455 - 436.51 \times 0.001 + 662.83 \times 0.001) = 80.5\%$$

When the cleaving reaction solution was analyzed by LCMS, it was observed that the purity of a deresinated product of Compound 27 was 93.7 area%, an epimer (Compound 27a) was 2.6 area%, and an excessively elongated product (Compound 27b) was 3.7 area%.

Analysis condition: HPLC method G

[Table 17]

| | Mw | m/z | Rt (min) | LC A% |
|---|---|---|---|---|
| Deresinated product (Compound 27) | 662.83 | 663.6 ([M+HD$^+$) | 1.79 | 93.7 |
| Epimer (Compound 27a) | 662.83 | 663.7 ([M+HD$^+$) | 2.02 | 2.6 |
| Excessively elongated product (Compound 27b) | 859.08 | 859.8 ([M+HD$^+$) | 2.20 | 3.7 |

The above results are summarized in the table below.

[Table 18]

| | Recovery rate (%) | Deresinated product, Compo und 27 (LC A%) | Epimer, Compound 27a (LC A%) | Excessively elonga ted product, Comp ound 27b (LC A%) | Defective product, Compound 27c (LC A%) |
|---|---|---|---|---|---|
| Condition 1 | 70.8 | 61.6 | 6.5 | 2.4 | 29.5 |
| Condition 2 | 80.5 | 93.7 | 2.6 | 3.7 | - |

[Example 2-3]

Synthesis of Fmoc-Ile-MeIle-MeAsp(O-Trt(2-Cl)resin)-pip (Compound 28)

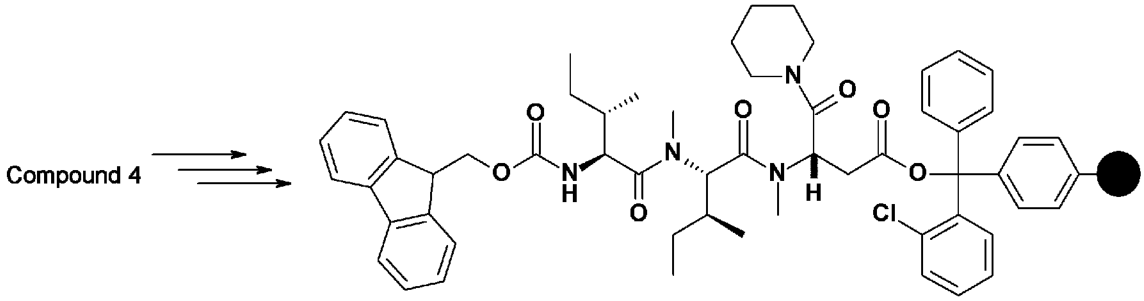


28

Synthesis of Compound 28 by Condition 1 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeIle-OH (HOAt, 40°C, 2.5 hours) and then elongation of Fmoc-Ile-OH (HOAt, 40°C, 2.5 hours) were carried out according to Synthesis Method 1 to synthesize Compound 28. Using a dry resin (10.81 mg), the loading rate was calculated by the Fmoc quantification method and was 0.326 mmol/g (UVarea value at 294 nm: 3524.40, UVarea value at 304 nm: 3171.55).

The recovery rate was calculated by the following formula according to the recovery rate calculation method.

$$\text{Recovery rate} = 0.326 \times (1 / 0.455 - 436.51 \times 0.001 + 676.86 \times 0.001) = 79.5\%$$

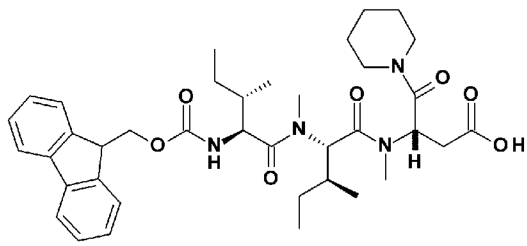

**28** (Deresinated product)

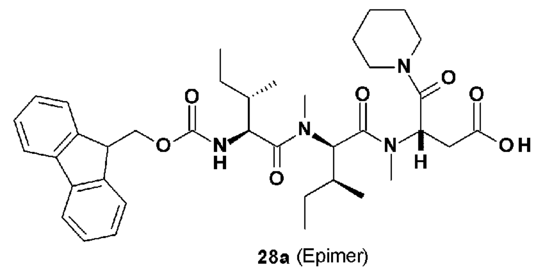

**28a** (Epimer)

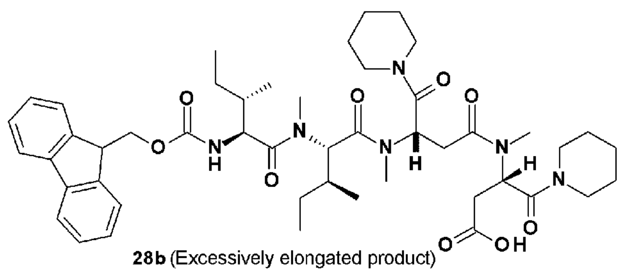

**28b** (Excessively elongated product)

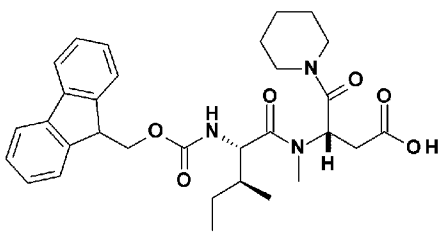

**27c** (Defective product)

When the cleaving reaction solution was analyzed by LCMS, it was observed that the purity of a deresinated product of Compound 28 was 58.0 area%, an epimer (Compound 28a) was 1.4 area%, an excessively elongated product (Compound 28b) was 1.8 area%, and a defective product (Compound 27c) was 38.8 area%.

Analysis Condition: HPLC method G

[Table 19]

| | Mw | m/z | Rt (min) | LC A% |
|---|---|---|---|---|
| Deresinated product (Compound 28) | 676.86 | 677.6 ([M+H]$^+$) | 2.00 | 58.0 |
| Epimer (Compound 28a) | 676.86 | 677.7 ([M+H]$^+$) | 2.20 | 1.4 |
| Excessively elongated product (Compound 28b) | 873.11 | 873.8 ([M+H]$^+$) | 2.37 | 1.8 |
| Defective product (Compound 27c) | 549.67 | 550.5 ([M+H]$^+$) | 1.62 | 38.8 |

Synthesis of Compound 28 by Condition 2 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeIle-OH (Oxyma, 50°C, 10 hours) and then elongation of Fmoc-Ile-OH (HOAt, 40°C, 2.5 hours) were carried out according to Synthesis Method 1 to synthesize Compound 28. Using a dry resin (10.41 mg), the loading rate was calculated by the Fmoc quantification method and was 0.330 mmol/g (UVarea value at 294 nm: 3437.60, UVarea value at 304 nm: 3100.91).

The recovery rate was calculated by the following formula according to the recovery rate calculation method.

$$\text{Recovery rate} = 0.330 \times (1 / 0.455 - 436.51 \times 0.001 + 676.86 \times 0.001) = 80.5\%$$

When the cleaving reaction solution was analyzed by LCMS, it was observed that the purity of a deresinated product of Compound 28 was 95.2 area%, an epimer (Compound 28a) was 1.4 area%, and an excessively elongated product (Compound 28b) was 3.4 area%.

Analysis Condition: HPLC method G

[Table 20]

| | Mw | m/z | Rt (min) | LC A% |
|---|---|---|---|---|
| Deresinated product (Compound 28) | 676.86 | 677.7 ([M+H]$^+$) | 2.01 | 95.2 |
| Epimer (Compound 28a) | 676.86 | 677.6 ([M+H]$^+$) | 2.22 | 1.4 |
| Excessively elongated product (Compound 28b) | 873.11 | 873.9 ([M+H]$^+$) | 2.39 | 3.4 |

The above results are summarized in the table below.

[Table 21]

| | Recovery rate (%) | Deresinated product, Compo und 28 (LC A%) | Epimer, Compound 28a (LC A%) | Excessively elonga ted product, Comp ound 28b (LC A%) | Defective product, Compound 27c (LC A%) |
|---|---|---|---|---|---|
| Condition 1 | 79.5 | 58.0 | 1.4 | 1.8 | 38.8 |
| Condition 2 | 80.5 | 95.2 | 1.4 | 3.4 | - |

[Example 2-4]

Synthesis of Fmoc-Ile-MeGly(cPent)-MeAsp(O-Trt(2-Cl)resin)-pip (Compound 29)

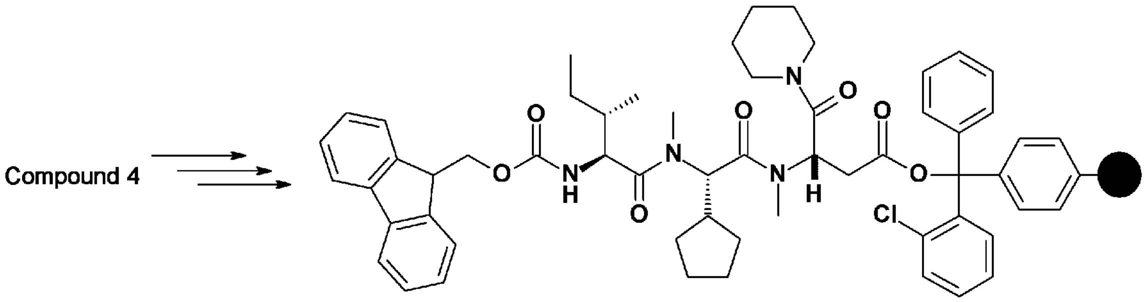


29

Synthesis of Compound 29 by Condition 1 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeGly(cPent)-OH (HOAt, 40°C, 2.5 hours) and then elongation of Fmoc-Ile-OH (HOAt, 40°C, 2.5 hours) were carried out according to Synthesis Method 1 to synthesize Compound 29. Using a dry resin (10.05 mg), the loading rate was calculated by the Fmoc quantification method and was 0.320 mmol/g (UVarea value at 294 nm: 3216.77, UVarea value at 304 nm: 2905.18).

The recovery rate was calculated by the following formula according to the recovery rate calculation method.

$$\text{Recovery rate} = 0.320 \times (1 / 0.455 - 436.51 \times 0.001 + 688.87 \times 0.001) = 78.4\%$$

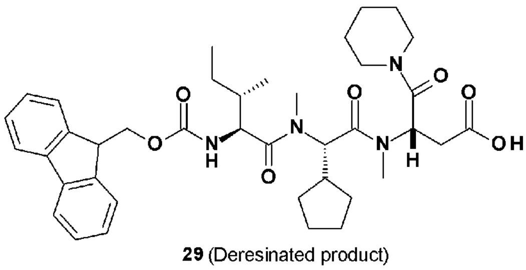

**29** (Deresinated product)

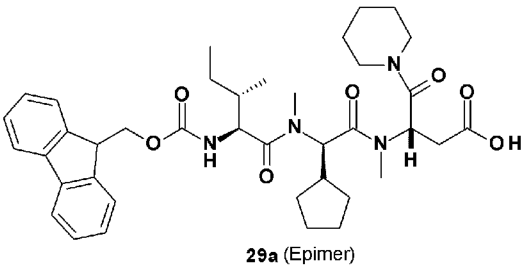

**29a** (Epimer)

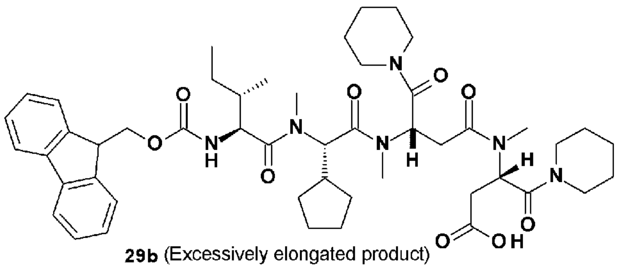

**29b** (Excessively elongated product)

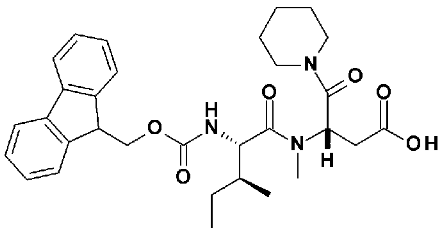

**27c** (Defective product)

When the cleaving reaction solution was analyzed by LCMS, it was observed that the purity of a deresinated product of Compound 29 was 83.9 area%, an epimer (Compound 29a) was 1.2 area%, an excessively elongated product (Compound 29b) was 3.9 area%, and a defective product (Compound 27c) was 11.0 area%.

Analysis Condition: HPLC method G

[Table 22]

| | Mw | m/z | Rt (min) | LC A% |
|---|---|---|---|---|
| Deresinated product (Compound 29) | 688.87 | 689.6 ([M+HD$^+$]) | 2.06 | 83.9 |

(continued)

| | Mw | m/z | Rt (min) | LC A% |
|---|---|---|---|---|
| Epimer (Compound 29a) | 688.87 | 689.7 ([M+HD$^+$) | 2.27 | 1.2 |
| Excessively elongated product (Compound 29b) | 885.12 | 885.8 ([M+H]$^+$) | 2.43 | 3.9 |
| Defective product (Compound 27c) | 549.67 | 550.5 ([M+H]$^+$) | 1.63 | 11.0 |

Synthesis of Compound 29 by Condition 2 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeGly(cPent)-OH (Oxyma, 50°C, 10 hours) and then elongation of Fmoc-Ile-OH (HOAt, 40°C, 2.5 hours) were carried out according to Synthesis Method 1 to synthesize Compound 29. Using a dry resin (9.45 mg), the loading rate was calculated by the Fmoc quantification method and was 0.232 mmol/g (UVarea value at 294 nm: 2194.45, UVarea value at 304 nm: 1975.12).

The recovery rate was calculated by the following formula according to the recovery rate calculation method.

$$\text{Recovery rate} = 0.232 \times (1 / 0.455 - 436.51 \times 0.001 + 688.87 \times 0.001) = 56.8\%$$

When the cleaving reaction solution was analyzed by LCMS, it was observed that the purity of a deresinated product of Compound 29 was 91.4 area%, an epimer (Compound 29a) was 0.1 area%, and an excessively elongated product (Compound 29b) was 8.5 area%.

Analysis Condition: HPLC method G

[Table 23]

| | Mw | m/z | Rt (min) | LC A% |
|---|---|---|---|---|
| Deresinated product (Compound 29) | 688.87 | 689.7 ([M+HD$^+$) | 2.09 | 91.4 |
| Epimer (Compound 29a) | 688.87 | 689.5 ([M+H]$^+$) | 2.29 | 0.1 |
| Excessively elongated product (Compound 29b) | 885.12 | 885.8 ([M+H]‘) | 2.45 | 8.5 |

The above results are summarized in the table below.

[Table 24]

| | Recovery rate (%) | Deresinated product, Compo und 29 (LC A%) | Epimer, Compound 29a (LC A%) | Excessively elonga ted product, Comp ound 29b (LC A%) | Defective product, Compound 27c (LC A%) |
|---|---|---|---|---|---|
| Condition 1 | 78.4 | 83.9 | 1.2 | 3.9 | 11.0 |
| Condition 2 | 56.8 | 91.4 | 0.1 | 8.5 | - |

[Example 2-5]

Synthesis of Fmoc-Ile-MeChg-MeAsp(O-Trt(2-Cl)resin)-pip (Compound 30)

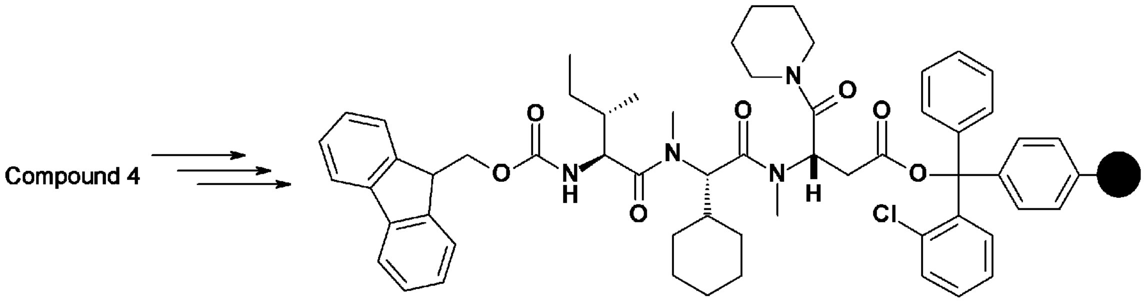


30

Synthesis of Compound 30 by Condition 1 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeChg-OH (HOAt, 40°C, 2.5 hours) and then elongation of Fmoc-Ile-OH (HOAt, 40°C, 2.5 hours) were carried out according to Synthesis Method 1 to synthesize Compound 30. Using a dry resin (9.68 mg), the loading rate was calculated by the Fmoc quantification method and was 0.314 mmol/g (UVarea value at 294 nm: 3037.14, UVarea value at 304 nm: 2743.09).

The recovery rate was calculated by the following formula according to the recovery rate calculation method.

$$\text{Recovery rate} = 0.314 \times (1 / 0.455 - 436.51 \times 0.001 + 702.89 \times 0.001) = 77.4\%$$

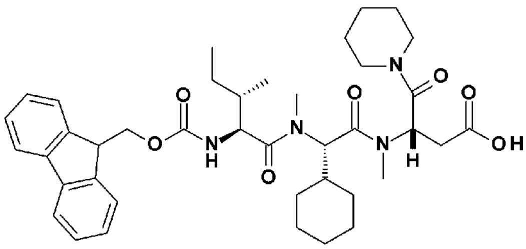

**30** (Deresinated product)

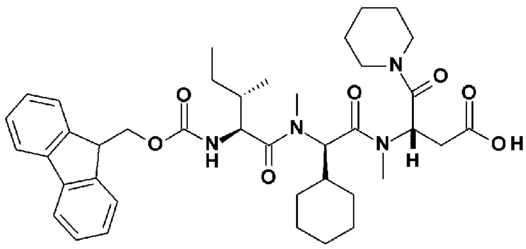

**30a** (Epimer)

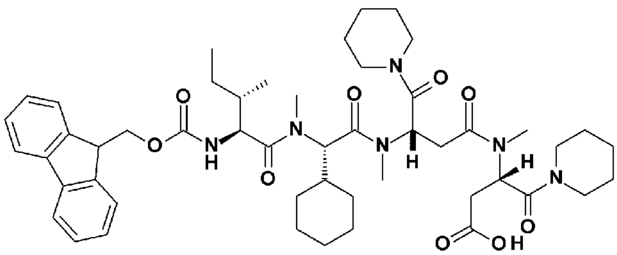

**30b** (Excessively elongated product)

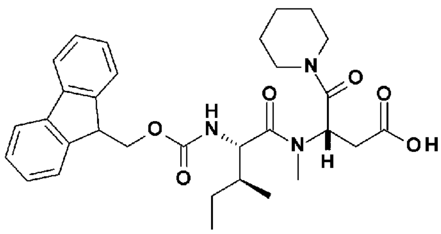

**27c** (Defective product)

When the cleaving reaction solution was analyzed by LCMS, it was observed that the purity of a deresinated product of Compound 30 was 74.1 area%, an epimer (Compound 30a) was 2.3 area%, an excessively elongated product (Compound 30b) was 4.0 area%, and a defective product (Compound 27c) was 19.5 area%.

Analysis Condition: HPLC method G

[Table 25]

| | Mw | m/z | Rt (min) | LC A% |
|---|---|---|---|---|
| Deresinated product (Compound 30) | 702.89 | 703.6 ([M+H]$^+$) | 2.24 | 74.1 |
| Epimer (Compound 30a) | 702.89 | 703.6 ([M+H]$^+$) | 2.43 | 2.3 |

(continued)

| | Mw | m/z | Rt (min) | LC A% |
|---|---|---|---|---|
| Excessively elongated product (Compound 30b) | 899.14 | 899.8 ([M+HD$^+$) | 2.58 | 4.0 |
| Defective product (Compound 27c) | 549.67 | 550.5 ([M+H]$^+$) | 1.63 | 19.5 |

Synthesis of Compound 30 by Condition 2 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeChg-OH (Oxyma, 50°C, 10 hours) and then elongation of Fmoc-Ile-OH (HOAt, 40°C, 2.5 hours) were carried out according to Synthesis Method 1 to synthesize Compound 30. Using a dry resin (9.91 mg), the loading rate was calculated by the Fmoc quantification method and was 0.226 mmol/g (UVarea value at 294 nm: 2235.50, UVarea value at 304 nm: 2016.81).

The recovery rate was calculated by the following formula according to the recovery rate calculation method.

$$\text{Recovery rate} = 0.226 \times (1 / 0.455 - 436.51 \times 0.001 + 702.89 \times 0.001) = 55.7\%$$

When the cleaving reaction solution was analyzed by LCMS, it was observed that the purity of a deresinated product of Compound 30 was 91.7 area%, an epimer (Compound 30a) was 0.6 area%, and an excessively elongated product (Compound 30b) was 7.7 area%.

Analysis Condition: HPLC method G

[Table 26]

| | Mw | m/z | Rt (min) | LC A% |
|---|---|---|---|---|
| Deresinated product (Compound 30) | 702.89 | 703.6 ([M+H]$^+$) | 2.26 | 91.7 |
| Epimer (Compound 30a) | 702.89 | 703.7 ([M+H]$^+$) | 2.44 | 0.6 |
| Excessively elongated product (Compound 30b) | 899.14 | 899.8 ([M+H]$^+$) | 2.60 | 7.7 |

The above results are summarized in the table below.

[Table 27]

| | Recovery rate (%) | Deresinated product, Compo und 30 (LC A%) | Epimer, Compound 30a (LC A%) | Excessively elonga ted product, Comp ound 30b (LC A%) | Defective product, Compound 27c (LC A%) |
|---|---|---|---|---|---|
| Condition 1 | 77.4 | 74.1 | 2.3 | 4.0 | 19.5 |
| Condition 2 | 55.7 | 91.7 | 0.6 | 7.7 | - |

[Example 2-6]

Synthesis of Fmoc-Ile-MeLeu-MeAsp(O-Trt(2-Cl)resin)-pip (Compound 31)

Compound 4

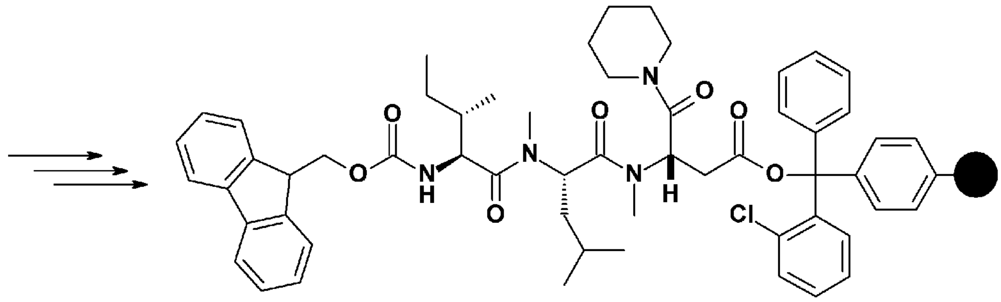

31

Synthesis of Compound 31 by Condition 1 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeLeu-OH (HOAt, 40°C, 2.5 hours) and then elongation of Fmoc-Ile-OH (HOAt, 40°C, 2.5 hours) were carried out according to Synthesis Method 1 to synthesize Compound 31. Using a dry resin (10.74 mg), the loading rate was calculated by the Fmoc quantification method and was 0.369 mmol/g (UVarea value at 294 nm: 3953.19, UVarea value at 304 nm: 3570.96).

The recovery rate was calculated by the following formula according to the recovery rate calculation method.

$$\text{Recovery rate} = 0.369 \times (1 / 0.455 - 436.51 \times 0.001 + 676.86 \times 0.001) = 90.0\%$$

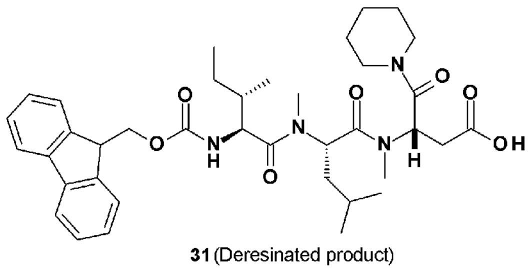

**31** (Deresinated product)

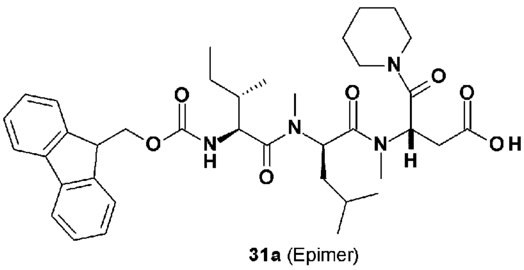

**31a** (Epimer)

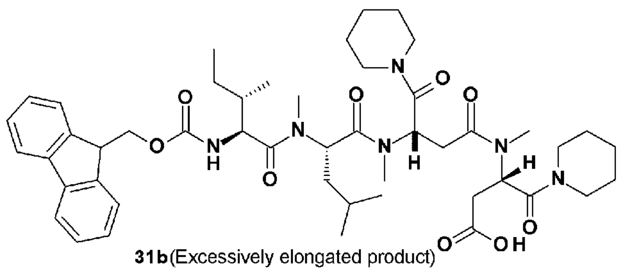

**31b**(Excessively elongated product)

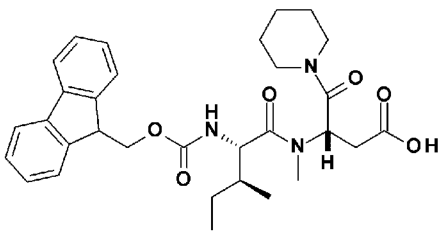

**27c** (Defective product)

When the cleaving reaction solution was analyzed by LCMS, it was observed that the purity of a deresinated product of Compound 31 was 98.3 area%, an epimer (Compound 31a) was 0.1 area%, and an excessively elongated product (Compound 31b) was 1.6 area%. With this substrate, a defective product (Compound 27c) was not observed even under Condition 1.

Analysis Condition: HPLC method G

[Table 28]

| | Mw | m/z | Rt (min) | LC A% |
|---|---|---|---|---|
| Deresinated product (Compound 31) | 676.86 | 677.6 ([M+H]$^+$) | 2.03 | 98.3 |

(continued)

| | Mw | m/z | Rt (min) | LC A% |
|---|---|---|---|---|
| Epimer (Compound 31a) | 676.86 | 677.6 ($[M+H]^+$) | 2.23 | 0.1 |
| Excessively elongated product (Compound 31b) | 873.11 | 873.8 ($[M+H]^+$) | 2.41 | 1.6 |
| Defective product (Compound 27c) | 549.67 | Not detected | | |

Synthesis of Compound 31 by Condition 2 (Synthesis Method 1)

Using the amino acid-loaded resin (Compound 4) (Fmoc-MeAsp(O-Trt(2-Cl)resin)-pip) (100 mg, 0.455 mmol/g) prepared in Synthesis of Solid phase raw material 1 as a starting material, elongation of Fmoc-MeLeu-OH (Oxyma, 50°C, 10 hours) and then elongation of Fmoc-Ile-OH (HOAt, 40°C, 2.5 hours) were carried out according to Synthesis Method 1 to synthesize Compound 31. Using a dry resin (10.73 mg), the loading rate was calculated by the Fmoc quantification method and was 0.337 mmol/g (UVarea value at 294 nm: 3610.72, UVarea value at 304 nm: 3254.78).

The recovery rate was calculated by the following formula according to the recovery rate calculation method.

$$\text{Recovery rate} = 0.337 \times (1 / 0.455 - 436.51 \times 0.001 + 676.86 \times 0.001) = 82.2\%$$

When the cleaving reaction solution was analyzed by LCMS, it was observed that the purity of a deresinated product of Compound 31 was 97.7 area%, an epimer (Compound 31a) was 0.1 area%, and an excessively elongated product (Compound 31b) was 2.2 area%.

Analysis Condition: HPLC method G

[Table 29]

| | Mw | m/z | Rt (min) | LC A% |
|---|---|---|---|---|
| Deresinated product (Compound 31) | 676.86 | 677.6 ($[M+H]^+$) | 2.05 | 97.7 |
| Epimer (Compound 31a) | 676.86 | 677.6 ($[M+H]^+$) | 2.25 | 0.1 |
| Excessively elongated product (Compound 31b) | 873.11 | 873.9 ($[M+H]^+$) | 2.42 | 2.2 |

The above results are summarized in the table below.

[Table 30]

| | Recovery rate (%) | Deresinated product, Compo und 31 (LC A%) | Epimer, Compound 31a (LC A%) | Excessively elonga ted product, Comp ound 31b (LC A%) | Defective product, Compound 27c (LC A%) |
|---|---|---|---|---|---|
| Condition 1 | 90.0 | 98.3 | 0.1 | 1.6 | - |
| Condition 2 | 82.2 | 97.7 | 0.1 | 2.2 | - |

[Example 2-7]

Synthesis of Fmoc-Ile-MeGly(cPent)-MeAsp(O-Trt(2-Cl)resin)-NMe2 (Compound 32)

Compound 22

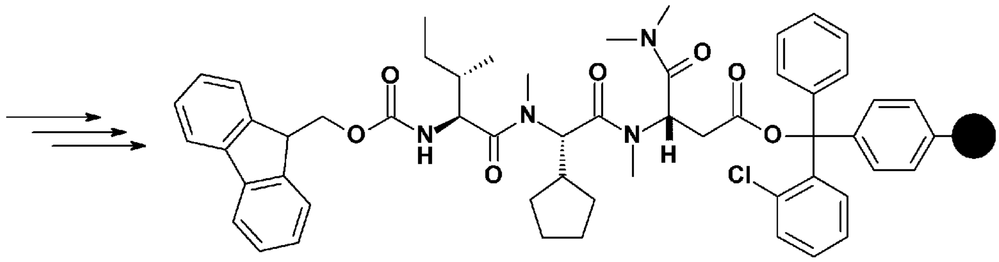

32

Synthesis of Compound 32 by Condition 1 (Synthesis Method 1)

Using the amino acid-loaded resin (Compound 22) (Fmoc-MeAsp(O-Trt(2-Cl)resin)-NMe2) (100 mg, 0.442 mmol/g) prepared in Synthesis of Solid phase raw material 2 as a starting material, elongation of Fmoc-MeGly(cPent)-OH (HOAt, 40°C, 2.5 hours) and then elongation of Fmoc-Ile-OH (HOAt, 40°C, 2.5 hours) were carried out according to Synthesis Method 1 to synthesize Compound 32. Using a dry resin (10.73 mg), the loading rate was calculated by the Fmoc quantification method and was 0.304 mmol/g (UVarea value at 294 nm: 3263.10, UVarea value at 304 nm: 2945.28).

The recovery rate was calculated by the following formula according to the recovery rate calculation method.

$$\text{Recovery rate} = 0.304 \times (1 / 0.442 - 396.44 \times 0.001 + 648.80 \times 0.001) = 76.5\%$$

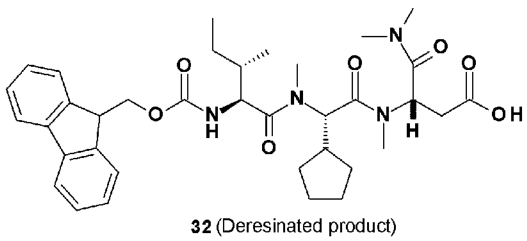

**32** (Deresinated product)

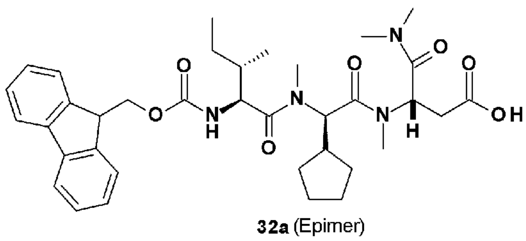

**32a** (Epimer)

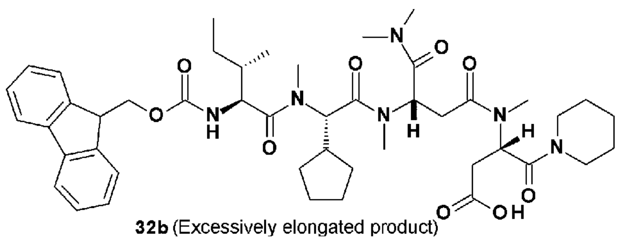

**32b** (Excessively elongated product)

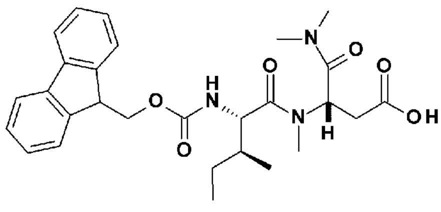

**32c** (Defective product)

When the cleaving reaction solution was analyzed by LCMS, it was observed that the purity of a deresinated product of Compound 32 was 76.3 area%, an epimer (Compound 32a) was 1.2 area%, an excessively elongated product (Compound 32b) was 5.6 area%, and a defective product (Compound 32c) was 16.9 area%.

Analysis Condition: HPLC METHOD E

[Table 31]

| | Mw | m/z | Rt (min) | LC A% |
|---|---|---|---|---|
| Deresinated product (Compound 32) | 648.80 | 649.7 ([M+HD$^+$) | 0.97 | 76.3 |
| Epimer (Compound 32a) | 648.80 | 649.6 ([M+H]$^+$) | 1.03 | 1.2 |
| Excessively elongated product (Compound 32b) | 804.98 | 805.7 ([M+H]$^+$) | 0.93 | 5.6 |
| Defective product (Compound 32c) | 509.60 | 510.5 ([M+H]$^+$) | 0.88 | 16.9 |

Synthesis of Compound 32 by Condition 2 (Synthesis Method 1)

Using the amino acid-loaded resin (Compound 22) (Fmoc-MeAsp(O-Trt(2-Cl)resin)-NMe2) (100 mg, 0.442 mmol/g) prepared in Synthesis of Solid phase raw material 2 as a starting material, elongation of Fmoc-MeGly(cPent)-OH (Oxyma, 50°C, 10 hours) and then elongation of Fmoc-Ile-OH (HOAt, 40°C, 2.5 hours) were carried out according to Synthesis Method 1 to synthesize Compound 32. Using a dry resin (10.60 mg), the loading rate was calculated by the Fmoc quantification method and was 0.211 mmol/g (UVarea value at 294 nm: 2239.56, UVarea value at 304 nm: 2018.13).

The recovery rate was calculated by the following formula according to the recovery rate calculation method.

$$\text{Recovery rate} = 0.211 \times (1 / 0.442 - 396.44 \times 0.001 + 648.80 \times 0.001) = 53.1\%$$

When the cleaving reaction solution was analyzed by LCMS, it was observed that the purity of a deresinated product of Compound 32 was 85.9 area%, and an excessively elongated product (Compound 32a) was 14.1 area%.

Analysis Condition: HPLC METHOD E

[Table 32]

| | Mw | m/z | Rt (min) | LC A% |
|---|---|---|---|---|
| Deresinated product (Compound 32) | 648. 80 | 649. 6 ([M+H]$^+$) | 0. 96 | 85. 9 |
| Epimer (Compound 32a) | 648. 80 | Not detected | | |
| Excessively elongated product (Compound 32b) | 804. 98 | 805. 7 ([M+H]$^+$) | 0. 92 | 14. 1 |

The above results are summarized in the table below.

[Table 33]

| | Recovery rate (%) | Deresinated product, Compo und 32 (LC A%) | Epimer, Compound 32a (LC A%) | Excessively elonga ted product, Comp ound 32b (LC A%) | Defective product, Compound 32c (LC A%) |
|---|---|---|---|---|---|
| Condition 1 | 76. 5 | 76. 3 | 1.2 | 5.6 | 16. 9 |
| Condition 2 | 53. 1 | 85. 9 | - | 14.1 | - |

[Example 2-8]

Synthesis of Fmoc-Ile-MeLeu-MeAsp(O-Trt(2-Cl)resin)-NMe2 (Compound 33)

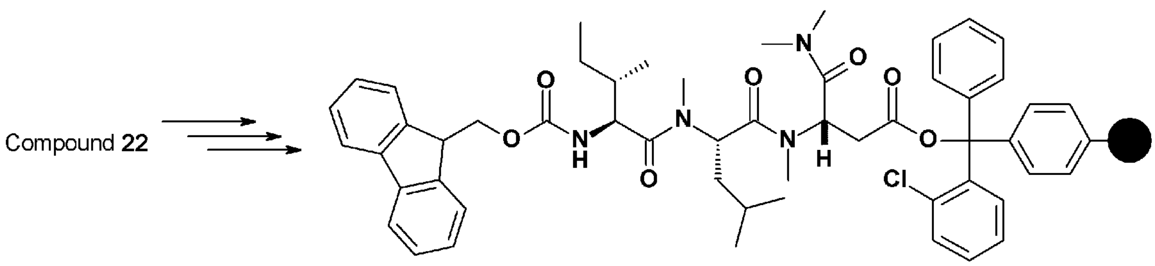

33

Synthesis of Compound 33 by Condition 1 (Synthesis Method 1)

Using the amino acid-loaded resin (Compound 22) (Fmoc-MeAsp(O-Trt(2-Cl)resin)-NMe2) (100 mg, 0.442 mmol/g) prepared in Synthesis of Solid phase raw material 2 as a starting material, elongation of Fmoc-MeLeu-OH (HOAt, 40°C, 2.5 hours) and then elongation of Fmoc-Ile-OH (HOAt, 40°C, 2.5 hours) were carried out according to Synthesis Method 1 to synthesize Compound 33. Using a dry resin (10.15 mg), the loading rate was calculated by the Fmoc quantification method and was 0.371 mmol/g (UVarea value at 294 nm: 3770.57, UVarea value at 304 nm: 3391.62).
The recovery rate was calculated by the following formula according to the recovery rate calculation method.

$$\text{Recovery rate} = 0.371 \times (1 / 0.442 - 396.44 \times 0.001 + 636.79 \times 0.001) = 92.9\%$$

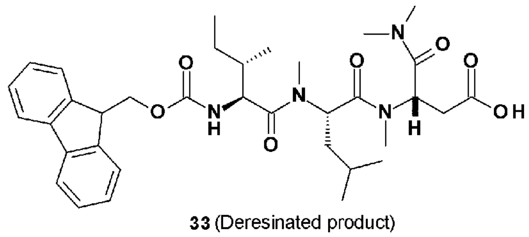

**33** (Deresinated product)

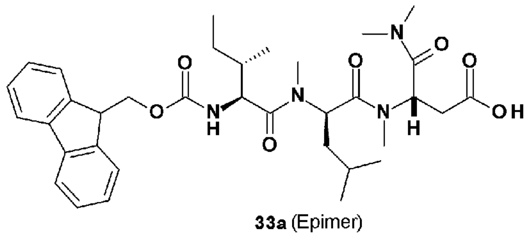

**33a** (Epimer)

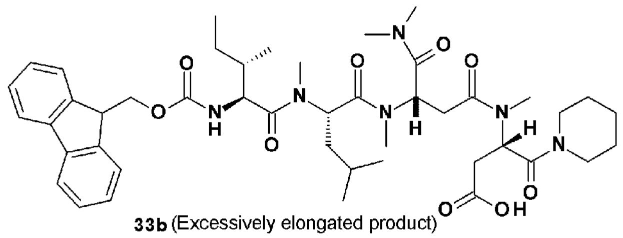

**33b** (Excessively elongated product)

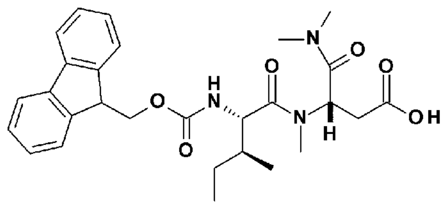

**32c** (Defective product)

When the cleaving reaction solution was analyzed by LCMS, it was observed that the purity of a deresinated product of Compound 33 was 97.9 area%, and an excessively elongated product (Compound 33b) was 2.1 area%. With this substrate, a defective product (Compound 32c) was not observed even under Condition 1.

Analysis Condition: HPLC METHOD E

[Table 34]

| | Mw | m/z | Rt (min) | LC A% |
|---|---|---|---|---|
| Deresinated product (Compound 33) | 636.79 | 637.7 ([M+H] $^+$) | 0. 97 | 97.9 |
| Epimer (Compound 33a) | 636.79 | Not detected | | |
| Excessively elongated product (Compound 33b) | 792.97 | 793.7 ([M+H]$^+$) | 0. 92 | 2.1 |
| Defective product (Compound 32c) | 509.60 | Not detected | | |

Synthesis of Compound 33 by Condition 2 (Synthesis Method 1)

Using the amino acid-loaded resin (Compound 22) (Fmoc-MeAsp(O-Trt(2-Cl)resin)-NMe2) (100 mg, 0.442 mmol/g) prepared in Synthesis of Solid phase raw material 2 as a starting material, elongation of Fmoc-MeLeu-OH (Oxyma, 50°C, 10 hours) and then elongation of Fmoc-Ile-OH (HOAt, 40°C, 2.5 hours) were carried out according to Synthesis Method 1 to synthesize Compound 33. Using a dry resin (10.73 mg), the loading rate was calculated by the Fmoc quantification method and was 0.334 mmol/g (UVarea value at 294 nm: 3587.19, UVarea value at 304 nm: 3234.07).
The recovery rate was calculated by the following formula according to the recovery rate calculation method.

$$\text{Recovery rate} = 0.334 \times (1 / 0.442 - 396.44 \times 0.001 + 636.79 \times 0.001) = 83.6\%$$

When the cleaving reaction solution was analyzed by LCMS, it was observed that the purity of a deresinated product of Compound 33 was 96.6 area%, and an excessively elongated product (Compound 33b) was 3.4 area%.

Analysis Condition: HPLC METHOD E

[Table 35]

| | Mw | m/z | Rt (min) | LC A% |
|---|---|---|---|---|
| Deresinated product (Compound 33) | 636.79 | 637.6 ([M+H] +) | 0.96 | 96.6 |
| Epimer (Compound 33a) | 636.79 | Not detected | | |
| Excessively elongated product (Compound 33b) | 792.97 | 793.7 ([M+H] +) | 0.92 | 3.4 |

The above results are summarized in the table below.

[Table 36]

| | Recovery rate (%) | Deresinated product, Compo und 33 (LC A%) | Epimer, Compound 33a (LC A%) | Excessively elonga ted product, Comp ound 33b (LC A%) | Defective product, Compound 32c (LC A%) |
|---|---|---|---|---|---|
| Condition 1 | 92.9 | 97.9 | - | 2.1 | - |
| Condition 2 | 83.6 | 96.6 | - | 3.4 | - |

[Example 2-9]

Synthesis of Fmoc-Ile-MeVal-D-3-MeAbu-O-Trt(2-Cl)resin (Compound 14)

Compound **24**

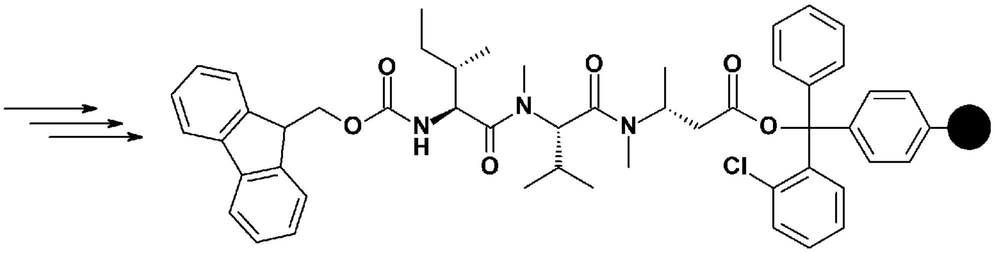

**34**

Synthesis of Compound 34 by Condition 1 (Synthesis Method 1)

Using the amino acid-loaded resin (Compound 24) (Fmoc-D-3-MeAbu-O-Trt(2-Cl)resin) (100 mg, 0.369 mmol/g) prepared in Synthesis of Solid phase raw material 3 as a starting material, elongation of Fmoc-MeVal-OH (HOAt, 40°C, 2.5 hours) and then elongation of Fmoc-Ile-OH (HOAt, 40°C, 2.5 hours) were carried out according to Synthesis Method 1 to synthesize Compound 34. Using a dry resin (10.62 mg), the loading rate was calculated by the Fmoc quantification method and was 0.295 mmol/g (UVarea value at 294 nm: 3138.26, UVarea value at 304 nm: 2821.05).
The recovery rate was calculated by the following formula according to the recovery rate calculation method.

$$\text{Recovery rate} = 0.295 \times (1 / 0.369 - 339.39 \times 0.001 + 565.71 \times 0.001) = 86.6\%$$

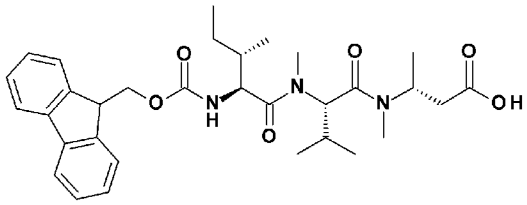

**34** (Deresinated product)

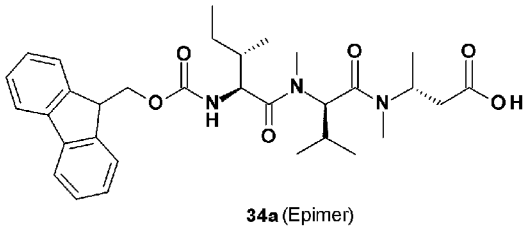

**34a** (Epimer)

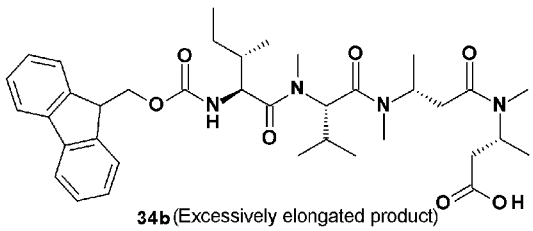

**34b** (Excessively elongated product)

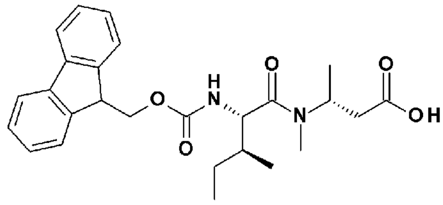

**34c** (Defective product)

When the cleaving reaction solution was analyzed by LCMS, it was observed that the purity of a deresinated product of Compound 34 was 99.1 area%, and an excessively elongated product (Compound 34b) was 1.0 area%. With this substrate, a defective product (Compound 34c) was not observed even under Condition 1.

Analysis Condition: HPLC method G

[Table 37]

| | Mw | m/z | Rt (min) | LC A% |
|---|---|---|---|---|
| Deresinated product (Compound 34) | 565.71 | 566.5 ([M+H]$^+$) | 1.50 | 99.1 |
| Epimer (Compound 34a) | 565.71 | Not detected | | |
| Excessively elongated product (Compound 34b) | 664.84 | 665.6 ([M+H]$^+$) | 1.58 | 1.0 |
| Defective product (Compound 34c) | 452.55 | Not detected | | |

Synthesis of Compound 34 by Condition 2 (Synthesis Method 1)

Using the amino acid-loaded resin (Compound 24) (Fmoc-D-3-MeAbu-O-Trt(2-Cl)resin) (100 mg, 0.369 mmol/g) prepared in Synthesis of Solid phase raw material 3 as a starting material, elongation of Fmoc-MeVal-OH (Oxyma, 50°C, 10 hours) and then elongation of Fmoc-Ile-OH (HOAt, 40°C, 2.5 hours) were carried out according to Synthesis Method 1 to synthesize Compound 34. Using a dry resin (10.44 mg), the loading rate was calculated by the Fmoc quantification method and was 0.284 mmol/g (UVarea value at 294 nm: 2964.86, UVarea value at 304 nm: 2667.78).

The recovery rate was calculated by the following formula according to the recovery rate calculation method.

$$\text{Recovery rate} = 0.284 \times (1 / 0.369 - 339.39 \times 0.001 + 565.71 \times 0.001) = 83.4\%$$

When the cleaving reaction solution was analyzed by LCMS, it was observed that the purity of a deresinated product of Compound 34 was 98.1 area%, and an excessively elongated product (Compound 34b) was 1.9 area%.

Analysis Condition: HPLC method G

[Table 38]

| | Mw | m/z | Rt (min) | LC A% |
|---|---|---|---|---|
| Deresinated product (Compound 34) | 565.71 | 566.6 ($[M+H]^+$) | 1.51 | 98.1 |
| Epimer (Compound 34a) | 565.71 | Not detected | | |
| Excessively elongated product (Compound 34b) | 664.84 | 665.6 ($[M+H]^+$) | 1.59 | 1.9 |

The above results are summarized in the table below.

[Table 39]

| | Recovery rate (%) | Deresinated product, Compo und 34 (LC A%) | Epimer, Compound 34a (LC A%) | Excessively elonga ted product, Comp ound 34b (LC A%) | Defective product, Compound 34c (LC A%) |
|---|---|---|---|---|---|
| Condition 1 | 86.6 | 99.1 | - | 1.0 | - |
| Condition 2 | 83.4 | 98.1 | - | 1.9 | - |

[Example 2-10]

Synthesis of Fmoc-Ile-MeChg-D-3-MeAbu-O-Trt(2-Cl)resin (Compound 35)

Compound **24**

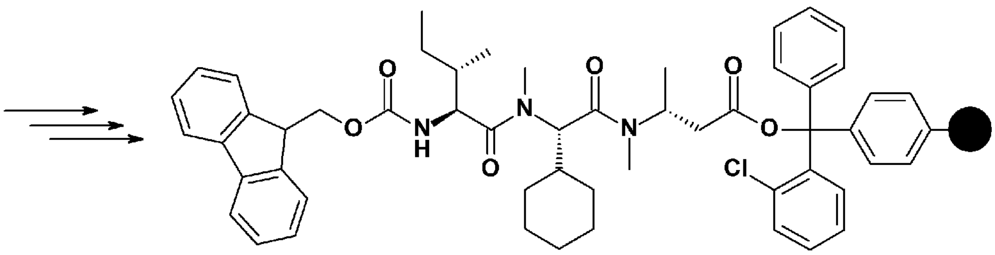

**35**

Synthesis of Compound 35 by Condition 1 (Synthesis Method 1)

Using the amino acid-loaded resin (Compound 24) (Fmoc-D-3-MeAbu-O-Trt(2-Cl)resin) (100 mg, 0.369 mmol/g) prepared in Synthesis of Solid phase raw material 3 as a starting material, elongation of Fmoc-MeChg-OH (HOAt, 40°C, 2.5 hours) and then elongation of Fmoc-Ile-OH (HOAt, 40°C, 2.5 hours) were carried out according to Synthesis Method 1 to synthesize Compound 35. Using a dry resin (10.20 mg), the loading rate was calculated by the Fmoc quantification method and was 0.293 mmol/g (UVarea value at 294 nm: 2987.04, UVarea value at 304 nm: 2692.68).

The recovery rate was calculated by the following formula according to the recovery rate calculation method.

$$\text{Recovery rate} = 0.293 \times (1 / 0.369 - 339.39 \times 0.001 + 605.78 \times 0.001) = 87.2\%$$

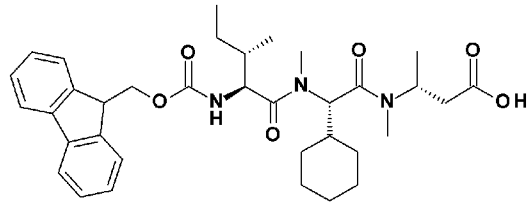

**35** (Deresinated product)

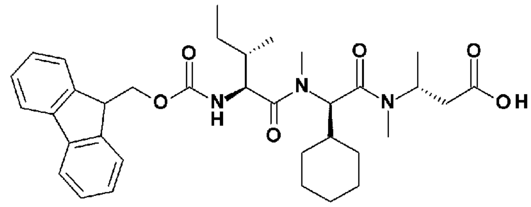

**35a** (Epimer)

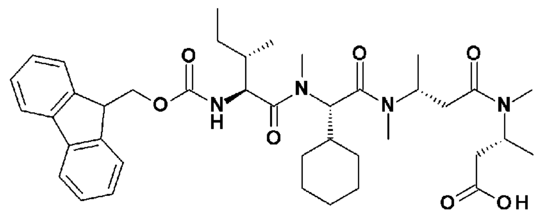

**35b** (Excessively elongated product)

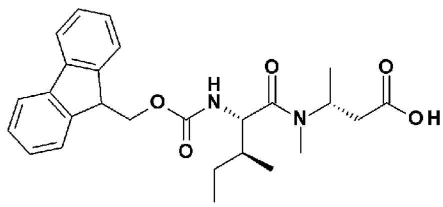

**34c** (Defective product)

When the cleaving reaction solution was analyzed by LCMS, it was observed that the purity of a deresinated product of Compound 35 was 92.8 area%, an excessively elongated product (Compound 35b) was 3.4 area%, and a defective product (Compound 34c) was 3.8 area%.

Analysis Condition: HPLC method F

[Table 40]

| | Mw | m/z | Rt (min) | LC A% |
|---|---|---|---|---|
| Deresinated product (Compound 35) | 605. 78 | 606. 5 ([M+H]$^+$) | 3. 03 | 92. 8 |
| Epimer (Compound 35a) | 605. 78 | Not detected | | |
| Excessively elongated product (Compound 35b) | 704. 91 | 705. 6 ([M+H]$^+$) | 2. 96 | 3. 4 |
| Defective product (Compound 34c) | 452. 56 | 453. 5 ([M+H]$^+$) | 2. 52 | 3. 8 |

Synthesis of Compound 35 by Condition 2 (Synthesis Method 1)

Using the amino acid-loaded resin (Compound 24) (Fmoc-D-3-MeAbu-O-Trt(2-Cl)resin) (100 mg, 0.369 mmol/g) prepared in Synthesis of Solid phase raw material 3 as a starting material, elongation of Fmoc-MeChg-OH (Oxyma, 50°C, 10 hours) and then elongation of Fmoc-Ile-OH (HOAt, 40°C, 2.5 hours) were carried out according to Synthesis Method 1 to synthesize Compound 35. Using a dry resin (9.56 mg), the loading rate was calculated by the Fmoc quantification method and was 0.217 mmol/g (UVarea value at 294 nm: 2072.81, UVarea value at 304 nm: 1864.82).

The recovery rate was calculated by the following formula according to the recovery rate calculation method.

$$\text{Recovery rate} = 0.217 \times (1 / 0.369 - 339.39 \times 0.001 + 605.78 \times 0.001) = 64.6\%$$

When the cleaving reaction solution was analyzed by LCMS, it was observed that the purity of a deresinated product of Compound 35 was 94.5 area%, and an excessively elongated product (Compound 35b) was 5.5 area%.

Analysis Condition: HPLC method F

[Table 41]

| | Mw | m/z | Rt (min) | LC A% |
|---|---|---|---|---|
| Deresinated product (Compound 35) | 605. 78 | 606. 6 ([M+H]$^+$) | 2. 97 | 94. 5 |
| Epimer (Compound 35a) | 605. 78 | Not detected | | |
| Excessively elongated product (Compound 35b) | 704. 91 | 705. 7 ([M+H]$^+$) | 2. 92 | 5. 5 |

The above results are summarized in the table below.

[Table 42]

| | Recovery rate (%) | Deresinated product, Compo und 35 (LC A%) | Epimer, Compound 35a (LC A%) | Excessively elonga ted product, Comp ound 35b (LC A%) | Defective product, Compound 34c (LC A%) |
|---|---|---|---|---|---|
| Condition 1 | 87. 2 | 92. 8 | - | 3.4 | 3. 8 |
| Condition 2 | 64. 6 | 94. 5 | - | 5. 5 | - |

[Example 2-11]

Synthesis of Fmoc-Ile-MeVal-MeGly-O-Trt(2-Cl)resin (Compound 36)

Compound 26

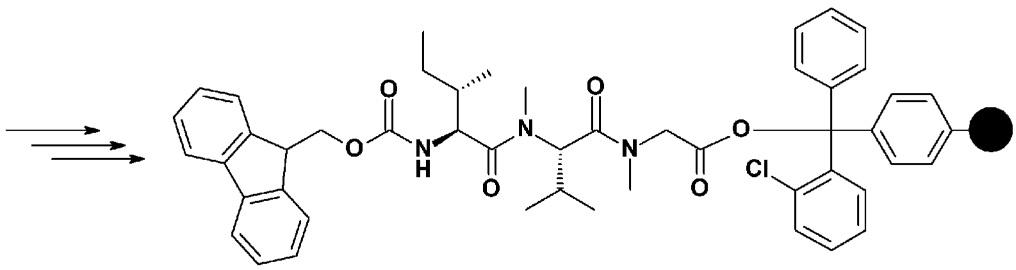

36

Synthesis of Compound 36 by Condition 1 (Synthesis Method 1)

Using the amino acid-loaded resin (Compound 26) (Fmoc-MeGly-O-Trt(2-Cl)resin ) (100 mg, 0.573 mmol/g) prepared in Synthesis of Solid phase raw material 4 as a starting material, elongation of Fmoc-MeVal-OH (HOAt, 40°C, 2.5 hours) and then elongation of Fmoc-Ile-OH (HOAt, 40°C, 2.5 hours) were carried out according to Synthesis Method 1 to synthesize Compound 36. Using a dry resin (10.20 mg), the loading rate was calculated by the Fmoc quantification method and was 0.326 mmol/g (UVarea value at 294 nm: 3322.12, UVarea value at 304 nm: 3002.34).
The recovery rate was calculated by the following formula according to the recovery rate calculation method.

$$\text{Recovery rate} = 0.326 \times (1 / 0.573 - 311.34 \times 0.001 + 537.66 \times 0.001) = 64.3\%$$

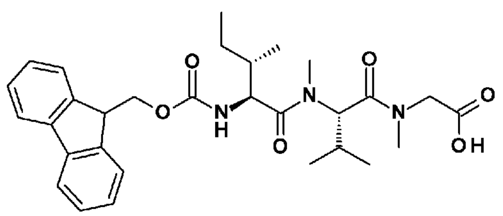

**36** (Deresinated product)

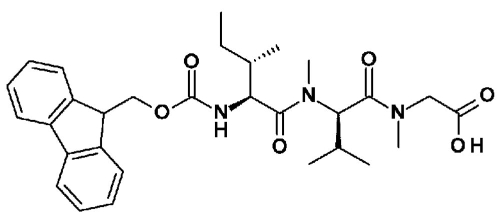

**36a** (Epimer)

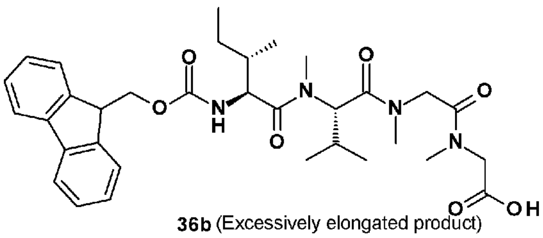

36b (Excessively elongated product)

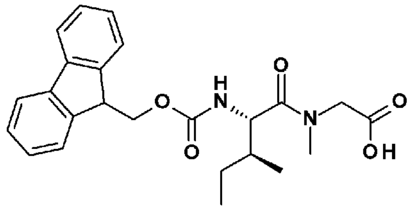

36c (Defective product)

When the cleaving reaction solution was analyzed by LCMS, it was observed that the purity of a deresinated product of Compound 36 was 95.6 area%, and an excessively elongated product (Compound 36b) was 4.4 area%. With this substrate, a defective product (Compound 36c) was not observed even under Condition 1.

Analysis Condition: HPLC METHOD E

[Table 43]

| | Mw | m/z | Rt (min) | LC A% |
|---|---|---|---|---|
| Deresinated product (Compound 36) | 537. 66 | 538. 5 ([M+H]$^+$) | 0. 94 | 95. 6 |
| Epimer (Compound 36a) | 537. 66 | Not detected | | |
| Excessively elongated product (Compound 36b) | 608. 74 | 607. 4 ([M+H]$^+$) | 0. 90 | 4. 4 |
| Defective product (Compound 36c) | 424. 50 | Not detected | | |

<u>Synthesis of Compound 36 by Condition 2 (Synthesis Method 1)</u>

Using the amino acid-loaded resin (Compound 26) (Fmoc-MeGly-O-Trt(2-Cl)resin) (100 mg, 0.573 mmol/g) prepared in Synthesis of Solid phase raw material 4 as a starting material, elongation of Fmoc-MeVal-OH (Oxyma, 50°C, 10 hours) and then elongation of Fmoc-Ile-OH (HOAt, 40°C, 2.5 hours) were carried out according to Synthesis Method 1 to synthesize Compound 36. Using a dry resin (10.69 mg), the loading rate was calculated by the Fmoc quantification method and was 0.331 mmol/g (UVarea value at 294 nm: 3542.66, UVarea value at 304 nm: 3189.92).

The recovery rate was calculated by the following formula according to the recovery rate calculation method.

$$\text{Recovery rate} = 0.331 \times (1 / 0.573 - 311.34 \times 0.001 + 537.66 \times 0.001) = 65.3\%$$

When the cleaving reaction solution was analyzed by LCMS, it was observed that the purity of a deresinated product of Compound 36 was 93.9 area% and an excessively elongated product (Compound 36b) was 6.1 area%.

Analysis Condition: HPLC METHOD E

[Table 44]

| | Mw | m/z | Rt (min) | LC A% |
|---|---|---|---|---|
| Deresinated product (Compound 36) | 537. 66 | 538. 5 ([M+H]$^+$) | 0. 93 | 93. 9 |
| Epimer (Compound 36a) | 537. 66 | Not detected | | |
| Excessively elongated product (Compound 36b) | 608. 74 | 607. 6 ([M+H]$^+$) | 0. 89 | 6. 1 |

The above results are summarized in the table below.

[Table 45]

| | Recovery rate (%) | Deresinated product, Compo und 36 (LC A%) | Epimer, Compound 36a (LC A%) | Excessively elonga ted product, Comp ound 36b (LC A%) | Defective product, Compound 36c (LC A%) |
|---|---|---|---|---|---|
| Condition 1 | 64. 3 | 95. 6 | - | 4. 4 | - |
| Condition 2 | 65. 3 | 93. 9 | - | 6. 1 | - |

Verification of premature cleavage suppressing effect of Example Condition A with various sequences

[Example 2-12]

Synthesis of Fmoc-MeVal-MeLeu-OCTC (Compound 39) (Synthesis by Example Condition A)

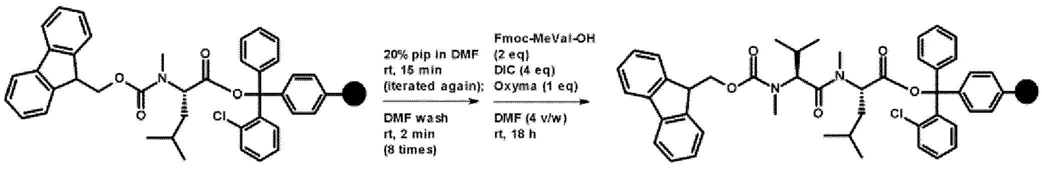


38

39

First, 0.195 g (0.650 mmol/g, 0.127 mmol, 1.0 eq) of a dry resin (Compound 38) was weighed into a 5 mL filter-equipped disposable syringe, 1.6 mL (10 v/w) of N,N-dimethylformamide was added, and the mixture was left to stand at room temperature for 30 minutes. After N,N-dimethylformamide was discharged, 1.3 mL (8 v/w) of an N,N-dimethylformamide solution containing 20% piperidine was added, and the mixture was shaken at room temperature for 15 minutes. After the reaction solution was discharged, 1.3 mL (8 v/w) of an N,N-dimethylformamide solution containing 20% piperidine was added again, and the mixture was shaken at room temperature for 15 minutes. After the reaction solution was discharged, the resin was washed with 1.6 mL (10 v/w) of N,N-dimethylformamide eight times. Next, 90 mg (0.25 mmol, 2.0 eq) of Fmoc-MeVal-OH and 18 mg (0.13 mmol, 1.0 eq) of Oxyma were weighed into a vial and dissolved by adding 0.62 mL (4 v/w) of N,N-dimethylformamide. Immediately after 80 μL (0.51 mmol, 4.0 eq) of DIC was added to this solution, the mixture was sucked into the disposable syringe, and the syringe was sealed and then shaken at room temperature for 18 hours. After the reaction solution was discharged, the resin was washed with 1.6 mL (10 v/w) of N,N-dimethylformamide four times, with 1.6 mL (10 v/w) of 2-propanol twice and with 1.6 mL (10 v/w) of methanol four times, and then dried under reduced pressure at room temperature overnight to give 0.201 g of a dry resin (Compound 39). Then, 16.7 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance of the solution using a spectrophotometer, and the yield was calculated from the resulting Fmoc quantitative value, the mass of the dry resin, and LC A%.

[Table 46]

| (HPLC method I: Identification of target molecule and purity measurement) | | | | | |
|---|---|---|---|---|---|
| | Mass (g) | Fmoc quantitative value (mmollg) | LC A% | Amount of substance (mmol) | Yield (%) |
| Starting material (Compound 38) | 0. 195 | 0. 650 | 100 | 0. 127 | - |
| Product (Compound 39) | 0.201 | 0. 494 | 98.6 | 0. 0978 | 77 |

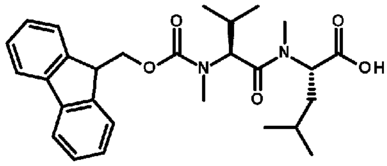

39 (Deresinated product)

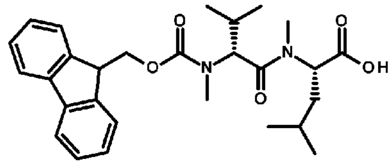

39a (Epimer)

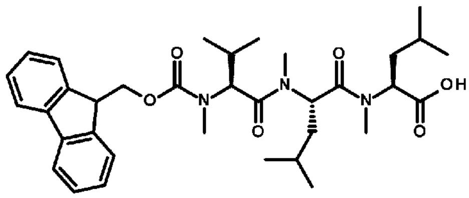

39b (Excessively elongated product)

[Table 47]

| (HPLC method I: Purity calculation) | | | | |
|---|---|---|---|---|
| | Mw | m/z | Rt (min) | LC A% |
| Deresinated product (Compound 39) | 480.3 | 481.2 ([M+H]$^+$) | 13.5 | 98.6 |
| Epimer (Compound 39a) | 480.3 | not detected | 13.7 | not detected |
| Excessively elongated product (Compound 39b) | 607.4 | not detected | not detected | not detected |

Glycine capping was carried out to verify the residual ratio of the starting material. More specifically, about 10 mg of the dry resin that had undergone a condensation reaction was weighed into a 5 mL filter-equipped disposable syringe, 1 mL of N,N-dimethylformamide was added, and the mixture was left to stand at room temperature for 15 minutes. After N,N-dimethylformamide was discharged, a cocktail for reaction (a light yellow uniform solution obtained by weighing about 70 mg of Fmoc-Gly-OH and about 0.11 g of HATU into a vial, suspending them in 0.45 mL of N,N-dimethylformamide, then adding 45 μL of N,N-diisopropylethylamine, and shaking the mixture for 1 minute) was sucked into the disposable syringe, and shaken at room temperature for 1 hour. After the reaction solution was discharged, the resin was washed with 1 mL of N,N-dimethylformamide six times and with 1 mL of dichloromethane four times, and then a deresination reaction was carried out. The residual ratio of the starting material calculated from the sum of LC A% of the Gly-cap product (Compound 40), the target molecule (Compound 39), the epimer (Compound 39a), and the excessively elongated product (Compound 39b) was 0% (0 / (0 + 98.6 + 0 + 0)).

[Table 48]

| (HPLC method H-b: Calculation of reaction conversion rate) | | |
|---|---|---|
| | Rt (min) | LC A% |
| Gly-cap product (Compound 40) | Not detected | Not detected |
| Deresinated product (Compound 39) Epimer (Compound 39a) | 3.11 | 98.2 |
| Excessively elongated product (Compound 39b) | Not detected | Not detected |

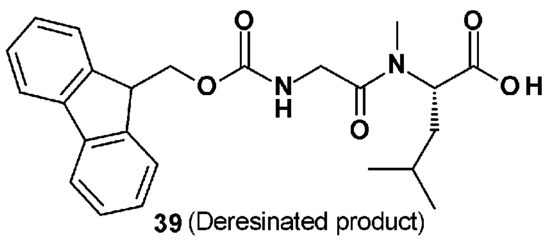

39 (Deresinated product)

The deresinated product (Compound 39) could be obtained in a good yield. No formation of the excessively elongated product (Compound 39b) and the epimer (Compound 39a) was confirmed.

[Reference Example 4]

Synthesis of Fmoc-MeVal-MeLeu-OCTC (Compound 39) under reaction condition described in Chem. Eur. J., 2009, 15, 9394-9403 (Reaction condition having Fmoc amino acid : DIC : Additive (Oxyma) = 2 : 2 : 2 relative to amino acid onto resin: hereinafter referred to as

Reference Example Condition A)

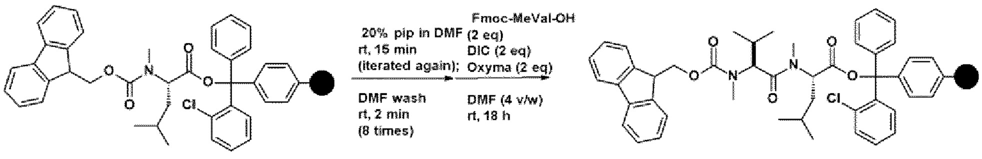


38 39

First, 0.204 g (0.650 mmol/g, 0.133 mmol, 1.0 eq) of a dry resin (Compound 38) was weighed into a 5 mL filter-equipped disposable syringe, 1.6 mL (10 v/w) of N,N-dimethylformamide was added, and the mixture was left to stand at room temperature for 30 minutes. After N,N-dimethylformamide was discharged, 1.3 mL (8 v/w) of an N,N-dimethylformamide solution containing 20% piperidine was added, and the mixture was shaken at room temperature for 15 minutes. After the reaction solution was discharged, 1.3 mL (8 v/w) of an N,N-dimethylformamide solution containing 20% piperidine was added again, and the mixture was shaken at room temperature for 15 minutes. After the reaction solution was discharged, the resin was washed with 1.6 mL (10 v/w) of N,N-dimethylformamide eight times. Next, 96 mg (0.26 mmol, 2.0 eq) of Fmoc-MeVal-OH and 38 mg (0.26 mmol, 2.0 eq) of Oxyma were weighed into a vial and dissolved by adding 0.65 mL (4 v/w) of N,N-dimethylformamide. Subsequently, 42 μL (0.26 mmol, 2.0 eq) of DIC was added to the vial at room temperature, and then the vessel was sealed and shaken at room temperature for 2 minutes. This solution was sucked into the disposable syringe, and the syringe was sealed and then shaken at room temperature for 18 hours. After the reaction solution was discharged, the resin was washed with 1.6 mL (10 v/w) of N,N-dimethylformamide four times, with 1.6 mL (10 v/w) of 2-propanol twice and with 1.6 mL (10 v/w) of methanol four times, and then dried under reduced pressure at room temperature overnight to give 0.181 g of a dry resin (Compound 3). Then, 19.0 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance of the solution using a spectrophotometer, and the yield was calculated from the resulting Fmoc quantitative value, the mass of the dry resin, and LC A%.

[Table 49]

| (HPLC method E-b: Identification of target molecule and purity measurement) | | | | | |
|---|---|---|---|---|---|
| | Mass (g) | Fmoc quantitative value (mmol/g) | LC A% | Amount of sub stance (mmol) | Yield (%) |
| Starting material (Compound 38) | 0.204 | 0.650 | 100 | 0.133 | - |
| Product (Compound 39) | 0.181 | 0.130 | 79.8 | 0.0188 | 14 |

Using the same procedure as Example Condition A, the starting material residual ratio was also confirmed as being 0% under Reference Example Condition A.

[Table 50]

| (HPLC method H-b: Calculation of reaction conversion rate) | | |
|---|---|---|
| | Rt (m i n) | LC A% |
| Gly-cap product (Compound 40) | Not detected | Not detected |

(continued)

| (HPLC method H-b: Calculation of reaction conversion rate) | | |
|---|---|---|
| | Rt (m i n) | LC A% |
| Deresinated product (Compound 39) | 3.11 | 79.8 |
| Epimer (Compound 39a) | | |

The yield and the purity of the target molecule were found to be both decreased under the reaction condition of the above Reference Example Condition A.

Based on the above one-residue + one-residue condensation experiment, the following sequences were synthesized under Example Condition A and under Reference Example Condition A.

[Example 2-13]

Synthesis of Fmoc-D-MeVal-MeLeu-OCTC (Compound 41) (Synthesis by Example Condition A)

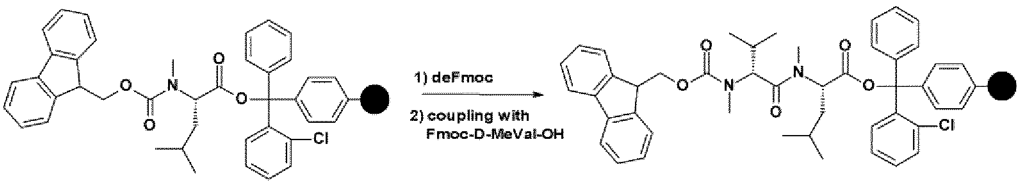


38

41

[Table 51]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.21 | 0.21 | 0 | 0.443 | 98.2 | 68 |

[Table 52]

| (HPLC method I: Identification of target molecule under Example Condition A) | | | | |
|---|---|---|---|---|
| | Mw | m/z | Rt (min) | LC A% |
| Deresinated product (41) | 480.3 | 481.3 ([M+H]$^+$) | 13.7 | 98.2 |

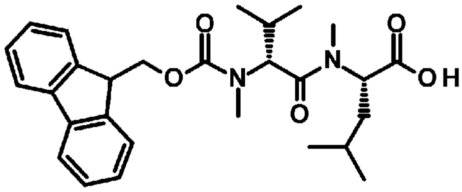

**41** (Deresinated product)

[Example 2-14]

Synthesis of Fmoc-Lys(Z)-MeVal-OCTC (Compound 43) (Synthesis by Example Condition A and by Reference Example Condition A)

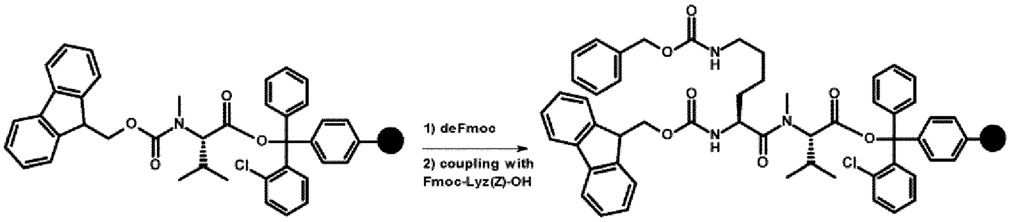


42

43

[Table 53]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.16 | 0.17 | 0 | 0.392 | 97.6 | 66 |
| Reference Example Condition A | 0.17 | 0.15 | 0 | 0.036 | 88.5 | 4 |

[Table 54]

| (HPLC method H-b: Identification of target molecule under Example Condition A) | | | | |
|---|---|---|---|---|
| | Mw | m/z | Rt (min) | LC A% |
| Deresinated product (43) | 615.3 | 616.3 ([M+H]$^+$) | 3.3 | 97.6 |

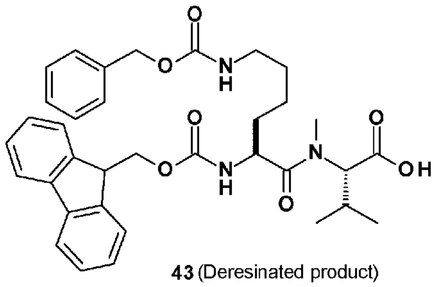

**43** (Deresinated product)

[Example 2-15]

Synthesis of Fmoc-MeVal-MeAla-OCTC (Compound 45) (Synthesis by Example Condition A and by Reference Example Condition A)

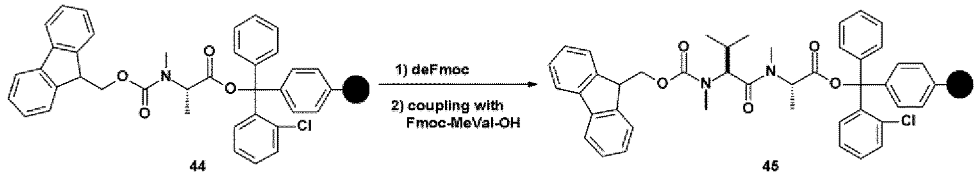


44 45

[Table 55]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.21 | 0 | 0.537 | 98.5 | 84 |
| Reference Example Condition A | 0.20 | 0.18 | 0 | 0.208 | 98.4 | 29 |

[Table 56]

| (HPLC method H-b: Identification of target molecule under Example Condition A) | | | | |
|---|---|---|---|---|
| | Mw | m/z | Rt (min) | LC A% |
| Deresinated product (45) | 438.2 | 439.2 ([M+H]$^+$) | 3.0 | 98.5 |

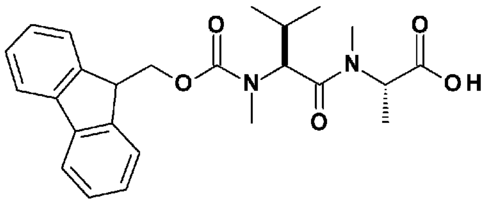

**45** (Deresinated product)

[Example 2-16]

Synthesis of Fmoc-MeVal-MeGly-OCTC (Compound 47) (Synthesis by Example Condition A and by Reference Example Condition A)

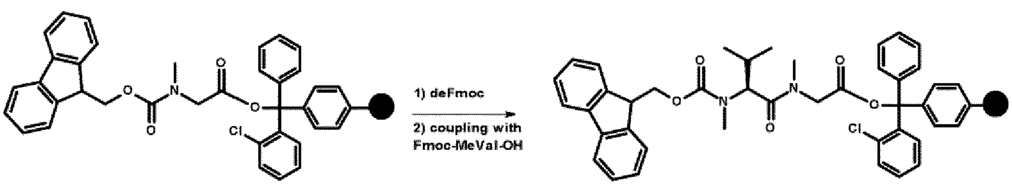


46 47

[Table 57]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmollg) | Purity (LC A%) | Yield (%) |
| Example Condition A | 0.20 | 0.21 | 0 | 0.516 | 95.7 | 81 |
| Reference Example Condition A | 0.20 | 0.19 | 0 | 0.395 | 91.2 | 57 |

[Table 58]

| (HPLC method H-b: Identification of target molecule under Example Condition A) | | | | |
|---|---|---|---|---|
| | Mw | m/z | Rt (min) | LC A% |
| Deresinated product (47) | 424.2 | 447.1 ([M+Na]$^+$) | 2.9 | 95.7 |

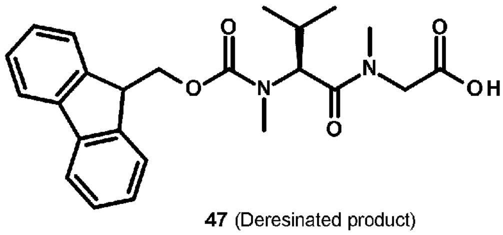

**47** (Deresinated product)

[Example 2-17]

Synthesis of Fmoc-MeVal-MePhe-OCTC (Compound 49) (Synthesis by Example Condition A and by Reference Example Condition A)

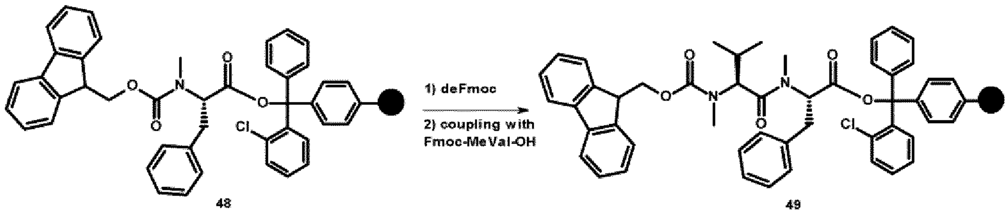


[Table 59]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.20 | 3 | 0.401 | 96.1 | 62 |
| Reference Example Condition A | 0.20 | 0. 17 | 0 | 0.066 | 48 | 4 |

[Table 60]

| (HPLC method H-b: Identification of target molecule under Example Condition A) | | | | |
|---|---|---|---|---|
| | Mw | m/z | Rt (min) | LC A% |
| Deresinated | product (49) 51.4.3 | ([M+H]+) 515.2 | 3.4 | 96.1 |

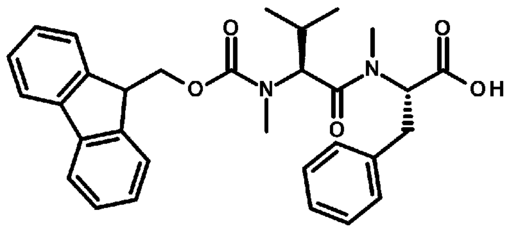

**49** (Deresinated product)

[Example 2-18]

Synthesis of Fmoc-Glu(OBzl)-MeTrp(Boc)-OCTC (Compound 51) (Synthesis by Example Condition A and by Reference Example Condition A)

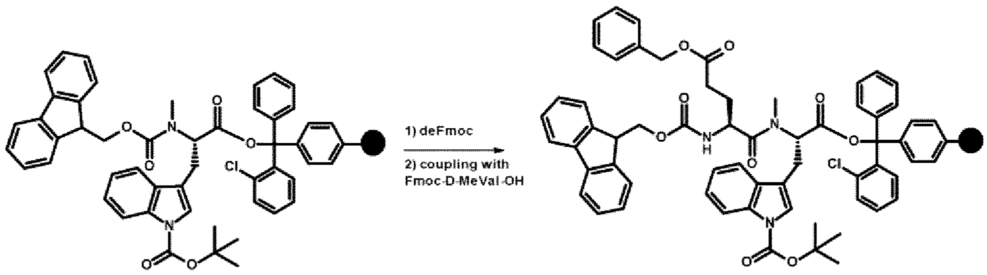


50

51

[Table 61]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.23 | 0 | 0.479 | 98.0 | 90 |
| Reference Example Condition A | 0.20 | 0.19 | 0 | 0.267 | 93.8 | 38 |

[Table 62]

| (HPLC method H-b: Identification of target molecule under Example Condition A) | | | | |
|---|---|---|---|---|
| | Mw | m/z | Rt (min) | LC A% |
| Deresinated product (51) | 759.3 | 704.2 ([M-C4H8+H]+) | 3.9 | 98.0 |

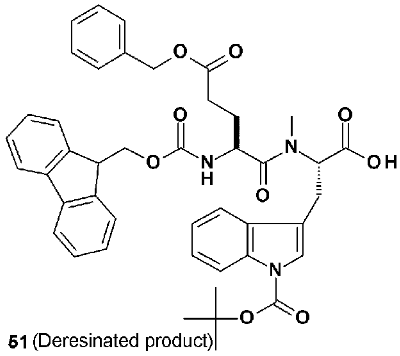

51 (Deresinated product)

[Example 2-19]

Synthesis of Fmoc-Aze(2)-MeThr(Bzl)-OCTC (Compound 53) (Synthesis by Example Condition A and by Reference Example Condition A)

[Table 63]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.21 | 0 | 0.432 | 97.4 | 70 |
| Reference Example Condition A | 0.20 | 0.17 | 0 | 0.107 | 92.9 | 14 |

[Table 64]

| (HPLC method H-b: Identification of target molecule under Example Condition A) | | | | |
|---|---|---|---|---|
| | Mw | m/z | Rt (min) | LC A% |
| Deresinated product (53) | 528.2 | 529.2 ([M+H]$^+$) | 3.2 | 97.4 |

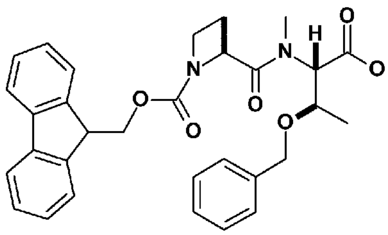

53 (Deresinated product)

[Example 2-20]

Synthesis of Fmoc-Lys(Z)-MeGlu(OtBu)-OCTC (Compound 55) (Synthesis by Example Condition A and by Reference Example Condition A)

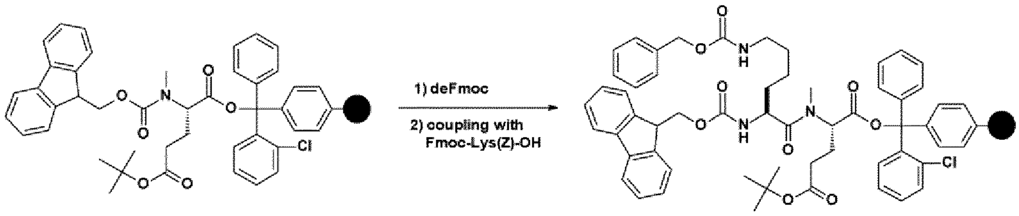


54 55

[Table 65]

| HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.24 | 0 | 0.378 | 98.3 | 74 |
| Reference Example Condition A | 0.20 | 0.19 | 0 | 0.148 | 93.3 | 21 |

[Table 66]

| (HPLC method H-b: Identification of target molecule under Example Condition A) | | | | |
|---|---|---|---|---|
| | Mw | m/z | Rt (min) | LC A% |
| Deresinated product (55) | 701.3 | 724.4 ([M+Na]$^+$) | 3.4 | 98.3 |

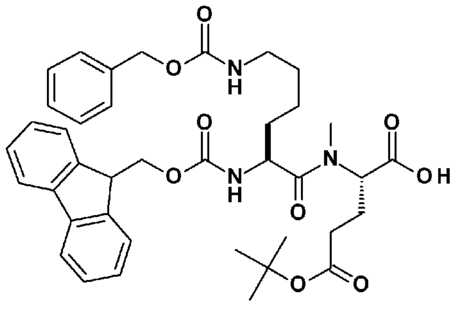

**55** (Deresinated product)

[Example 2-21]

Synthesis of Fmoc-Thr(tBu)-MeLys(Boc)-OCTC (Compound 57) (Synthesis by Example Condition A and by Reference Example Condition A)

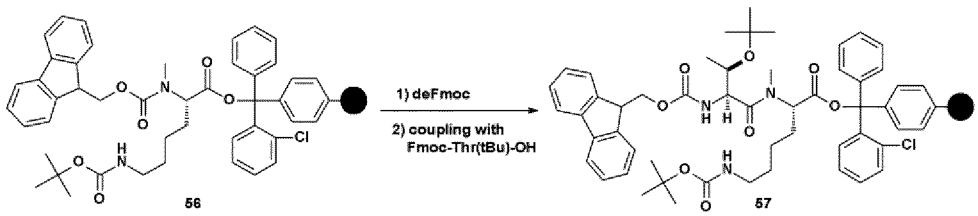


56 57

[Table 67]

| HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.21 | 0 | 0.426 | 97.0 | 75 |
| Reference Example Condition A | 0.20 | 0.18 | 0 | 0.207 | 92.4 | 31 |

[Table 68]

| (HPLC method H-b: Identification of target molecule under Example Condition A) | | | | |
|---|---|---|---|---|
| | Mw | m/z | Rt (min) | LC A% |
| Deresinated product (57) | 639.4 | 662.4 ([M+Na]+) | 3.5 | 97.0 |

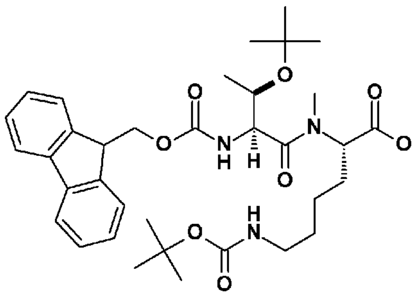

**57** (Deresinated product)

[Example 2-22]

Synthesis of Fmoc-MePhe-MeMet-OCTC (Compound 59) (Synthesis by Example Condition A and by Reference Example Condition A)

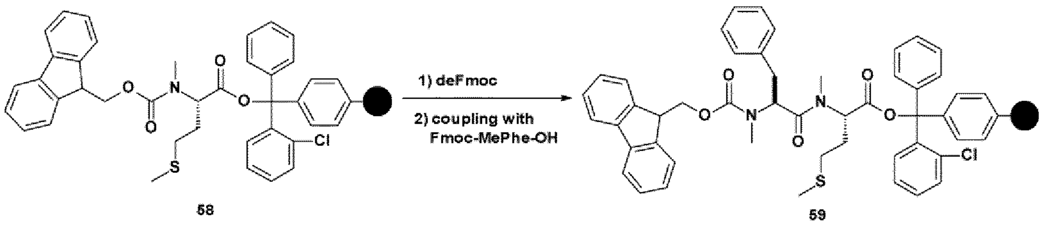


[Table 69]

| HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.21 | 0.22 | 0 | 0.450 | 96.7 | 76 |

(continued)

| HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Reference Example Condition A | 0.20 | 0.19 | 0 | 0.185 | 93.1 | 24 |

[Table 70]

| (HPLC method H-b: Identification of target molecule under Example Condition A) | | | | |
|---|---|---|---|---|
| | Mw | m/z | Rt (min) | LC A% |
| Deresinated product (34) | 546.2 | 547.1 ([M+H]$^+$) | 3.4 | 96.7 |

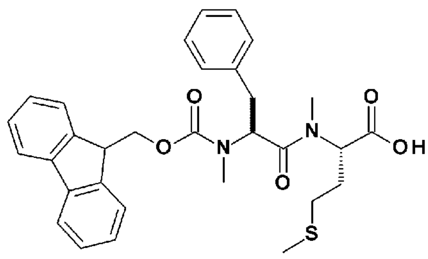

59 (Deresinated product)

[Example 2-23]

Synthesis of Fmoc-cVal-Aze(2)-OCTC (Compound 61) (Synthesis by Example Condition A and by Reference Example Condition A)

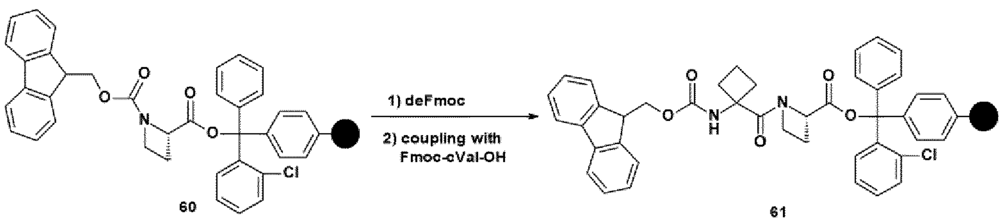


[Table 71]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.19 | 0.19 | 0 | 0.411 | 93.1 | 64 |
| Reference Example Condition A | 0.19 | 0.18 | 0 | 0.267 | 87.3 | 35 |

[Table 72]

| (HPLC method H-b: Identification of target molecule under Example Condition A) | | | | |
|---|---|---|---|---|
| | Mw | m/z | Rt (min) | LC A% |
| Deresinated product (61) | 420.2 | 421.2 ([M+H]+) | 2.7 | 93.1 |

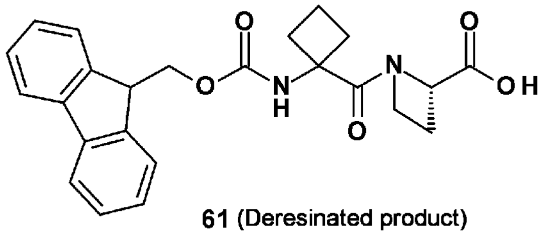

61 (Deresinated product)

[Example 2-24]

Synthesis of Fmoc-Gly-Gly-OCTC (Compound 63) (Synthesis by Example Condition A and by Reference Example Condition A)

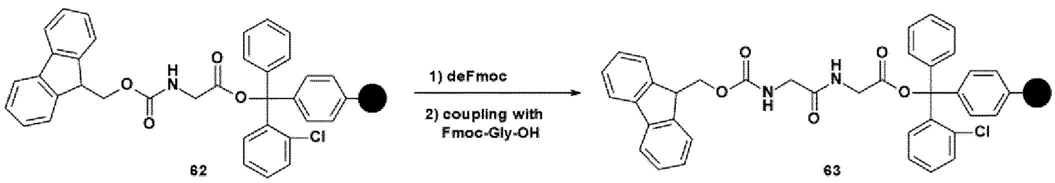


[Table 73]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.19 | 0 | 0.474 | 93.5 | 64 |
| Reference Example Condition A | 0.20 | 0.18 | 0 | 0.379 | 97.1 | 54 |

[Table 74]

| (HPLC method H-b: Identification of target molecule under Example Condition A) | | | | |
|---|---|---|---|---|
| | Mw | m/z | Rt (min) | LC A% |
| Deresinated product (62) | 354.1 | 355.0 ([M+H]+) | 2.4 | 93.5 |

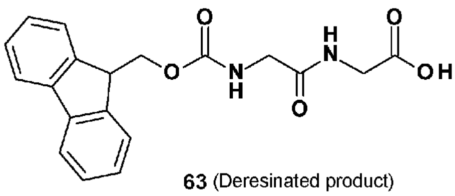

63 (Deresinated product)

[Table 75]

| Entry | Condition | Reaction time | Yield (%) |
|---|---|---|---|
| 1 | Example Condition A | 18 hours | 64 |
| 2 | Example Condition A | 1 hour | 67 |
| 3 | Reference Example Condition A | 1 hour | 61 |

For Entries 2 and 3, the reaction time was shortened from 18 hours to 1 hour.

[Example 2-25]

Synthesis of Fmoc-Gly-Leu-OCTC (Compound 65) (Synthesis by Example Condition A and by Reference Example Condition A)

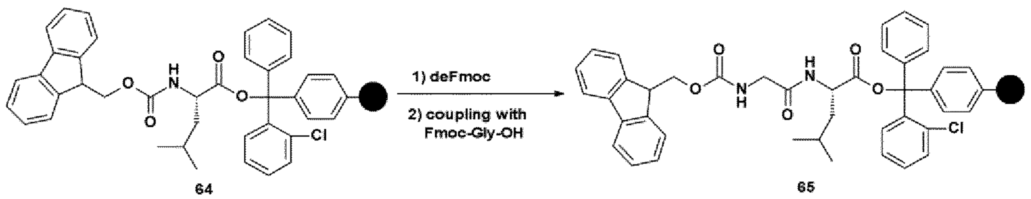


64 65

[Table 76]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.20 | 0 | 0.515 | 96.1 | 76 |
| Reference Example Condition A | 0.20 | 0.19 | 0 | 0.427 | 96.6 | 62 |

[Table 77]

| (HPLC method H-b: Identification of target molecule under Example Condition A) | | | | |
|---|---|---|---|---|
| | Mw | m/z | Rt (min) | LC A% |
| Deresinated product (65) | 410.2 | 411.2 ([M+H]$^+$) | 2.9 | 96.1 |

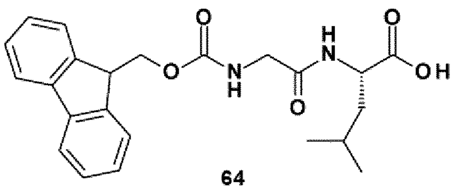

64

[Table 78]

| Entry | Condition | Reaction time | Yield (%) |
|---|---|---|---|
| 1 | Example Condition A | 18 hours | 76 |
| 2 | Example Condition A | 1 hour | 76 |
| 3 | Reference Example Condition A | 1 hour | 72 |

For Entries 2 and 3, the reaction time was shortened from 18 hours to 1 hour.

[Example 2-26]

Synthesis of Fmoc-Pro-Aib-OCTC (Compound 67) (Synthesis by Example Condition A and by Reference Example Condition A)

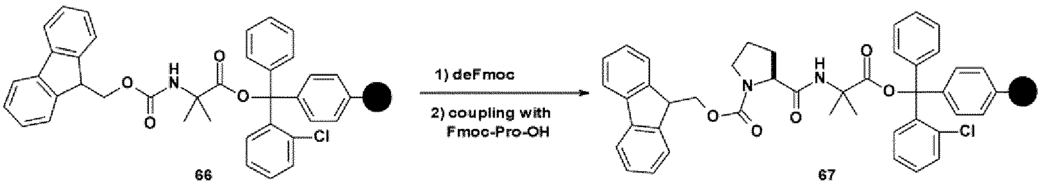


[Table 79]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0.20 | 0.22 | 0 | 0. 502 | 99. 1 | 84 |
| Reference Example Condition A | 0.20 | 0.22 | 0 | 0. 459 | 99.5 | 75 |

[Table 80]

| (HPLC method H-b: Identification of target molecule under Example Condition A) | | | | |
|---|---|---|---|---|
| | Mw | m/z | Rt (min) | LC A% |
| Deresinated product (67) | 422.2 | 423.2 ([M+H]+) | 2.7 | 99. 1 |

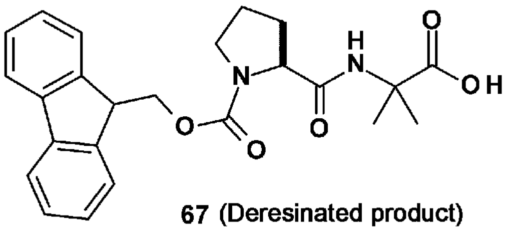

67 (Deresinated product)

[Example 2-27]

Synthesis of Fmoc-Gly-bAla-OCTC (Compound 69) (Synthesis by Example Condition A and by Reference Example Condition A)

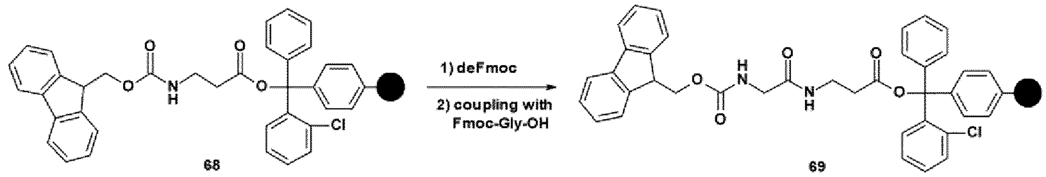


68 69

[Table 81]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 0. 23 | 0.26 | 0 | 0.555 | 98. 3 | 92 |
| Reference Example Condition A | 0.21 | 0.23 | 0 | 0.523 | 97. 8 | 83 |

[Table 82]

| (HPLC method H-b: Identification of target molecule under Example Condition A) | | | | |
|---|---|---|---|---|
| | Mw | m/z | Rt (min) | LC A% |
| Deresinated product (69) | 368.1 | 369.2 ([M+H]$^+$) | 2. 4 | 98. 3 |

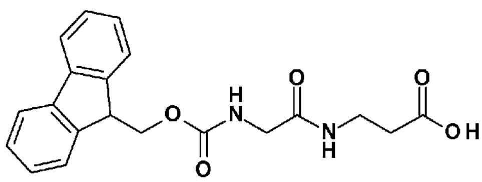

**69** (Deresinated product)

[Example 2-28]

Synthesis of Fmoc-MeVal-bMeAla-OCTC (Compound 71)

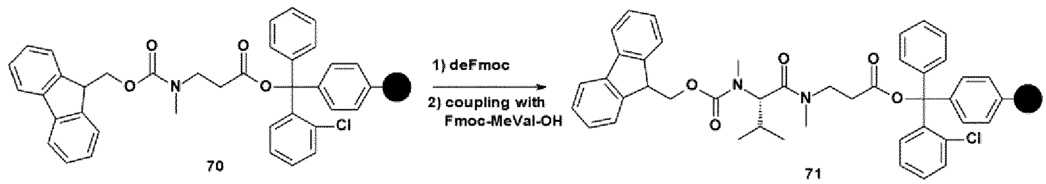


70 71

[Table 83]

| HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 3. 75 | 3.52 | 0 | 0.556 | 98. 3 | 88 |
| Reference Example Condition A | 0. 97 | 0. 89 | 0 | 0. 532 | 98. 5 | 83 |

Elongation of this sequence was carried out, with three changes being made to Example Condition A.

[Table 84]

| | Example Condition A | Elongation of this sequence |
|---|---|---|
| Additive | 0xyma | 0xyma-B |
| Amount of condensation medium | 4 v/w | 8 v/w |
| Reaction time | 18 hours | 3 hours |

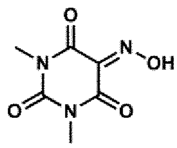

Oxyma B

[Table 85]

| (HPLC method H-b: Identification of target molecule in elongation reaction of this sequence) | | | | |
|---|---|---|---|---|
| | Mw | m/z | Rt (min) | LC A% |
| Deresinated product (71) | 438.2 | 439.2 ([M+H]$^+$) | 2.9 | 98.3 |

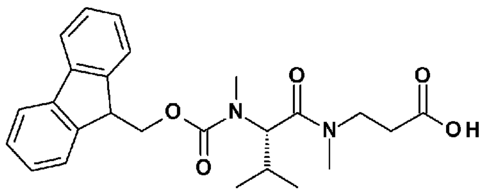

**71** (Deresinated product)

[Example 2-29]

Synthesis of Fmoc-MeVal-D-3-MeAbu-OCTC (Compound 73) (Synthesis by Example Condition A and by Reference Example Condition A)

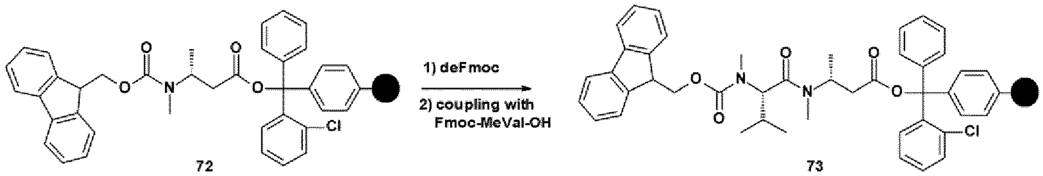


72

73

[Table 86]

| (HPLC method H-b: Calculation of reaction conversion rate and purity) | | | | | | |
|---|---|---|---|---|---|---|
| Condition | Raw resin (g) | Resin after reaction (g) | Residual ratio of starting material (%) | Fmoc quantitative value (mmol/g) | Purity (LC A%) | Yield (%) |
| Example Condition A | 6.05 | 5.98 | 0 | 0.586 | 89.7 | 86 |
| Reference Example Condition A | 0.99 | 0.93 | ND | 0.457 | No data | 70 |
| (ND: not determined, yield under Reference Example Condition A was determined by using amount of substance calculated from resin mass and Fmoc quantitative value, without taking purity into account.) | | | | | | |

Elongation of this sequence was carried out, with one change being made to Example Condition A.

[Table 87]

| | Example Condition A | Elongation of this sequence |
|---|---|---|
| Reaction time | 18 hours | 3 hours |

[Table 88]

| (HPLC method J: Identification of target molecule under Example Condition A) | | | | |
|---|---|---|---|---|
| | Mw | m/z | Rt (min) | LC A% |
| Deresinated product (73) | 452.2 | 453.2 ($[M+H]^+$) | 8.9 | 89.7 |

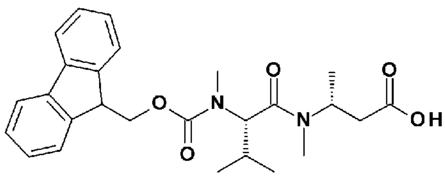

**73** (Deresinated product)

[Example 2-30]

Synthesis of Fmoc-Ser(tBu)-MeLeu-OCTC (Compound 75) (Synthesis by Example Condition A and Reference Example Condition A)

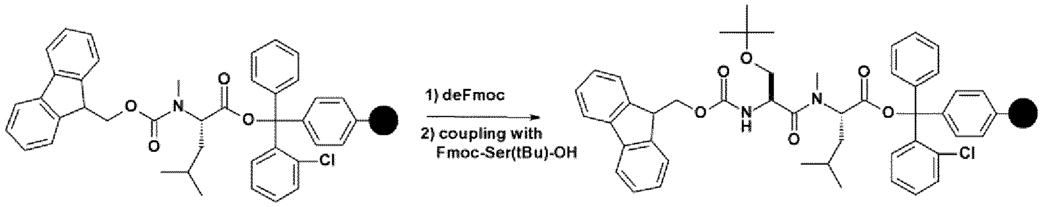


74 75

[Table 89]

| (HPLC method H-b: Calculation of reaction conversion rate and purity) | | | | | | |
|---|---|---|---|---|---|---|
| Condition | Raw resin (g) | Resin after reaction (g) | Residual ratio of raw material (%) | Fmoc quantitative value (mmol/g) | Purity (LC A%) | Yield (%) |
| Example Condition A | 0.21 | 0.23 | 0 | 0.491 | 98. 9 | 83 |
| Reference Example Condition A | 0.21 | 0.21 | 0 | 0.281 | 98.0 | 41 |

[Table 90]

| (HPLC method H: Identification of target molecule under Example Condition A) | | | | |
|---|---|---|---|---|
| | Mw | m/z | Rt (min) | LC A% |
| Deresinated product (75) | 510.3 | 533.2 ([M+Na]$^+$) | 3.4 | 98.9 |

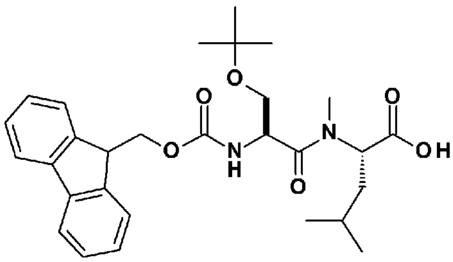

**75** (Deresinated product)

The above results are summarized in the table below.

[Table 91]

| Entry | Condition | 2$^{nd}$ Amino acid | 1$^{st}$ Amino acid | Purity (LC A%) | Yield (%) |
|---|---|---|---|---|---|
| 1 | Example Condition A | MeVal | MeLeu | 98. 6 | 77 |
| 2 | Reference Example Condition A | | | 79.8 | 14 |
| 3 | Example Condition A | D-MeVal | MeLeu | 98.2 | 68 |
| 4 | Example Condition A | Lys (Z) | MeVal | 97.6 | 66 |
| 5 | Reference Example Condition A | | | 88.5 | 4 |
| 6 | Example Condition A | MeVal | MeAla | 98.5 | 84 |
| 7 | Reference Example Condition A | | | 98.4 | 29 |

(continued)

| Entry | Condition | 2nd Amino acid | 1st Amino acid | Purity (LC A%) | Yield (%) |
|---|---|---|---|---|---|
| 8 | Example Condition A | MeVal | MeGly | 95.7 | 81 |
| 9 | Reference Example Condition A | | | 91. 2 | 57 |
| 10 | Example Condition A | MeVal | MePhe | 96.1 | 62 |
| 11 | Reference Example Condition A | | | 48 | 4 |
| 12 | Example Condition A | Glu(0Bzl) | MeTrp (Boc) | 98.0 | 90 |
| 13 | Reference Example Condition A | | | 93.8 | 38 |
| 14 | Example Condition A | Aze (2) | MeThr(Bzl) | 97.4 | 70 |
| 15 | Reference Example Condition A | | | 92.9 | 14 |
| 16 | Example Condition A | Lys (Z) | MeGlu(0tBu) | 98.3 | 74 |
| 17 | Reference Example Condition A | | | 93.3 | 21 |
| 18 | Example Condition A | Thr (tBu) | MeLys (Boc) | 97.0 | 75 |
| 19 | Reference Example Condition A | | | 92.4 | 31 |
| 20 | Example Condition A | MePhe | MeMet | 96.7 | 76 |
| 21 | Reference Example Condition A | | | 93.1 | 24 |
| 22 | Example Condition A | cVal | Aze (2) | 93.1 | 64 |
| 23 | Reference Example Condition A | | | 87.3 | 35 |
| 24 | Example Condition A | Gly | Gly | 93.5 | 64 |
| 25 | Reference Example Condition A | | | 97.1 | 54 |
| 26 | Example Condition A | Gly | Leu | 96.1 | 76 |
| 27 | Reference Example Condition A | | | 96.6 | 62 |
| 28 | Example Condition A | Pro | Aib | 99.1 | 84 |
| 29 | Reference Example Condition A | | | 99.5 | 75 |
| 30 | Example Condition A | Gly | bAla | 98.3 | 92 |
| 31 | Reference Example Condition A | | | 97.8 | 83 |
| 32 | Example Condition A | MeVal | bMeAla | 98.3 | 88 |
| 33 | Reference Example Condition A | | | 98.5 | 83 |
| 34 | Example Condition A | MeVal | D-3-MeAbu | 89.7 | 86 |
| 35 | Reference Example Condition A | | | ND | 70 |
| 36 | Example Condition A | Ser (tBu) | MeLeu | 98.9 | 83 |
| 37 | Reference Example Condition A | | | 98.0 | 41 |

In any sequence, Example Condition A suppressed premature cleavage more than Reference Example Condition A did, and provided a two-residue peptide in a good yield. In particular, the suppressing effect in the cases of N-Me $\alpha$ amino acids was significant.

[Example 2-31]

Experiment of searching for equivalent amount range of reagent that provides premature cleavage suppressing effect

Below shows the results of carrying out the same reaction as the above [one-residue + one-residue condensation experiment] (Example Condition A) except that the equivalent amount of reagent was changed.

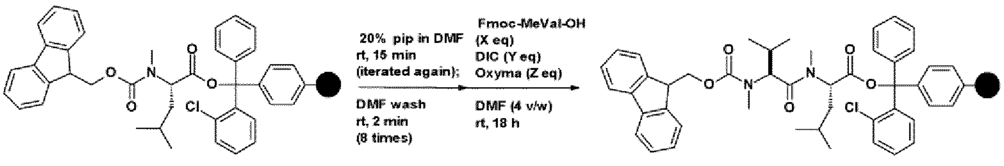


38 39

[Table 92]

| Entry | Fmoc-MeVal-OH (X eq) | DIC (Y eq) | Oxyma (Z eq) | Residual ratio of starting material (%) | Purity (LC A%) | Yield (%) |
|---|---|---|---|---|---|---|
| 1 | 2 | 2 | 2 | 0 | 79.8 | 14 |
| 2 | 2 | 4 | 1 | 0 | 98.6 | 77 |
| 3 | 3 | 3 | 3 | 2 | 84.3 | 17 |
| 4 | 1.5 | 1.5 | 1.3 | 1 | 84.8 | 21 |
| 5 | 1.8 | 5.4 | 1.8 | 0 | 88.2 | 46 |
| 6 | 2.5 | 4.8 | 2.5 | 0 | 89.8 | 47 |
| 7 | 3 | 9 | 2.9 | 0 | 90.8 | 56 |
| 8 | 2.6 | 2.6 | 1.3 | 0 | 98.0 | 72 |
| 9 | 2.4 | 7.2 | 1.2 | 0 | 97.8 | 80 |
| 10 | 2.4 | 4.9 | 1.3 | 0 | 99.4 | 82 |
| 11 | 3 | 6 | 1.6 | 0 | 99.6 | 86 |
| 12 | 1.5 | 4.5 | 0.1 | 59 | 98.3 | 31 |
| 13 | 2 | 2 | 0.1 | 35 | 92.3 | 47 |
| 14 | 2.6 | 5.2 | 0.1 | 34 | 99.4 | 54 |
| 15 | 3 | 9 | 0.1 | 22 | 99.2 | 63 |
| 16 | 3 | 3 | 0.1 | 13 | 97.3 | 69 |

The yield was barely improved even when the equivalent amount of the Fmoc amino acid was increased under Reference Example Condition A (Entries 1 and 3).

On the other hand, reducing the equivalent amount of Oxyma improved the purity and the yield (Entries 4, 8, 13, and 16). Furthermore, increasing the equivalent amount of DIC improved the purity and the yield (Entries 5 and 6).

Moreover, reducing the equivalent amount of Oxyma and increasing the equivalent amount of DIC improved the purity and the yield (Entries 2, 9, 10, 11, 12, 14, and 15).

In particular, in Entries 2, 9, 10, and 11, the results having a yield exceeding 75% were obtained.

Thus, in Examples 2-1 to 2-31, an amino acid or a peptide, of which N-terminus was N-methylated, was elongated under conditions wherein 1 equivalent amount or more of DIC was used and 1 equivalent amount or less of the additive was used relative to an Fmoc amino acid to synthesize the peptide of interest. The recovery rate obtained with elongation up to the third residue exceeded 53% in all cases, and was as high as 90% or more in some cases. Experiments compared with Reference Example Condition A provided a yield and a purity both equal to or greater than those of Reference Example Condition A.

[Reference Example 1]

Synthesis of Fmoc-MeVal-MeAsp(OCTC)-pip (Compound 5) of Chem. Eur. J., 2009, 15, 9394-9403 (Reaction condition having Fmoc amino acid : DIC: Additive (Oxyma) = 2 : 2 : 2 relative to resin amino acid: Reference Example Condition A)

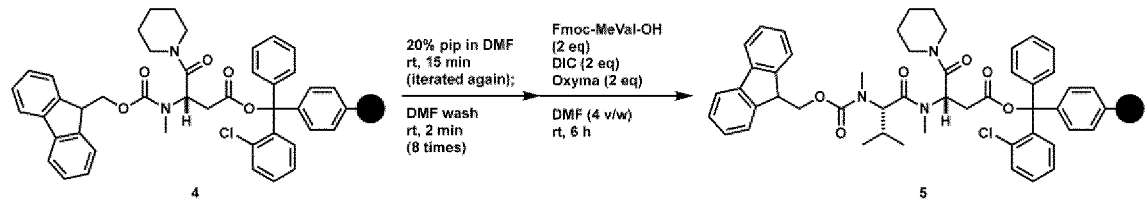


4 5

First, 0.200 g (0.525 mmol/g, 0.104 mmol, 1.0 eq) of a dry resin (Compound 4) was weighed into a 5 mL filter-equipped disposable syringe, 1.3 mL (10 v/w) of N,N-dimethylformamide was added, and the mixture was left to stand at room temperature for 30 minutes. After N,N-dimethylformamide was discharged, 1.1 mL (8 v/w) of an N,N-dimethylformamide solution containing 20% piperidine was added, and the mixture was shaken at room temperature for 15 minutes. After the reaction solution was discharged, 1.1 mL (8 v/w) of an N,N-dimethylformamide solution containing 20% piperidine was added again, and the mixture was shaken at room temperature for 15 minutes. After the reaction solution was discharged, the resin was washed with 1.3 mL (10 v/w) of N,N-dimethylformamide eight times. Next, 74 mg (0.21 mmol, 2.0 eq) of Fmoc-MeVal-OH and 30 mg (0.21 mmol, 2.0 eq) of Oxyma were weighed into a vial and dissolved by adding 0.54 mL (4 v/w) of N,N-dimethylformamide. Subsequently, 33 μL (0.21 mmol, 2.0 eq) of DIC was added to the vial at room temperature, and then the vessel was sealed and shaken at room temperature for 2 minutes. This solution was sucked into the disposable syringe, and the syringe was sealed and then shaken at room temperature for 6 hours. After the reaction solution was discharged, the resin was washed with 1.3 mL (10 v/w) of N,N-dimethylformamide four times, with 1.3 mL (10 v/w) of 2-propanol twice and with 1.3 mL (10 v/w) of methanol four times, and then dried under reduced pressure overnight at room temperature to give 0.178 g of a dry resin (Compound 5). Then, 21.6 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance of the solution using a spectrophotometer, and the yield was calculated from the resulting Fmoc quantitative value, the mass of the dry resin, and LC A%.

[Table 93]

| (HPLC method B: Identification of target molecule and purity measurement) | | | | | |
|---|---|---|---|---|---|
| | Mass | Fmoc quantitative (g) value (mmol/g) | LC A% | Amount of substance (mmol) | Yield (%) |
| Starting material (Compound 4) | 0.200 | 0.525 | 99.0 | 0.104 | - |
| Product (Compound 5) | 0.178 | 0.382 | 81.4 | 0.0554 | 53.3 |

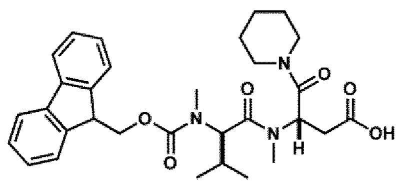

6

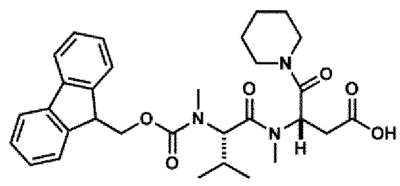

7

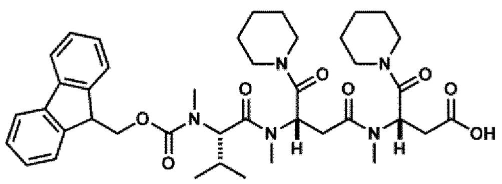

8

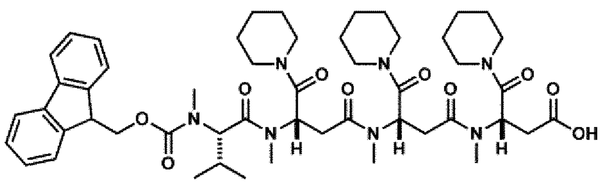

9

[Table 94]

| | Mw | m/z | Rt (min) | LC A% |
|---|---|---|---|---|
| Epimer (Compound 6) | 549.7 | 550.5 ([M+H]$^+$) | 9.0 | 0.4 |
| Target molecule (Compound 7) | 549.7 | 550.5 ([M+H]$^+$) | 9.4 | 81.4 |
| Excessively elongated product (Compound 8) | 745.9 | 661.5 ([M-pip+H]$^+$) | 12.1 | 13.8 |
| Excessively elongated product (Compound 9) | 942.2 | 491.3* | 14.2 | 2.4 |
| * Identified as excessively elongated product B from mass spectrometry of Glycine capping experiment. | | | | |

Glycine capping was carried out to verify the residual ratio of the starting material. More specifically, about 10 mg of a resin obtained by filtration of the reaction solution after the condensation reaction was transferred as an N,N-dimethylformamide suspension to a 5 mL filter-equipped disposable syringe. After N,N-dimethylformamide was discharged, the resin was washed with 1 mL of N,N-dimethylformamide three times. A cocktail for reaction (a light yellow uniform solution obtained by weighing about 70 mg of Fmoc-Gly-OH and about 0.11 g of HATU into a vial, suspending them in 0.45 mL of N,N-dimethylformamide, then adding 45 μL of N,N-diisopropylethylamine, and shaking the mixture for 1 minute) was sucked into the disposable syringe, and shaken at room temperature for 1 hour. After the reaction solution was discharged, the resin was washed with 1 mL of N,N-dimethylformamide six times and then with 1 mL of dichloromethane four times, and then a deresination reaction was carried out. The residual ratio of the starting material calculated from the sum of LC A% of the Gly-cap product (Compound 13), the epimer (Compound 6), the target molecule (Compound 7), the excessively elongated product (Compound 8), and the excessively elongated product (Compound 9) was 1.3% (1.3 / (1.3 + 79.9 + 13.4 + 2.3)).

[Table 95]

| (HPLC method A-c: Product identification and calculation of reaction conversion rate) | | | | |
|---|---|---|---|---|
| | Mw | m/z | Rt (min) | LC A% |
| Gly-cap product (Compound 13) | 493.6 | 494.3 ([M+H]$^+$) | 2.87 | 1.3 |
| Epimer (Compound 6) Target molecule (Compound 7) | 549. 7 | 550.5 ([M+H]$^+$) | 3.28 | 79.9 |
| Excessively elongated product (Compound 8) | 745.9 | 661.5 ([M-pip+H]$^+$) | 3.35 | 13.4 |
| Excessively elongated product (Compound 9) | 942.2 | 491.3* | 3.44 | 2.3 |

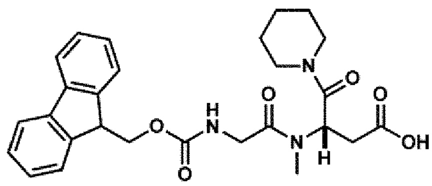

13

The yield of the target molecule was found to be decreased under the above reaction conditions. It was confirmed from formation of the Gly-cap product (Compound 13) that the reaction was incomplete, while formation of excessively elongated products (Compound 8 and Compound 9) was observed.

[Reference Example 2]

Synthesis of Fmoc-MeVal-MeAsp(OCTC)-pip (Compound 5) under reaction condition described in Eur. J. Org. Chem., 2013, 6372-6378 (Reaction condition having Fmoc amino acid : DIC: Additive (K-Oxyma) = 2 : 2 : 2 relative to amino acid onto resin)

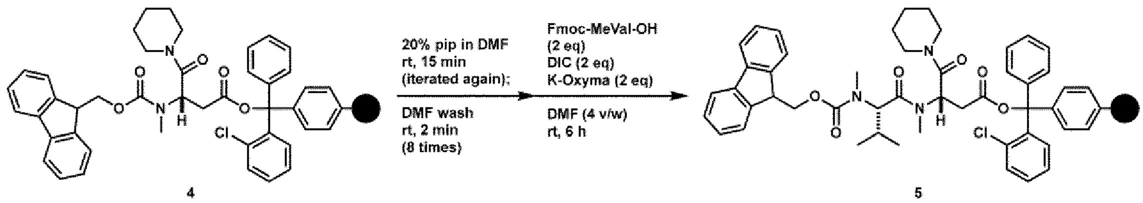


4

5

First, 0.202 g (0.525 mmol/g, 0.105 mmol, 1.0 eq) of a dry resin (Compound 4) was weighed into a 5 mL filter-equipped disposable syringe, 1.3 mL (10 v/w) of N,N-dimethylformamide was added, and the mixture was left to stand at room temperature for 30 minutes. After N,N-dimethylformamide was discharged, 1.1 mL (8 v/w) of an N,N-dimethylformamide solution containing 20% piperidine was added, and the mixture was shaken at room temperature for 15 minutes. After the reaction solution was discharged, 1.1 mL (8 v/w) of an N,N-dimethylformamide solution containing 20% piperidine was added again, and the mixture was shaken at room temperature for 15 minutes. After the reaction solution was discharged, the resin was washed with 1.3 mL (10 v/w) of N,N-dimethylformamide eight times. Next, 75 mg (0.21 mmol, 2.0 eq) of Fmoc-MeVal-OH and 40 mg (0.21 mmol, 2.0 eq) of K-Oxyma were weighed into a vial and dissolved by adding 0.54 mL (4 v/w) of N,N-dimethylformamide. Immediately after 33 μL (0.21 mmol, 2.0 eq) of DIC was added to the vial at room temperature, this solution was sucked into the disposable syringe, and the syringe was sealed and then shaken at room temperature for 6 hours. After the reaction solution was discharged, the resin was washed with 1.3 mL (10 v/w) of N,N-dimethylformamide four times, with 1.3 mL (10 v/w) of 2-propanol twice and with 1.3 mL (10 v/w) of methanol four times, and then dried under reduced pressure at room temperature overnight to give 0.188 g of a dry resin (Compound 5). Then, 21.0 mg of the resulting resin was weighed into a 100 mL volumetric flask, an N,N-dimethylformamide solution containing 20% piperidine was added to adjust the volume, and the mixture was shaken at room temperature for 45 minutes. Fmoc quantification was carried out from the absorbance of the solution using a spectrophotometer, and the yield was calculated from the resulting Fmoc quantitative value, the mass of the dry resin, and LC A%.

[Table 96]

| (HPLC method B: Identification of target molecule and purity measurement) | | | | | |
|---|---|---|---|---|---|
| | Mass (g) | Fmoc quantitative value (mmol/g) | LC A% | Amount of substance (mmol) | Yield (%) |
| Starting material (Compound 4) | 0.202 | 0.525 | 99.0 | 0.105 | - |
| Product (Compound 5) | 0.188 | 0.512 | 77.6 | 0.0748 | 71.5 |

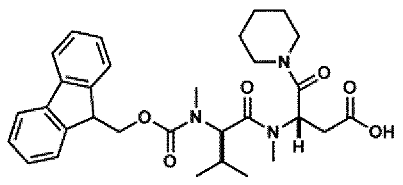

6

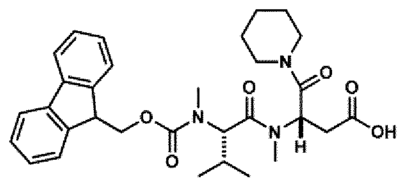

7

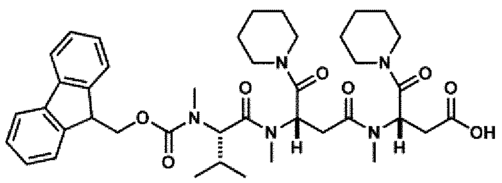

8

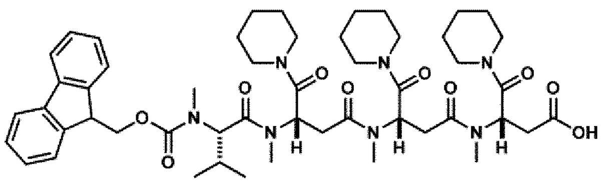

9

[Table 97]

| | Mw | m/z | Rt (min) | LC A% |
|---|---|---|---|---|
| Epimer (Compound 6) | 549.7 | 550.5 ([M+H]$^+$) | 9.0 | 20.2 |
| Target molecule (Compound 7) | 549.7 | 550.5 ([M+H]$^+$) | 9.4 | 77.6 |
| Excessively elongated product (Compound 8) | 745.9 | 746.5 ([M+H]$^+$) | 12.1 | 0.6 |
| Excessively elongated product (Compound 9) | 942.2 | Not detected | Not detected | Not detected |

The yield of the target molecule was found to be decreased under the above reaction conditions. An excessively elongated product (Compound 8) was suppressed, but increased production of an epimer (Compound 6) was confirmed.

[One-residue + two-residue condensation experiment] [Synthesis of Starting material]

H-MeVal-MeAsp(OCTC)-pip (Compound 14), which is a starting material for a condensation reaction, was synthesized according to the loading, condensation, and Fmoc removal conditions described herein. Condensation of one-residue + one-residue was carried out according to Example Condition A.

The peptide loaded ratio of the raw material for condensation reaction (Compound 14) was theoretically 0.63 mmol/g. This was calculated as follows.

1. The Fmoc quantitative value of a dry resin (Compound 4) was measured after loading Fmoc-MeAsp(OH)-pip (Compound 3) onto CTC resin, and the measured value was 0.59 mmol/g.
2. Considering that the molecular weight of Compound 3 was 436.51 (g/mol), 1 g of this dry resin (Compound 4) was composed of 0.258 g of Compound 1 and 0.742 g of a resin component according to the following formula:

Compound 3:

$$0.59\ (\text{mmol/g}) \times 1\ (\text{g}) \times 436.51\ (\text{g/mol}) = 0.258\ (\text{g}).$$

Resin component:

$$1\ (\text{g}) - 0.258\ (\text{g}) = 0.742\ (\text{g})$$

3. Considering that the mass of H-MeVal-MeAsp(OH)-pip (Compound 15) can be calculated based on the amount of substance of Compound 3 and the molecular weight of the peptide and that the mass of the resin component does not change before and after the reaction, the theoretical yield of the raw material for condensation reaction (Compound 14) obtained from 1 g of the dry resin (Compound 4) was:

Compound 14:

$$0.59\ (\text{mmol/g}) \times 1\ (\text{g}) \times 327.43\ (\text{g/mol}) + 0.742 = 0.935\ (\text{g}).$$

Accordingly, the theoretical peptide loading rate of the raw material for condensation reaction (Compound 14) was 0.63 (mmol/g) (0.59 / 0.935 = 0.63).

In this Reference Example as well, the amount of solvent when carrying out a solid phase reaction may be expressed in multiple (v/w). Unlike the one-residue + one-residue condensation experiments, the standard for multiple in this Reference Example is the mass of the dry resin (Compound 14), and thus when 0.80 mL of N,N-dimethylformamide is used relative to 100 mg of the dry resin, the multiple is 0.80 mL / 0.100 (g) = 8 v/w.

[Example 3]

Synthesis of Fmoc-cLeu-MeVal-MeAsp(OCTC)-pip (Compound 16) (Synthesis by Example Condition A)

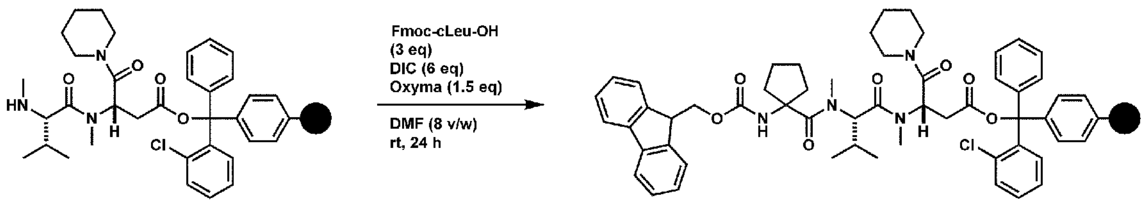


14 16

[Condensation Experiment]

First, 67 mg (0.19 mmol, 3.0 eq) of Fmoc-cLeu-OH and 13 mg (0.095 mmol, 1.5 eq) of Oxyma were weighed into a vial, and dissolved by adding 0.80 mL (8 v/w) of N,N-dimethylformamide. Then, 59 μL (0.38 mmol, 6.0 eq) of DIC was added to this solution, and the solution was shaken at room temperature for 2 hours. Then, 0.10 g (0.63 mmol/g, 0.063 mmol, 1.0 eq) of the dry resin (Compound 14) was added to this reaction solution, and the mixture was shaken at room temperature for 24 hours.

[Conversion Rate Verification Experiment]

First, 62 mg (0.21 mmol, 3.3 eq) of Fmoc-Gly-OH and 30 mg (0.21 mmol, 3.3 eq) of Oxyma were weighed into another vial, and dissolved by adding 0.40 mL (4 v/w) of N,N-dimethylformamide. Then, 65 μL (0.42 mmol, 6.6 eq) of DIC was added to this solution, and the mixture was shaken at room temperature for 1 hour. This solution was transferred to the reaction solution of Fmoc-cLeu-OH, and the mixture was shaken at room temperature for 2 hours. The reaction solution was transferred to a filter-equipped disposable syringe, and the reaction solution and the resin were separated by filtration. The resin was washed alternately with 0.80 mL (8 v/w) of N,N-dimethylformamide and with 0.80 mL (8 v/w) of isopropanol twice, then washed with 0.80 mL (8 v/w) of N,N-dimethylformamide twice, and finally washed with 0.80 mL (8 v/w) of MTBE. Then, the resin was air-dried at room temperature. The resulting dry resin (Compound 16) was immersed in 1 mL of a 1% TFA solution in dichloromethane to carry out a deresination reaction.

From LC A% of the Gly-cap product (Compound 17) and the target molecule (Compound 18), the conversion rate was calculated to be 75% (68.9 / (23.5 + 68.9)).

[Table 98]

| (HPLC method D: Calculation of conversion rate) | | |
|---|---|---|
| | Rt (min) | LC A% |
| Gly-cap product (Compound 17) | 14.6 | 23.5 |
| Target molecule (Compound 18) | 16.2 | 68.9 |

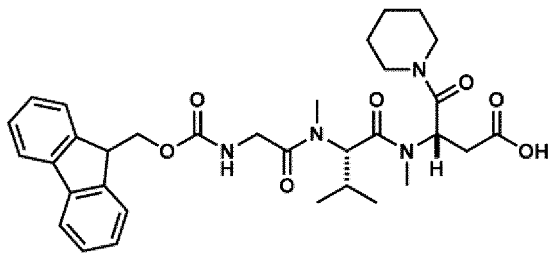

17

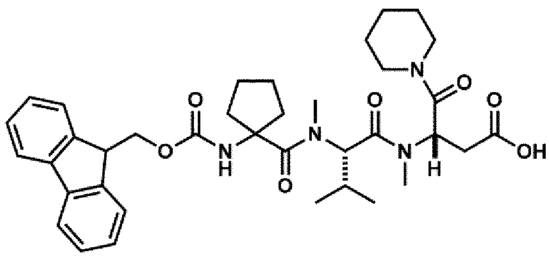

18

Below shows the results of carrying out the same reaction as the above [Condensation Experiment] except that a part of [Condensation Experiment] was modified.

[Table 99]

| Entry | Modified condition | Condition after modification | Conversion Rate (%) |
|---|---|---|---|
| 1 | - | - | 75 |

(continued)

| Entry | Modified condition | Condition after modification | Conversion Rate (%) |
|---|---|---|---|
| 2 | Oxyma 1.5 eq | HOAt 1.5 eq | 21 |
| 3 | Oxyma 1.5 eq | HOOBt 1.5 eq | 3 |
| 4 | Oxyma 1.5 eq | TCNHPI 1.5 eq | 6 |
| 5 | Oxyma 1.5 eq | Oxyma 0 eq | 6 |
| 6 | DMF for reaction 8 v/w | DMF for reaction 4 v/w | 92 |

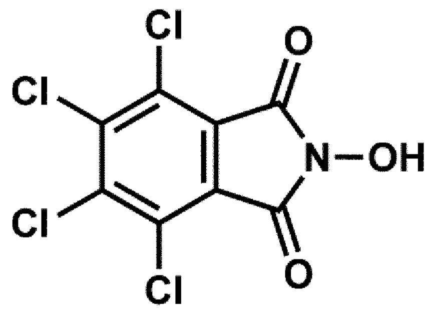

TCNHPI

It was found that when DIC and Oxyma were used in the reaction for condensing Fmoc-cLeu-OH, which is an amino acid having large steric hindrance, the condensation reaction proceeded efficiently. When the additive was changed from Oxyma to HOAt, HOOBt, and TCNHPI, respectively (Entry 2 to Entry 4), the conversion rate was significantly reduced as compared with Entry 1. When Oxyma was not used (Entry 5), the conversion rate was significantly reduced. When the solvent was changed from 8 v/w (Entry 1) to 4 v/w (Entry 6), the conversion rate was increased. Oxyma was found to be an excellent additive. The use of Oxyma was found to increase the conversion rate. Reducing the reaction solvent was found to increase the conversion rate.

[Reference Example 3]

Synthesis of Fmoc-cLeu-MeVal-MeAsp(OCTC)-pip (Compound 15) under the reaction condition described in Chem. Eur. J., 2009, 15, 9404-9416 (Reaction condition of Fmoc amino acid : condensing agent (COMU) = 3:3 relative to the amino acid onto resin)

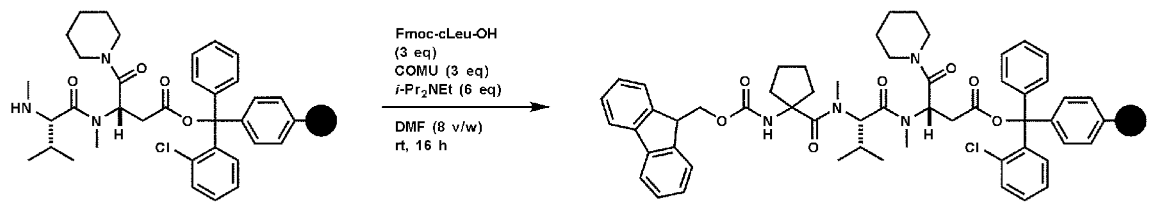


14 16

[Condensation Experiment]

First, 73 mg (0.21 mmol, 3.0 eq) of Fmoc-cLeu-OH and 89 mg (0.21 mmol, 3.0 eq) of COMU were weighed into a vial, and dissolved by adding 0.80 mL (8 v/w) of N,N-dimethylformamide. Then, 73 μL (0.42 mmol, 6.0 eq) of N,N-diisopropylethylamine was added to this solution, and the solution was shaken at room temperature for 30 minutes. Then, 0.10 g (0.63 mmol/g, 0.063 mmol, 1.0 eq) of the dry resin (Compound 14) was added to this reaction solution, and the mixture was shaken at room temperature for 16 hours.

[Conversion Rate Verification Experiment]

First, 62 mg (0.21 mmol, 3.3 eq) of Fmoc-Gly-OH and 30 mg (0.21 mmol, 3.3 eq) of Oxyma were weighed into another vial, and dissolved by adding 0.40 mL (4 v/w) of N,N-dimethylformamide. Then, 65 $\mu$L (0.42 mmol, 6.6 eq) of DIC was added to this solution, and the mixture was shaken at room temperature for 1 hour. This solution was transferred to the reaction solution of Fmoc-cLeu-OH, and the mixture was shaken at room temperature for 2 hours. The reaction solution was transferred to a filter-equipped disposable syringe, and the reaction solution and the resin were separated by filtration. The resin was washed alternately with 0.80 mL (8 v/w) of N,N-dimethylformamide and with 0.80 mL (8 v/w) of isopropanol twice, then washed with 0.80 mL (8 v/w) of N,N-dimethylformamide twice, and finally washed with 0.80 mL (8 v/w) of MTBE. Then, the resin was air-dried at room temperature. The resulting dry resin (Compound 16) was immersed in 1 mL of a 1% TFA solution in dichloromethane to carry out a deresination reaction.

From LC A% of the Gly-cap product (Compound 17) and the target molecule (Compound 18), the conversion rate was calculated to be 5.1% (4.7 / (88.3 + 4.7)).

[Table 100]

| (HPLC method D: Calculation of conversion rate) | | |
|---|---|---|
| | Rt (min) | LC A% |
| Gly-cap product (Compound 17) | 15.5 | 88.3 |
| Target molecule (Compound 18) | 17.1 | 4.7 |

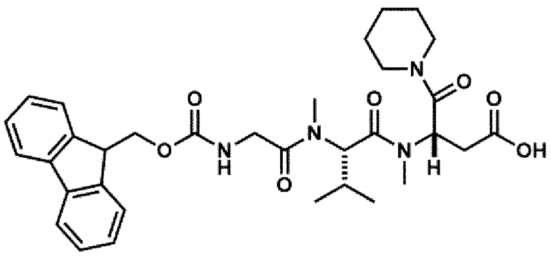

17

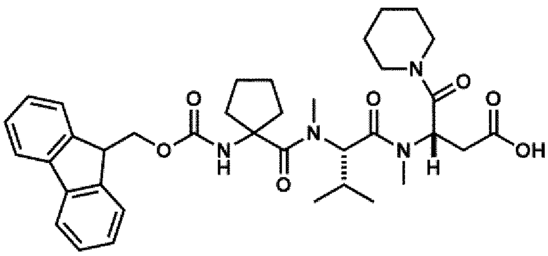

18

When COMU was used in the reaction for condensing Fmoc-cLeu-OH, which is an amino acid having large steric hindrance, the progress rate of the condensation reaction was 5%, and the main product was an unreacted Gly-cap product (Compound 17).

[Industrial Applicability]

The present invention provides methods of efficiently producing a peptide compound containing a sequence having poor reactivity in which a condensation reaction becomes incomplete when a conventional method is employed.

## Claims

1. A method of producing a peptide compound, the method comprising the step of condensing a first amino acid or peptide and a second amino acid or peptide in the presence of an additive and a condensing agent to give a condensation product, wherein
   the number of moles of the additive is smaller than the number of moles of the second amino acid or peptide.

2. The method according to claim 1, wherein a molar ratio of the additive to the second amino acid or peptide is 0.8 or less.

3. The method according to claim 1 or 2, wherein a molar ratio of the condensing agent to the second amino acid or peptide is 1.0 or more.

4. The method according to any one of claims 1 to 3, wherein a molar ratio of the condensing agent to the second amino acid or peptide is 1.2 to 4.0.

5. The method according to any one of claims 1 to 4, wherein a molar ratio of the second amino acid or peptide to the first amino acid or peptide is 10 or less.

6. The method according to any one of claims 1 to 5, wherein a molar ratio of the second amino acid or peptide to the first amino acid or peptide is 1 to 2, and a molar ratio of the additive to the second amino acid or peptide is 0.7 or less.

7. The method according to any one of claims 1 to 6, wherein a molar ratio of the second amino acid or peptide to the first amino acid or peptide (a first molar ratio) is 2 or more, and a molar ratio of the additive to the second amino acid or peptide is the first molar ratio - 1 or less.

8. The method according to any one of claims 1 to 7, wherein a molar ratio of the additive to the first amino acid or peptide is 2.0 or less.

9. The method according to any one of claims 1 to 8, wherein a molar ratio of the condensing agent to the first amino acid or peptide is 1.3 or more.

10. The method according to any one of claims 1 to 9, wherein the additive is Oxyma, HOBt, HOOBt, or HOAt.

11. The method according to any one of claims 1 to 10, wherein the condensing agent is DIC, DCC, EDCI, or EDCI·HCl.

12. The method according to any one of claims 1 to 11, wherein the peptide compound is the condensation product or comprises the condensation product in its structure.

13. The method according to any one of claims 1 to 12, wherein the first amino acid, or an N-terminal amino acid of the first peptide and/or a C-terminal amino acid of the first peptide, is an N-alkylamino acid.

14. The method according to claim 13, wherein the first amino acid, or a C-terminal amino acid of the first peptide, is an N-alkyl β-amino acid.

15. The method according to any one of claims 1 to 14, wherein the second amino acid or the C-terminal amino acid of the second peptide is an α,α-disubstituted amino acid, a β-branched amino acid, or an N-alkylamino acid.

16. A method of producing a cyclic peptide compound, the method comprising the steps of:

obtaining a peptide compound by the method according to any one of claims 1 to 15; and
cyclizing the peptide compound.

## INTERNATIONAL SEARCH REPORT

International application No.
PCT/JP2021/021125

**A. CLASSIFICATION OF SUBJECT MATTER**
C07K 1/04(2006.01)i; C07K 1/06(2006.01)i
FI: C07K1/04; C07K1/06
According to International Patent Classification (IPC) or to both national classification and IPC

**B. FIELDS SEARCHED**
Minimum documentation searched (classification system followed by classification symbols)
C07K1/04; C07K1/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII);
CAplus/REGISTRY/CASREACT/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2018/225851 A1 (CHUGAI PHARMACEUTICAL CO., LTD.) 13 December 2018 (2018-12-13) claims, paragraphs [0109]-[0114], examples, in particular, example 2, tables 2-1, 2-2, example 4-1 | 1-3, 5, 7, 9-16 |
| Y | claims, paragraphs [0109]-[0114], examples, in particular, example 2, tables 2-1, 2-2, example 4-1 | 4 |
| X | JP 2001-514659 A (BASF AG.) 11 September 2001 (2001-09-11) claims, in particular, claim 1, page 36, line 7 to page 40, line 2, examples, in particular, example 1A, e), g), example 1B, e), g), example 2B | 1-3, 5, 6, 8, 10-13, 15 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16 August 2021 (16.08.2021) | Date of mailing of the international search report<br>24 August 2021 (24.08.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/021125

C (Continuation). DOCUMENTS CONSIDERED TO BE RELEVANT

| Category* | Citation of document, with indication, where appropriate, of the relevant passages | Relevant to claim No. |
|---|---|---|
| X | NOZAKI, S., "Delay of coupling caused by excess additives", Journal of peptide science, 2006, vol. 12, pp. 147-153, https://doi.org/10.1002/psc.689 abstract, introduction, page 147 "Carbodiimide-additive Procedure", table 2 | 1-3, 5, 6, 8, 10-12, 16 |
| Y | JP 2018-070590 A (CEM CORP.) 10 May 2018 (2018-05-10) claims, examples | 4 |

Form PCT/ISA/210 (continuation of second sheet) (January 2015)

**INTERNATIONAL SEARCH REPORT**
Information on patent family members

International application No.
PCT/JP2021/021125

| Patent Documents referred in the Report | Publication Date | Patent Family | Publication Date |
|---|---|---|---|
| WO 2018/225851 A1 | 13 Dec. 2018 | EP 3636656 A1 claims, paragraphs [0120]-[0126], examples, in particular, example 2, tables 2-1, 2-2, example 4-1<br>CN 110799520 A<br>KR 10-2020-0016941 A | |
| JP 2001-514659 A | 11 Sep. 2001 | US 6103698 A claims, in particular, claim 1, column 15, line 53 to column 18, line 2, examples, in particular, example 1A, e), g), example 1B, e), g), example 2B<br>WO 1998/040092 A1<br>EP 981358 A1<br>CN 1252728 A<br>KR 10-0555604 B1 | |
| JP 2018-070590 A | 10 May 2018 | US 2018/0066013 A1 claims, examples<br>EP 3290430 A1<br>CN 107793467 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2011006644 A
- WO 2015154031 A
- WO 2017070512 A
- WO 2018225851 A
- WO 2019117274 A
- WO 2018225864 A


### Non-patent literature cited in the description


- *Future Med. Chem.,* 2009, vol. 1, 1289-1310
- *Acc Chem. Res.,* 2008, vol. 41, 1331-1342
- *Angew. Chem. Int. Ed.,* 2013, vol. 52, 254-269
- *Amino Acids, Peptides and Proteins in Organic Chemistry: Building Blocks, Catalysis and Coupling Chemistry,* 2011, vol. 3
- *Amino Acids,* 2018, vol. 50, 39-68
- Solid phase peptide synthesis. Bachem, 28 May 2020
- *QSAR Comb. Sci.,* 2007, vol. 26, 1027-1035
- *ACS Comb. Sci.,* 2013, vol. 15, 229-234
- *Eur. J. Org. Chem.,* 2013, 6372-6378
- Side reactions in Peptide Synthesis. Academic Press, 2015, 1-31
- *Chem. Eur. J.,* 2009, vol. 15, 9394-9403
- *Green. Chem.,* 2019, vol. 21, 2594-2600
- Comprehensive Organic Transformations, A Guide to Functional Group Preparations
- March's Advanced Organic Chemistry: Reactions, Mechanisms, and Structure
- Greene's ''Protective Groups in Organic Synthesis. John Wiley & Sons, 2014
- Greene's, ''Protective Groups in Organic Synthesis. John Wiley & Sons, 2014